# EUROPEAN PATENT APPLICATION

(11) **EP 3 961 736 A1**
(43) Date of publication of application: **02.03.2022**
(21) Application number: 20903027.9
(22) Date of filing: 04.12.2020
(51) Int. Cl.: H01L 51/00, H01L 51/50

(54) **ORGANIC LIGHT EMITTING DEVICE**

(30) Priority: 19.12.2019 KR 20190170947; 03.12.2020 KR 20200167777
(71) Applicant: LG CHEM, LTD., Yeongdeungpo-gu, Seoul 07336 (KR)
(72) Inventor: SUH, Sang Duk, Daejeon 34122 (KR); JUNG, Min Woo, Daejeon 34122 (KR); LEE, Jungha, Daejeon 34122 (KR); HAN, Su Jin, Daejeon 34122 (KR); PARK, Seulchan, Daejeon 34122 (KR); HWANG, Sunghyun, Daejeon 34122 (KR); LEE, Dong Hoon, Daejeon 34122 (KR)
(74) Representative: Hoffmann Eitle
(86) International application number: PCT/KR2020/017667
(87) International publication number: WO 2021/125649

(57) **Abstract**

The present disclosure provides an organic light emitting device having improved driving voltage, efficiency and lifetime.

## Description

### [TECHNICAL FIELD]

### Cross-reference to Related Application(s)

This application claims the benefit of Korean Patent Application No. 10-2019-0170947 filed on December 19, 2019 and Korean Patent Application No. 10-2020-0167777 filed on December 3, 2020 in the Korean Intellectual Property Office, each of which is incorporated herein by reference in its entirety.

The present disclosure relates to an organic light emitting device having improved driving voltage, efficiency and lifetime.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

In the above-mentioned organic light emitting devices, there is a continuing need for the development of an organic light emitting device having improved driving voltage, efficiency and lifetime.

### [DETAILED DESCRIPTION OF THE INVENTION]

### [TECHNICAL PROBLEM]

It is an object of the present disclosure to provide an organic light emitting device having improved driving voltage, efficiency and lifetime.

### [TECHNICAL SOLUTION]

Provided herein is the following organic light emitting device:
An organic light emitting device including: an anode, a cathode, and a light emitting layer interposed between the anode and the cathode,
wherein the light emitting layer includes a first compound represented by the following Chemical Formula 1 and a second compound represented by the following Chemical Formula 2. 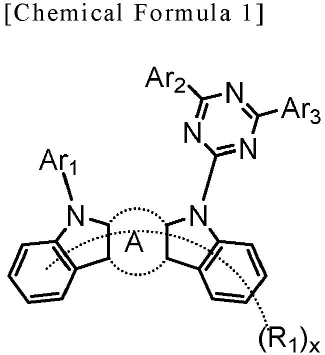
in the Chemical Formula 1,
A is a benzene ring fused with two adjacent pentagonal rings, respectively,
Ar₁ to Ar₃ are each independently a substituted or unsubstituted C₆₋₆₀ aryl; or a substituted or unsubstituted C₅₋₆₀ heteroaryl containing any one or more heteroatoms selected from the group consisting of N, O and S,
each R₁ is independently hydrogen; deuterium; halogen; cyano; a substituted or unsubstituted C₁₋₆₀ alkyl; a substituted or unsubstituted C₁₋₆₀ alkoxy; a substituted or unsubstituted C₂₋₆₀ alkenyl; a substituted or unsubstituted C₂₋₆₀ alkynyl; a substituted or unsubstituted C₃₋₆₀ cycloalkyl; a substituted or unsubstituted C₆₋₆₀ aryl; or a substituted or unsubstituted C₂₋₆₀ heteroaryl containing any one or more heteroatoms selected from the group consisting of N, O and S,
x is an integer of 1 to 10,
provided that at least one of Ar₁ to Ar₃, and R₁ is substituted with at least one deuterium, or at least one of R₁ is deuterium, and the first compound comprises at least 5 deuteriums, 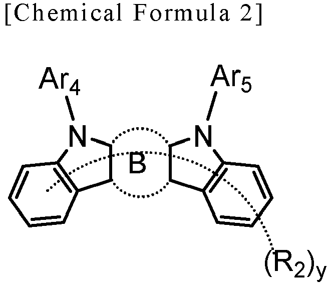
in the Chemical Formula 2,
B is a benzene ring fused with two adjacent pentagonal rings, respectively,
Ar₄ is a substituted or unsubstituted C₆₋₆₀ aryl,
Ars is a substituted or unsubstituted C₆₋₆₀ aryl; or a substituted or unsubstituted C₅₋₆₀ heteroaryl containing one heteroatom selected from the group consisting of N, O and S,
each R₂ is independently hydrogen; deuterium; halogen; cyano; a substituted or unsubstituted C₁₋₆₀ alkyl; a substituted or unsubstituted C₁₋₆₀ alkoxy; a substituted or unsubstituted C₂₋₆₀ alkenyl; a substituted or unsubstituted C₂₋₆₀ alkynyl; a substituted or unsubstituted C₃₋₆₀ cycloalkyl; a substituted or unsubstituted C₆₋₆₀ aryl; or a substituted or unsubstituted C₂₋₆₀ heteroaryl containing any one or more heteroatoms selected from the group consisting of N, O and S, and
y is an integer of 1 to 10.

### [ADVANTAGEOUS EFFECTS]

The above-mentioned organic light emitting device has excellent driving voltage, efficiency, and lifetime by containing the first compound represented by Chemical Formula 1 and the second compound represented by Chemical Formula 2 in the light emitting layer.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron injection and transport layer 9, and a cathode 4.

### [DETAILED DESCRIPTION OF THE EMBODIMENTS]

Hereinafter, embodiments of the present disclosure will be described in more detail to facilitate understanding of the invention.

As used herein, the notation 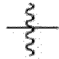 or 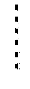 means a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent to which two or more substituents of the above-exemplified substituents are connected. For example, "a substituent in which two or more substituents are connected" may be a biphenyl group. Namely, a biphenyl group may be an aryl group, or it may be interpreted as a substituent in which two phenyl groups are connected.

In the present disclosure, the carbon number of a carbonyl group is not particularly limited, but is preferably 1 to 40. Specifically, the carbonyl group may be a compound having the following structural formulas, but is not limited thereto.

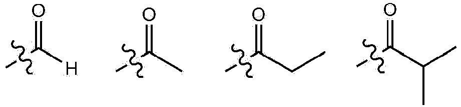

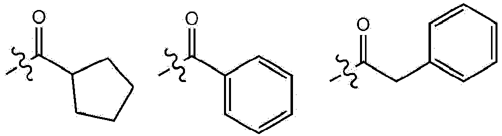

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a compound having the following structural formulas, but is not limited thereto.

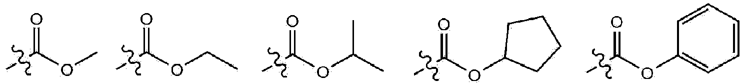

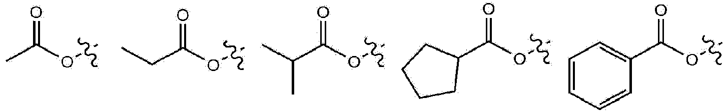

In the present disclosure, the carbon number of an imide group is not particularly limited, but is preferably 1 to 25. Specifically, the imide group may be a compound having the following structural formulas, but is not limited thereto.

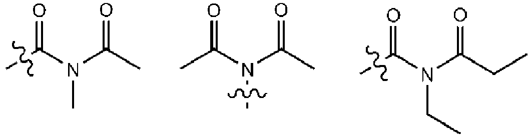

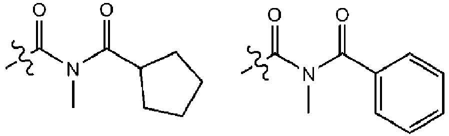

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthrenyl group, a pyrenyl group, a perylenyl group, a chrysenyl group, or the like, but is not limited thereto.

In the present disclosure, the fluorenyl group may be substituted, and two substituents may be linked with each other to form a spiro structure. In the case where the fluorenyl group is substituted, 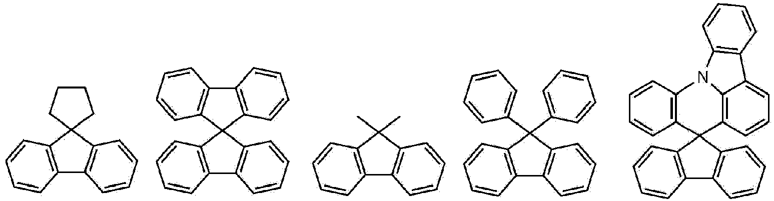 and the like can be formed. However, the structure is not limited thereto.

In the present disclosure, a heterocyclic group is a heterocyclic group containing one or more of O, N, Si and S as a heteroatom, and the carbon number thereof is not particularly limited, but is preferably 2 to 60. Examples of the heterocyclic group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine group can be applied to the aforementioned description of the heterocyclic group. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heterocyclic group can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heterocyclic group can be applied, except that the heterocycle is not a monovalent group but formed by combining two substituent groups.

The organic light emitting device according to the present disclosure may be a normal type organic light emitting device in which an anode, a light emitting layer and a cathode are sequentially stacked on a substrate, or an inverted type organic light emitting device in which a cathode, a light emitting layer and an anode are sequentially stacked on a substrate. Further, the organic light emitting device may further selectively include at least one of a hole injection layer, a hole transport layer, and an electron blocking layer between the anode and the light emitting layer, and may further include at least one of a hole blocking layer, an electron injection layer, and an electron transport layer between the cathode and the light emitting layer. In addition, the electron injection layer and the electron transport layer may be included in the form of a single layer of the electron injection and transport layer instead of a separate layer.

Hereinafter, the present disclosure will be described in detail for each configuration.

### Anode and Cathode

An anode and a cathode used in the present disclosure mean electrodes used in an organic light emitting device.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SNO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

### Hole Injection Layer

The organic light emitting device according to the present disclosure may further include a hole injection layer on the anode, if necessary.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. Further, it is preferable that a HOMO (highest occupied molecular orbital) of the hole injection material is between the work function of the anode material and a HOMO of a peripheral organic material layer.

Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive compound, and the like, but are not limited thereto.

### Hole Transport Layer

The organic light emitting device according to the present disclosure may include a hole transport layer on the anode (or on the hole injection layer if the hole injection layer exists), if necessary.

The hole transport layer is a layer that can receive the holes from the anode or the hole injection layer and transport the holes to the light emitting layer, and the hole transport material is suitably a material having large mobility to the holes, which may receive holes from the anode or the hole injection layer and transfer the holes to the light emitting layer.

Specific examples thereof include an arylamine-based organic material, a conductive polymer, a block copolymer in which a conjugate portion and a non-conjugate portion are present together, and the like, but are not limited thereto.

### Light Emitting Layer

The light emitting layer used in the present disclosure is a layer that can emit light in the visible light region by combining holes and electrons transported from the anode and the cathode. Generally, the light emitting layer includes a host material and a dopant material, and in the present disclosure, the first compound represented by Chemical Formula 1 and the second compound represented by Chemical Formula 2 is included as a host

Preferably, the first compound represented by Chemical Formula 1 may be substituted with 5 or more deuteriums. Specifically, at least one of Ar₁ to Ar₃ and R₁ in Chemical Formula 1 is substituted with at least one deuterium, or at least one of R₁ becomes deuterium, whereby the first compound may include 5 or more, or 6 or more, or 7 or more, or 8 or more, or 10 or more deuteriums in the compound, or the first compound may be completely substituted with deuterium.

Preferably, the Chemical Formula 1 may be represented by any one selected from the group consisting of the following Chemical Formulas 1-1 to 1-6: 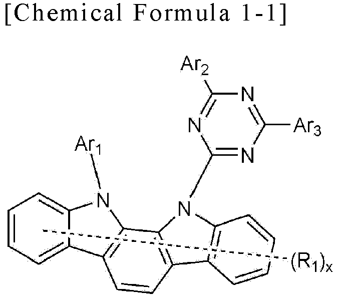 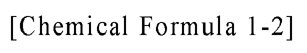 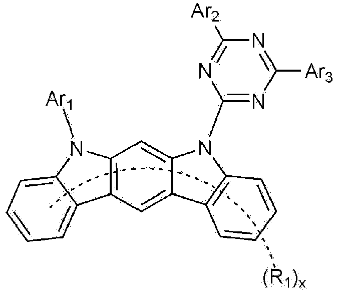 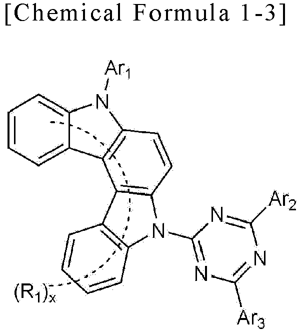 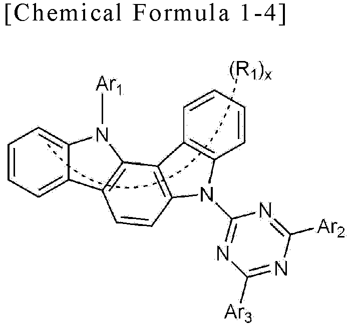 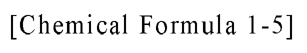 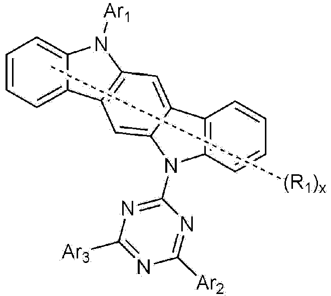 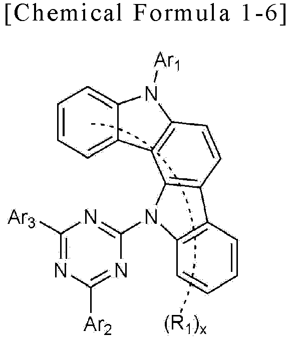 in the Chemical Formulas 1-1 to 1-6, Ar₁ to Ar₃, R₁, and x are as defined above.

In Chemical Formula 1, preferably, Ar₁ to Ar₃ may be each independently phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl.

In Chemical Formula 1, more preferably, at least one of Ar₁ to Ar₃ may be phenyl, and the rest may be each independently phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl.

Further, at least one of the Ar₁ to Ar₃ may be substituted with at least one deuterium. For example, at least one of the Ar₁ to Ar₃ may be selected from the group consisting of the following structures: 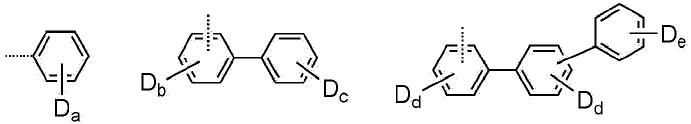 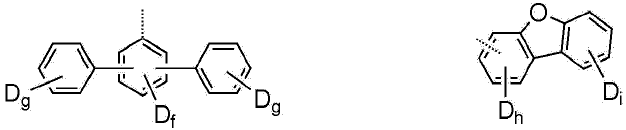 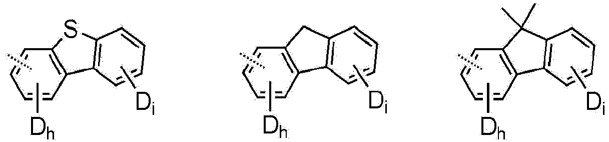 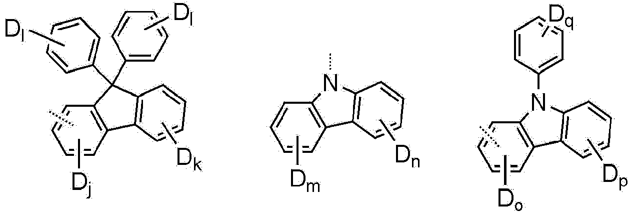 in the above formula, a is an integer of 1 to 5,
b is an integer of 0 to 4, c is an integer of 0 to 5, provided that b and c are not 0 at the same time,
each d is independently an integer of 0 to 3, e is an integer of 0 to 5, provided that d and e are not 0 at the same time,
f is an integer of 0 to 3, each g is independently an integer of 0 to 5, provided that f and g are not 0 at the same time,
h is an integer of 0 to 3, i is an integer of 0 to 4, provided that h and i are not 0 at the same time,
j is an integer of 0 to 3, k is an integer of 0 to 4, each 1 is independently an integer of 0 to 5, provided that j, k and 1 are not 0 at the same time,
m is an integer of 0 to 4, n is an integer of 0 to 4, provided that m and n are not 0 at the same time, and
o is an integer of 0 to 3, p is an integer of 0 to 4, q is an integer of 0 to 5, provided that o, p, and q are not 0 at the same time.

Further, preferably, at least one of the Ar₁ to Ar₃ may be substituted with 5 or more deuteriums.

More preferably, in Chemical Formula 1, at least one of the Ar₁ to Ar₃ may be phenyl substituted with 5 deuteriums, and the rest may be phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is unsubstituted.

As an example, all of the Ar₁ to Ar₃ are phenyl, provided that at least one of the Ar₁ to Ar₃, specifically any one, two, or all three of Ar₁ to Ar3 may be substituted with 5 deuteriums.

As another example, one of the Ar₁ to Ar₃ is biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is unsubstituted, and the rest may be phenyl, provided that at least one of the rest, that is, one or both of the rest may be substituted with 5 deuteriums.

Further, preferably, at least one of the Ar₁ to Ar₃ may be biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is substituted with 5 or more deuteriums, and the rest may be each independently an unsubstituted phenyl or an unsubstituted biphenyl.

As an example, one of the Ar₁ to Ar₃ may be biphenyl substituted with 5 deuteriums, and the rest may be each independently an unsubstituted phenyl or an unsubstituted biphenyl. At this time, those in which biphenyl is substituted with 5 deuteriums may be represented by the following structure as an example. 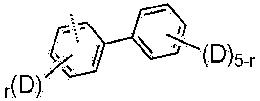 in the above formula, r is an integer of 0 to 4, more specifically 0.

As another example, two of the Ar₁ to Ar₃ may be biphenyl substituted with 5 deuteriums, and the rest may be unsubstituted phenyl. At this time, those in which biphenyl is substituted with 5 deuteriums may be represented by the above-mentioned structure.

As another example, one of the Ar₁ to Ar₃ may be biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is completely substituted with deuterium, and all the rest may be an unsubstituted phenyl.

Further, preferably, the Ar₁ to Ar₃ may be each independently phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, and all of the Ar₁ to Ar₃ may be completely substituted with deuterium.

Further, in Chemical Formula 1, preferably, all of the R₁ may be hydrogen. At this time, in Chemical Formula 1, x is an integer of 10, and at least one of Ar₁ to Ar₃ is substituted with 5 or more deuteriums.

Further, in Chemical Formula 1, preferably, at least 5 of R₁ may be deuterium, and the rest may be hydrogen. At this time, in Chemical Formula 1, x is an integer of 10. For example, in Chemical Formula 1, 5, or 6, or 7, or 8 of the R₁ are deuterium, and the rest may be hydrogen, or all 10 R₁s may be deuterium.

Representative examples of the first compound represented by Chemical Formula 1 are as follows:

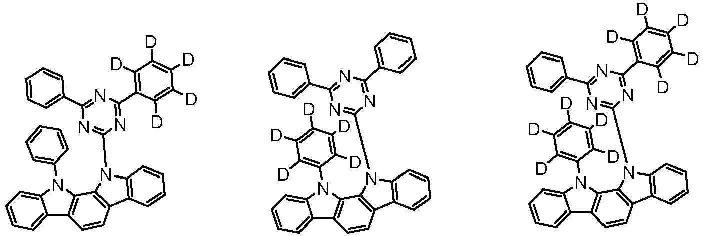

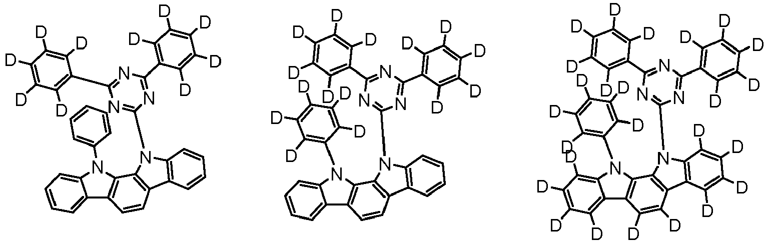

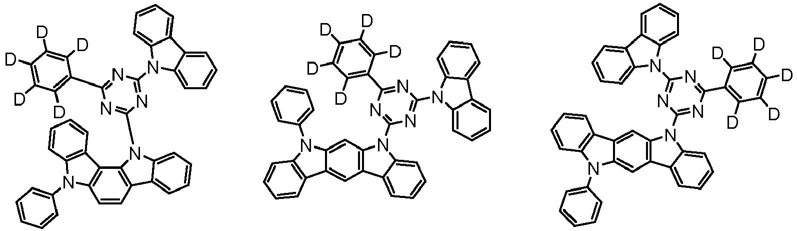

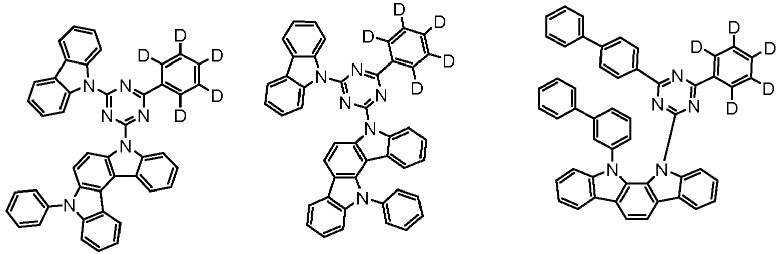

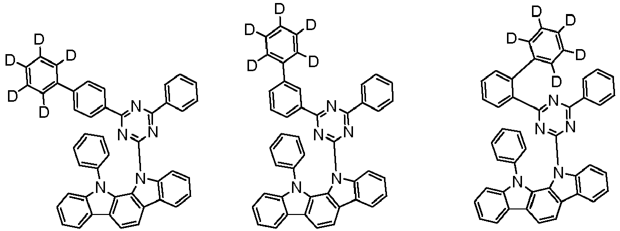

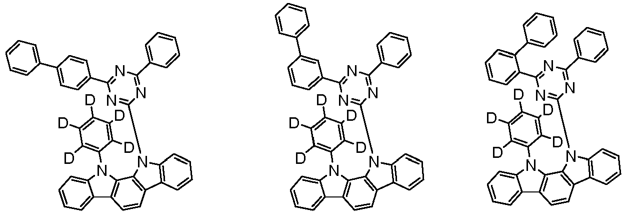

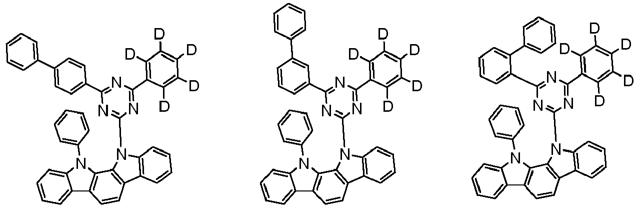

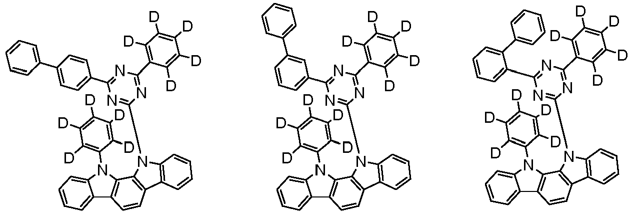

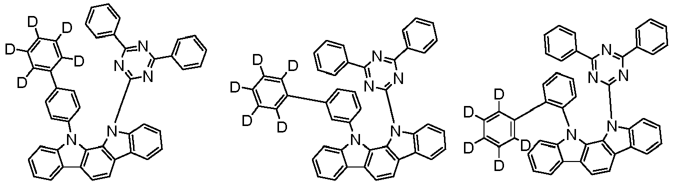

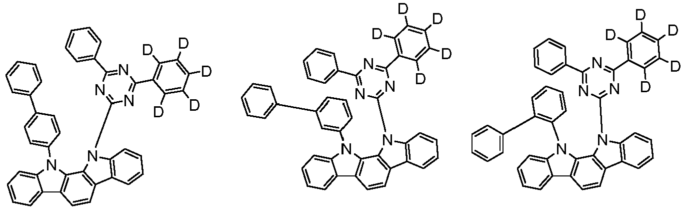

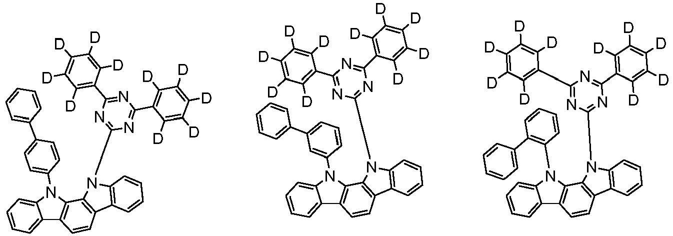

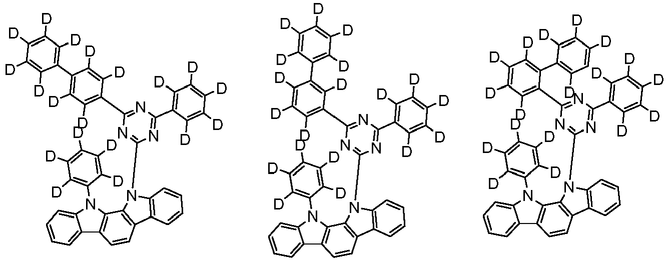

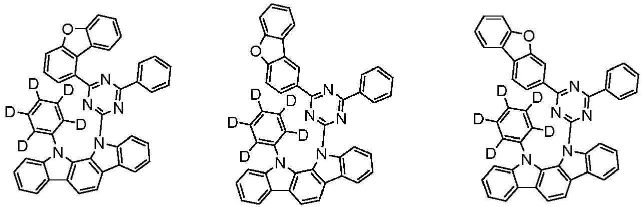

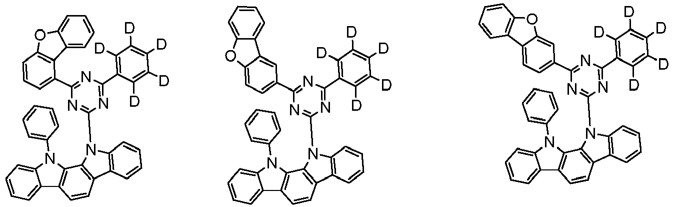

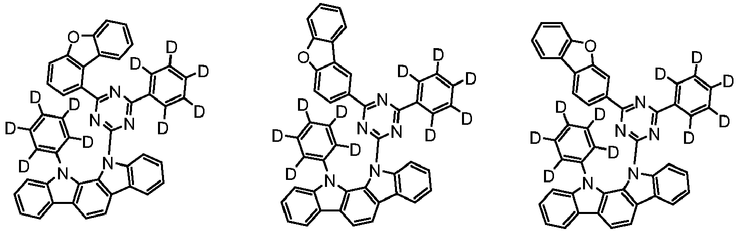

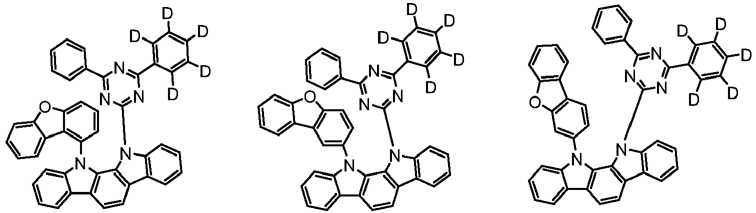

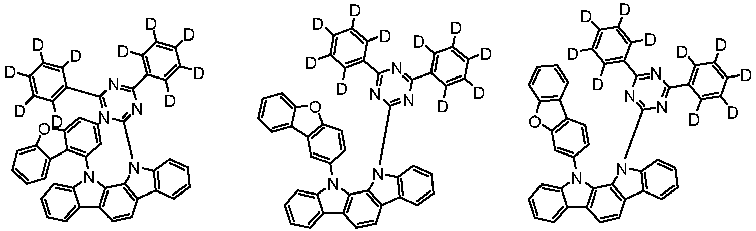

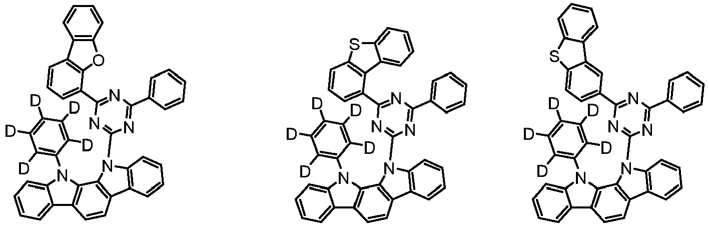

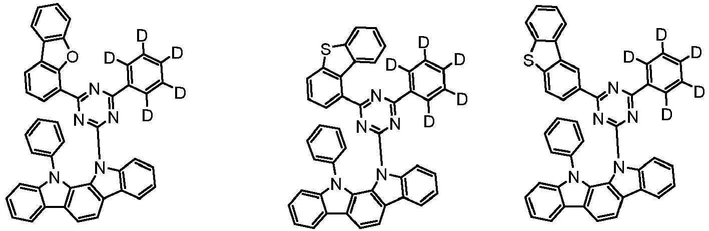

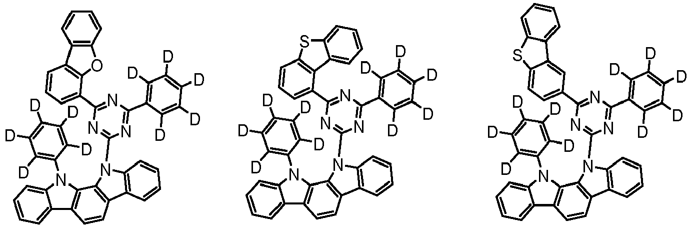

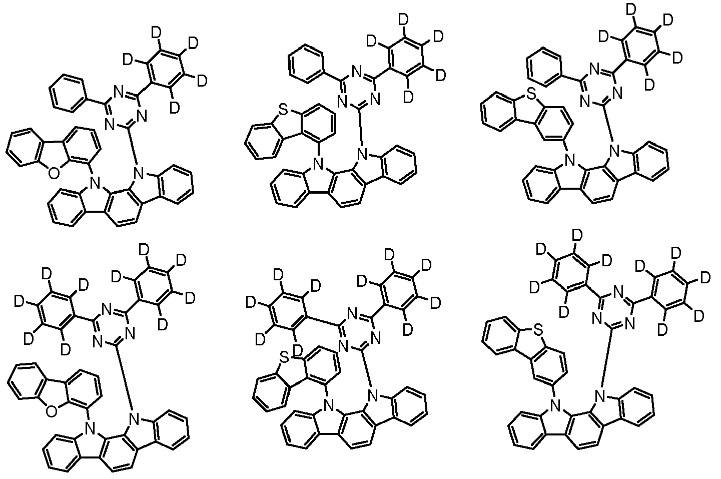

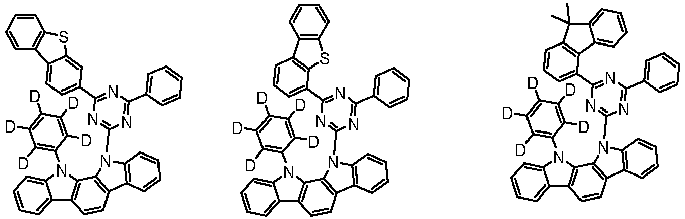

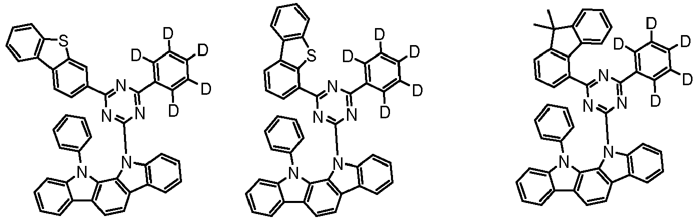

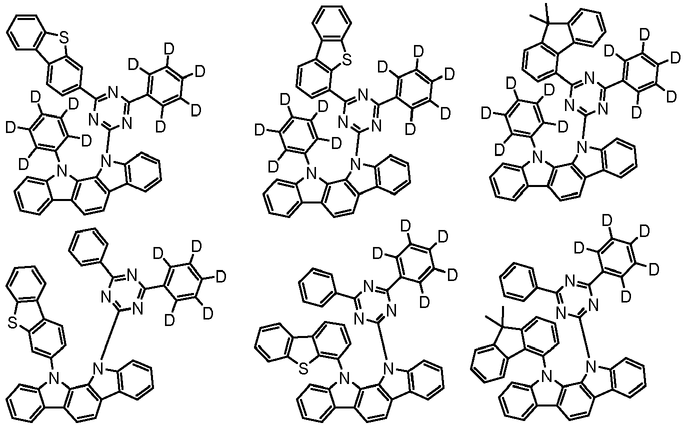

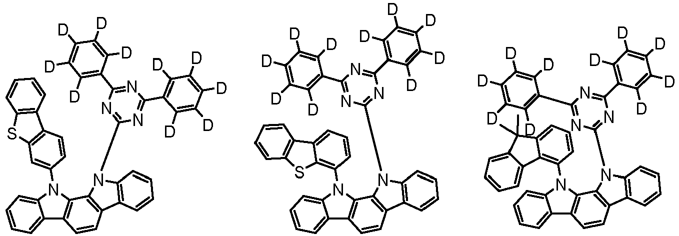

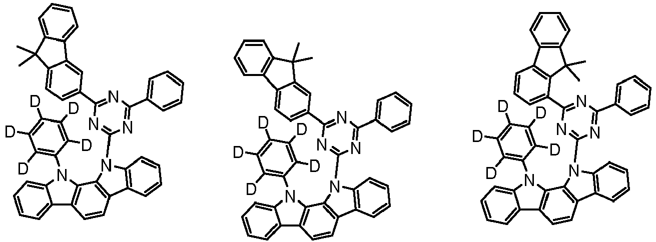

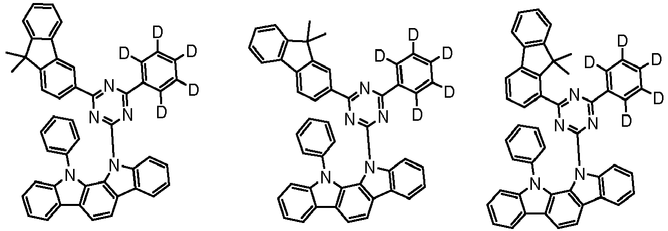

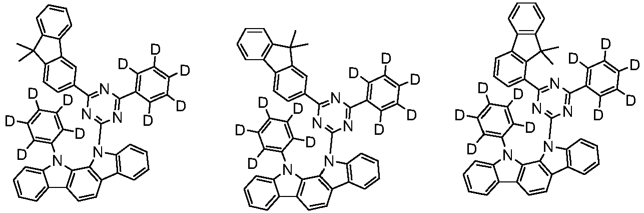

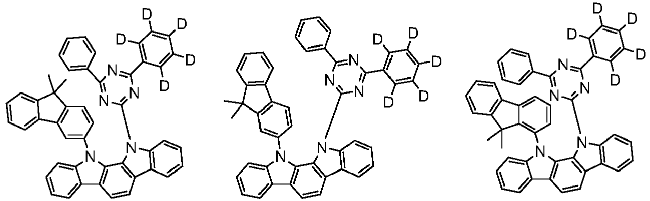

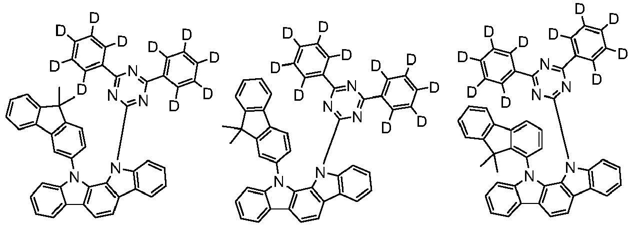

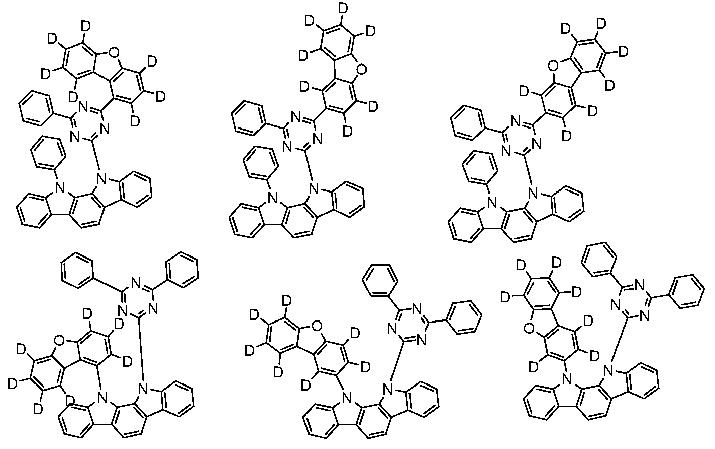

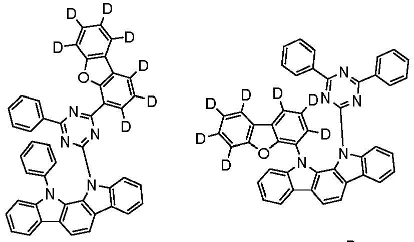

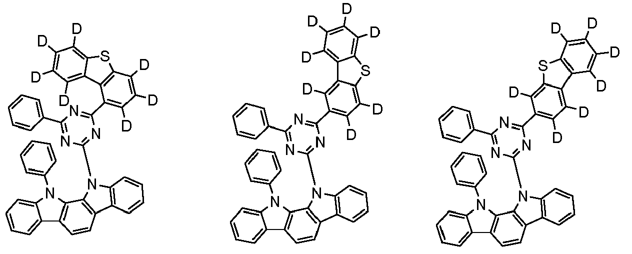

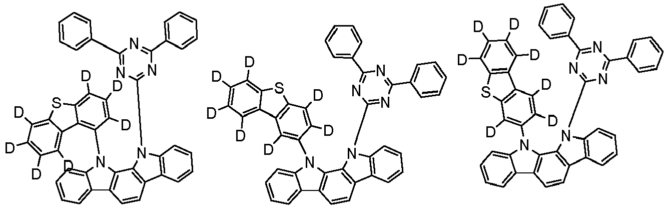

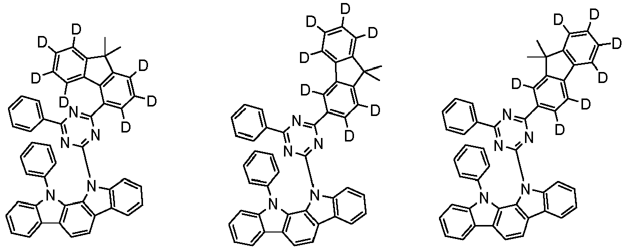

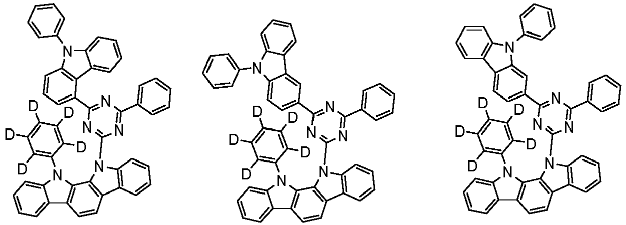

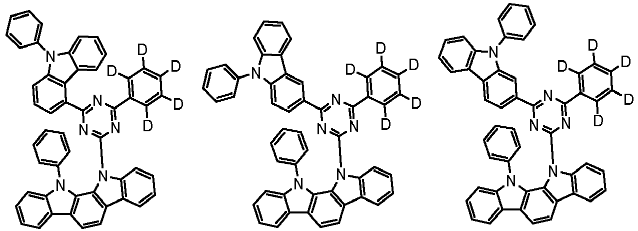

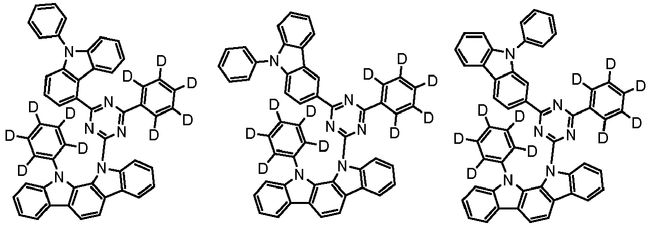

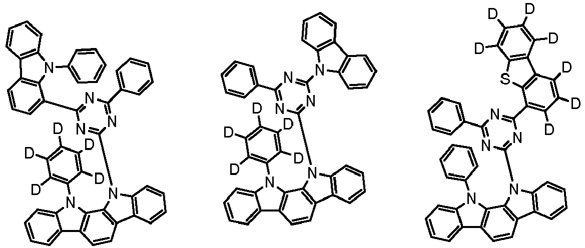

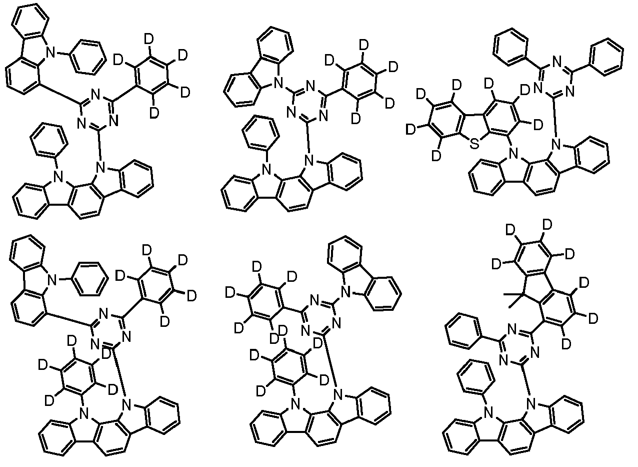

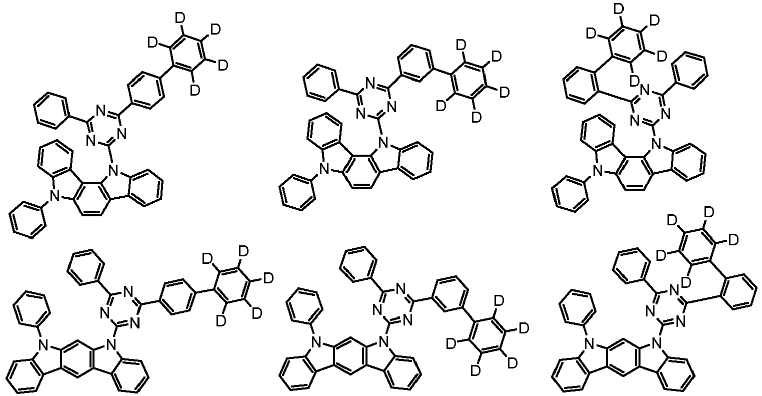

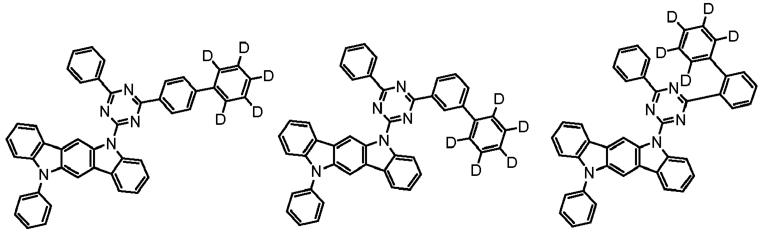

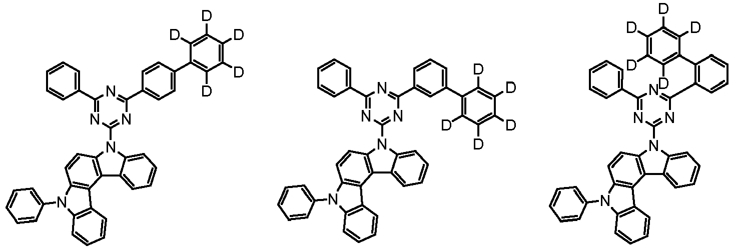

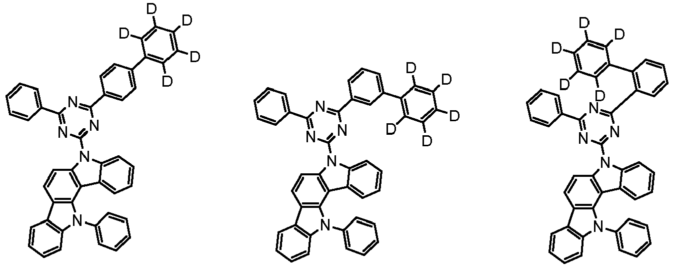

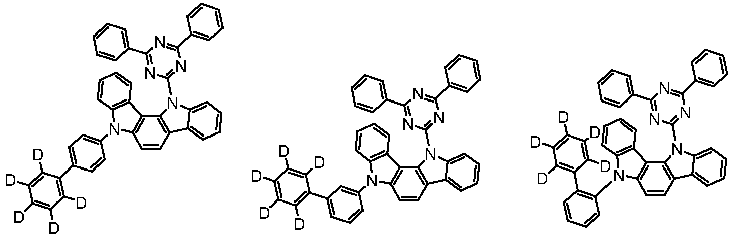

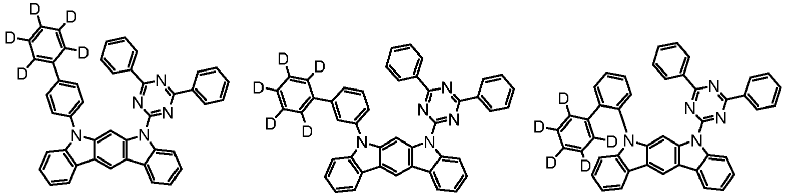

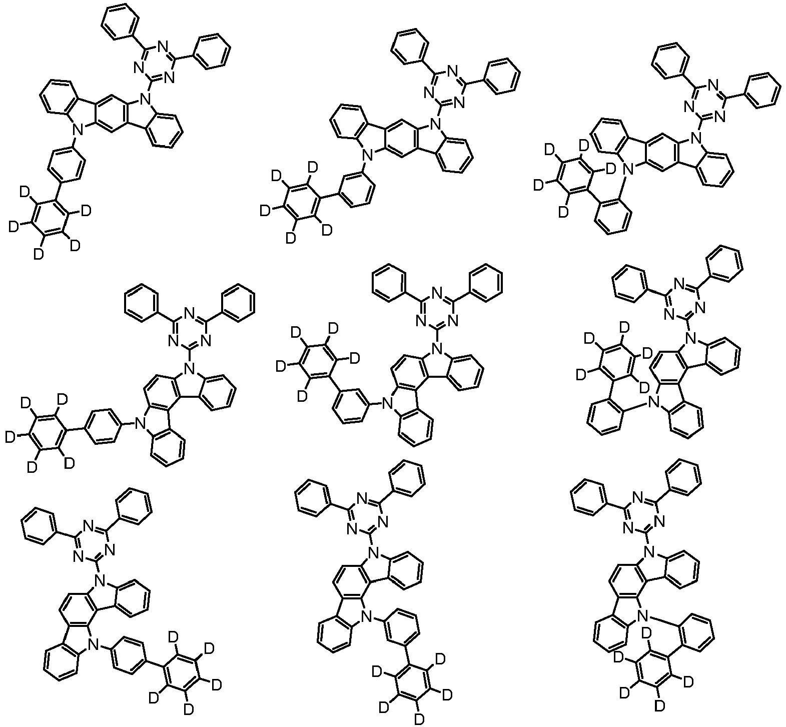

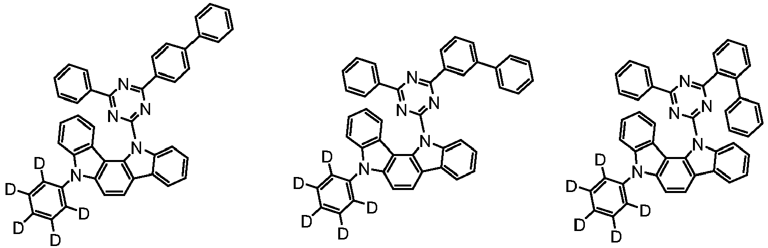

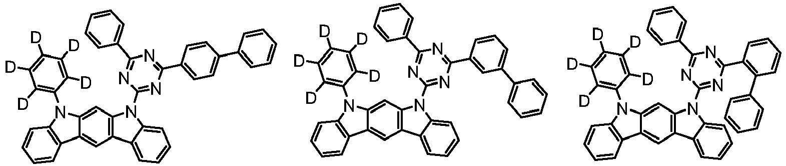

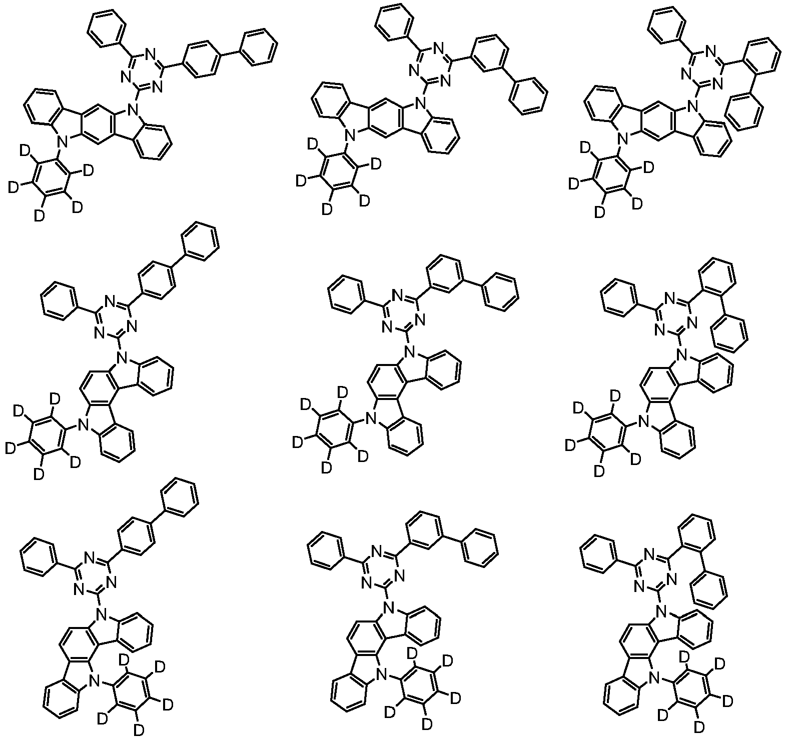

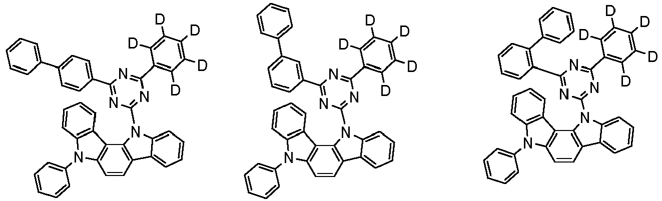

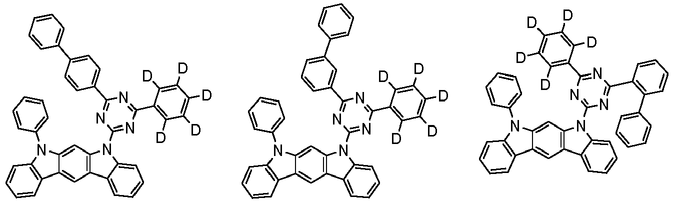

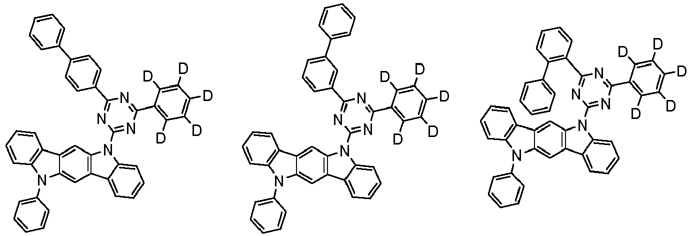

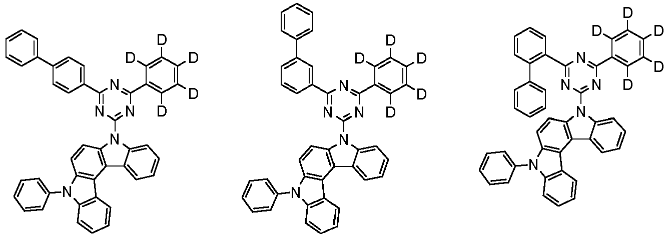

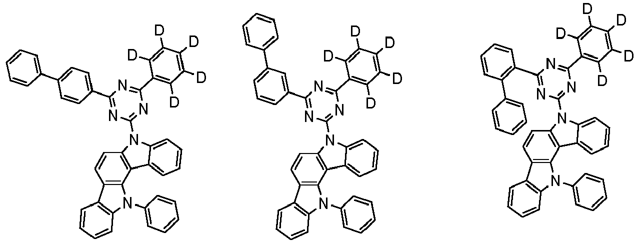

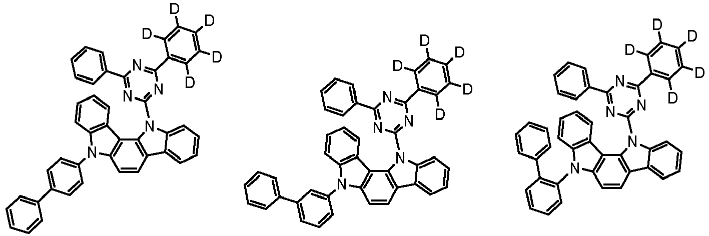

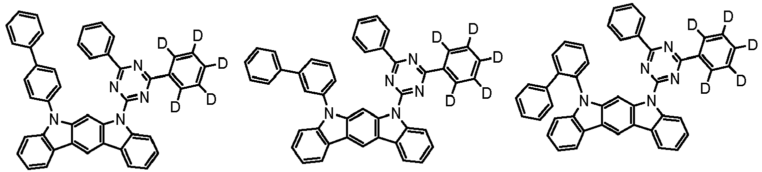

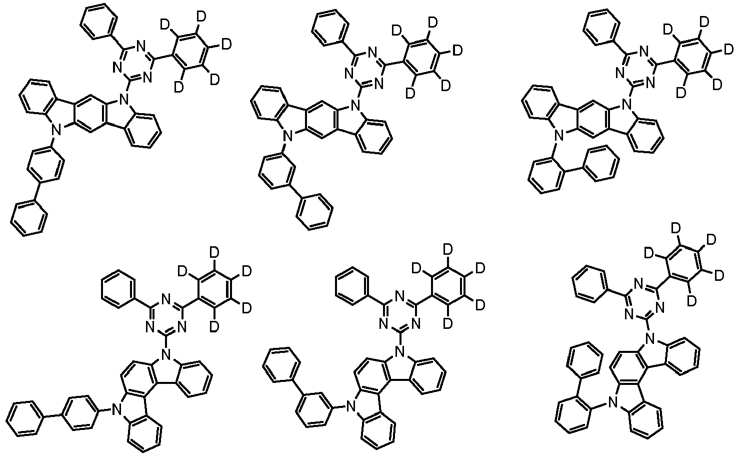

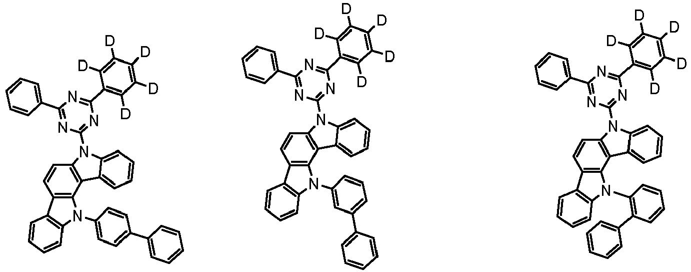

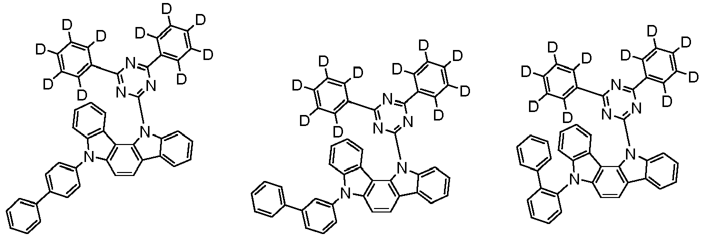

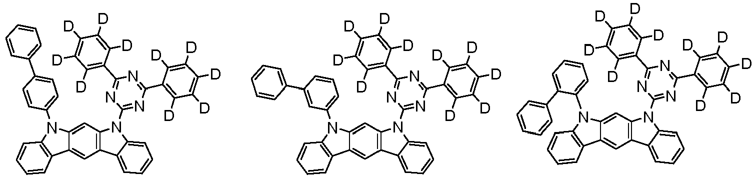

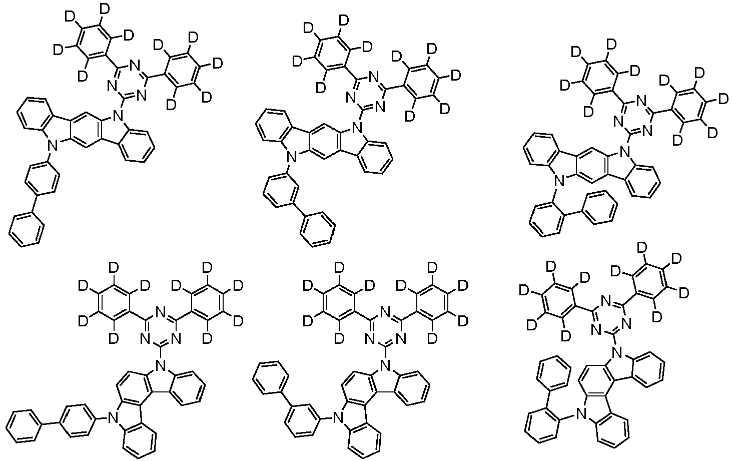

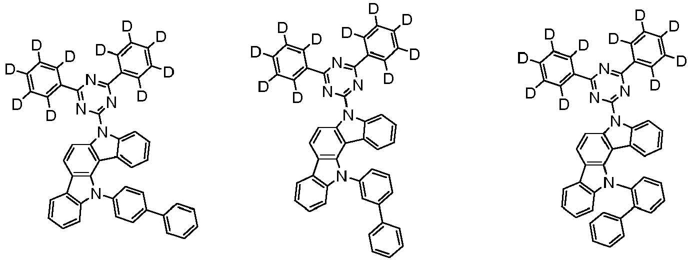

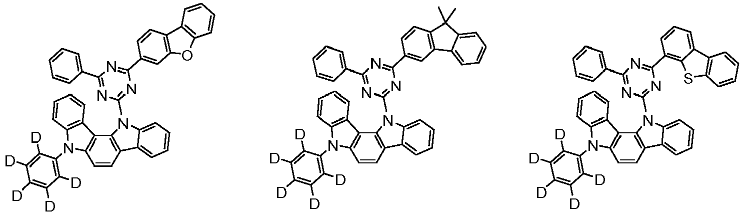

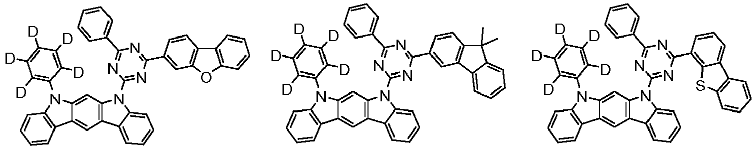

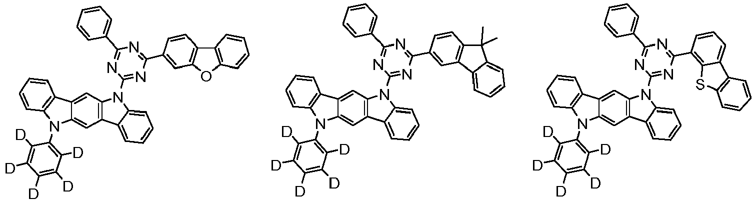

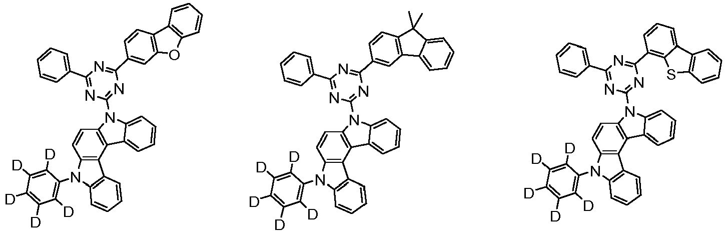

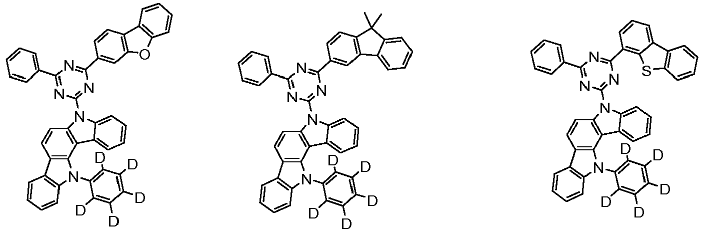

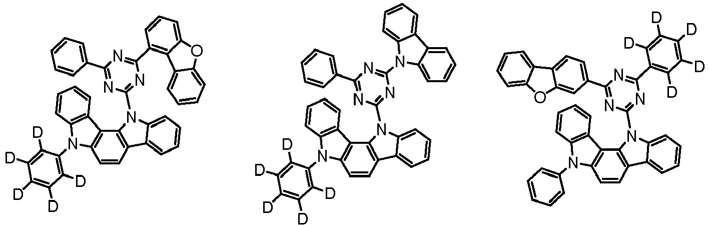

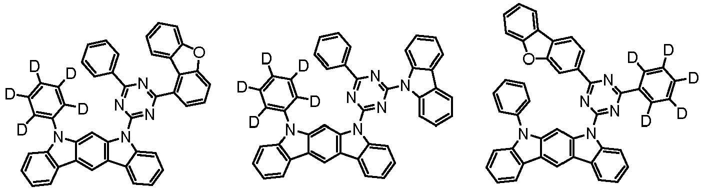

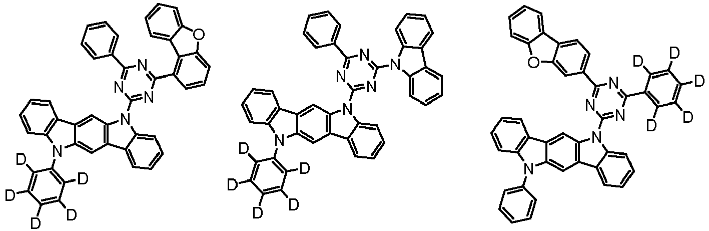

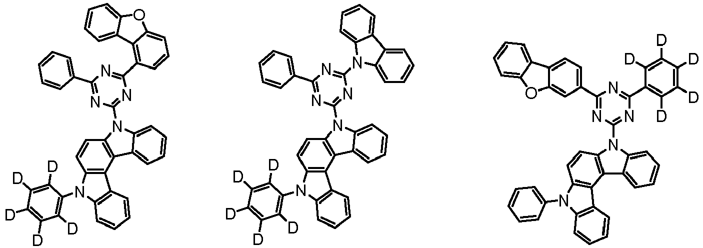

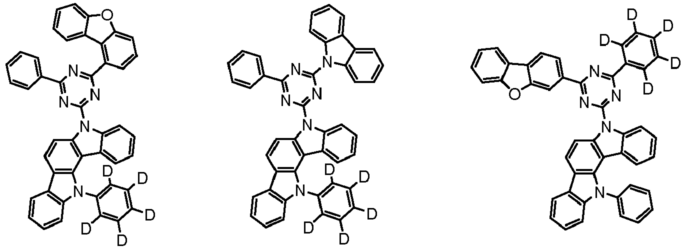

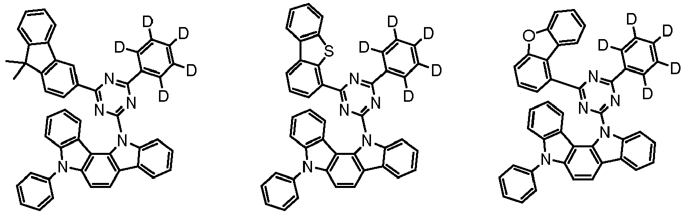

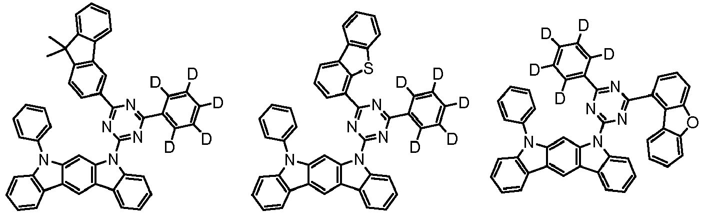

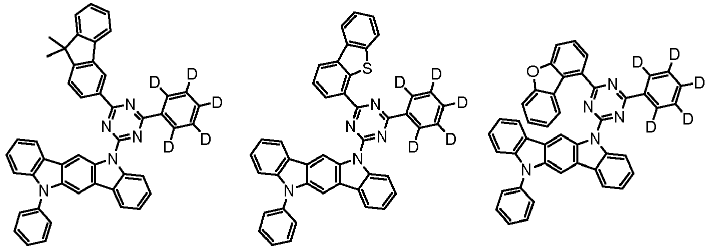

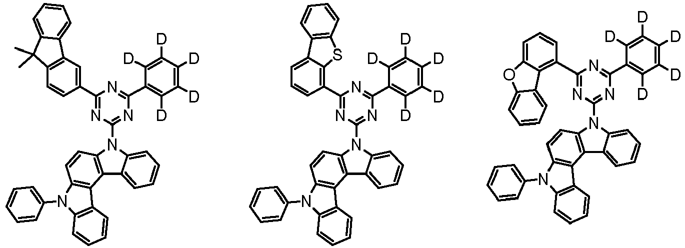

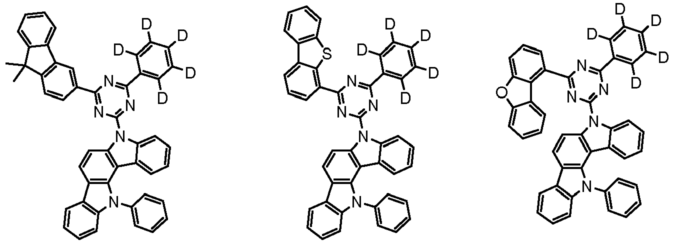

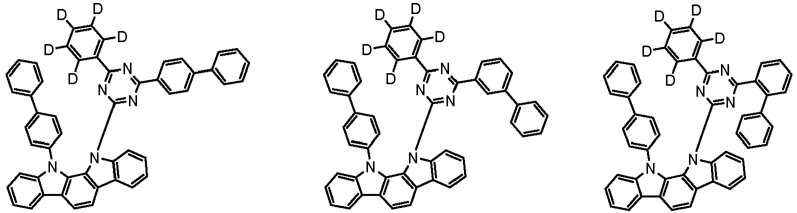

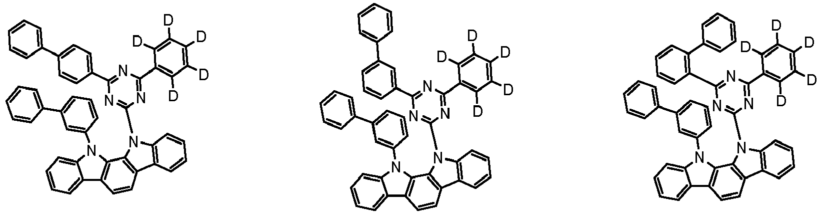

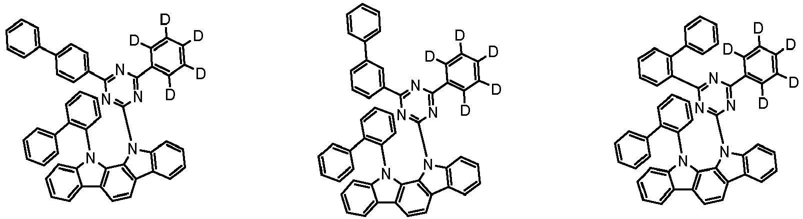

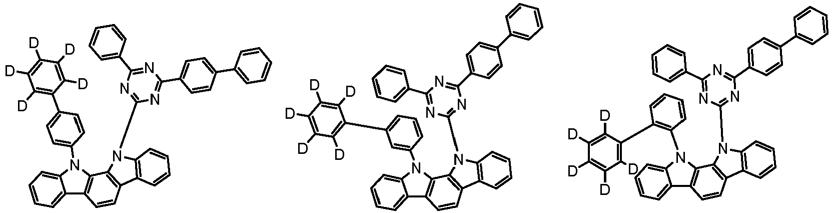

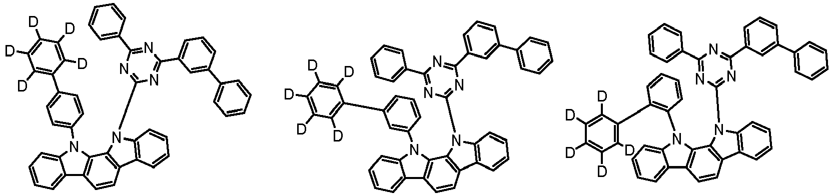

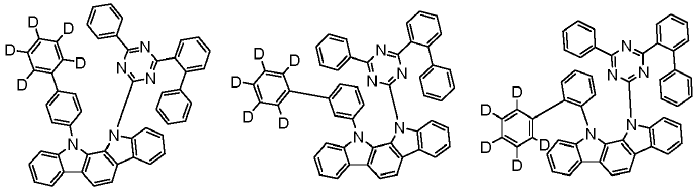

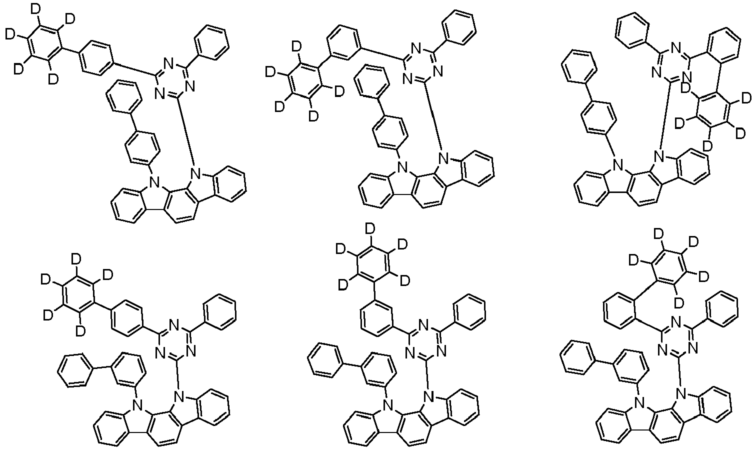

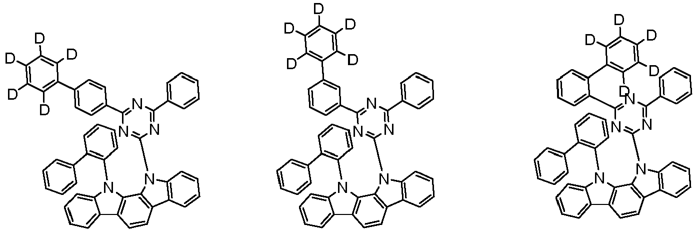

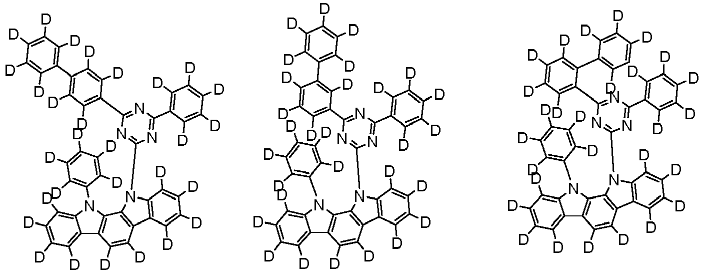

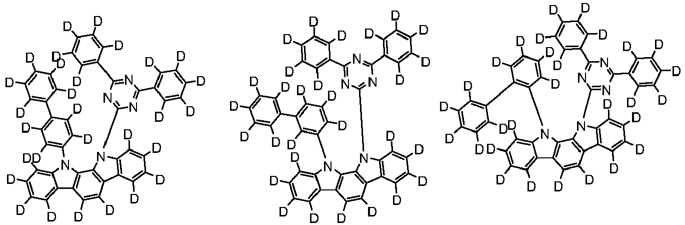

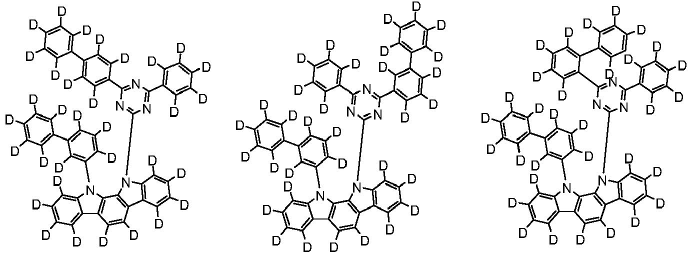

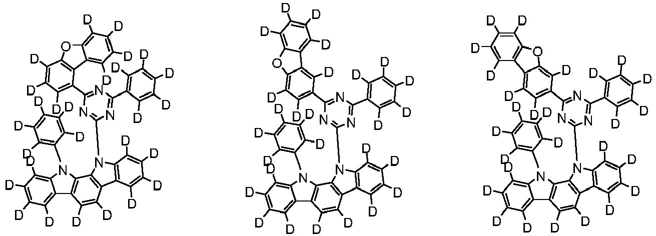

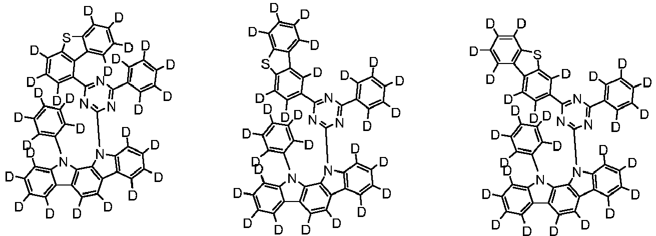

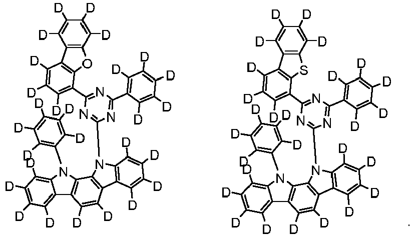

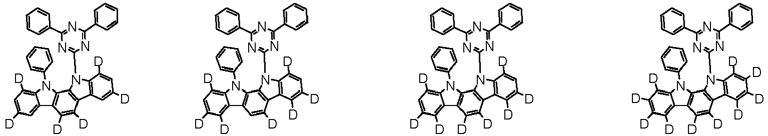

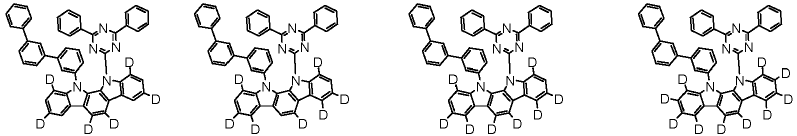

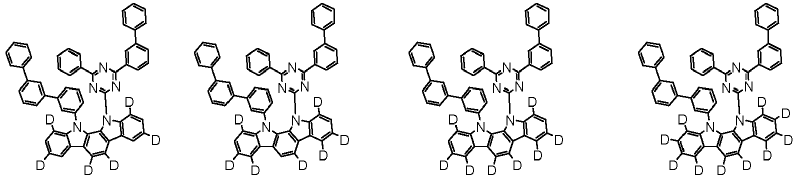

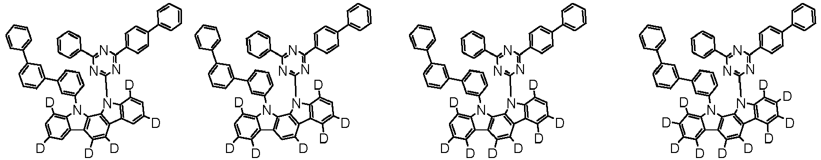

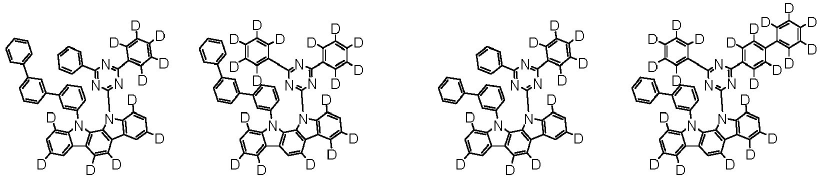

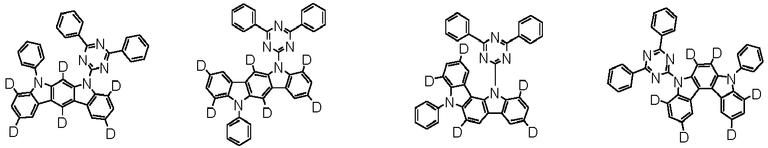

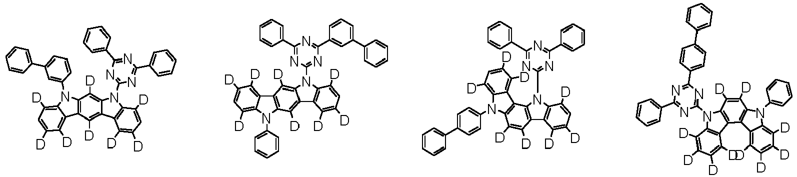

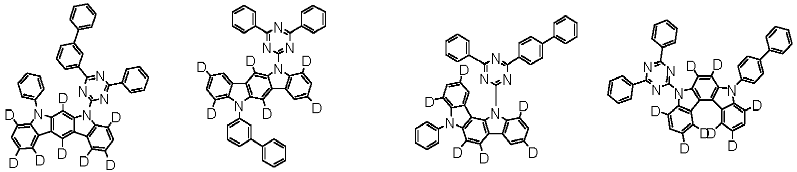

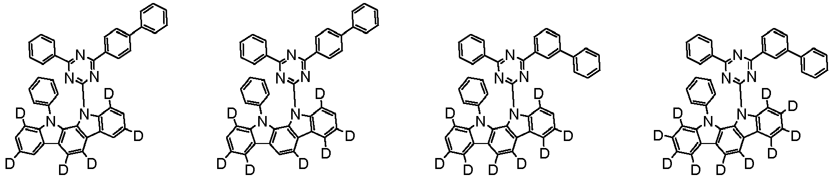

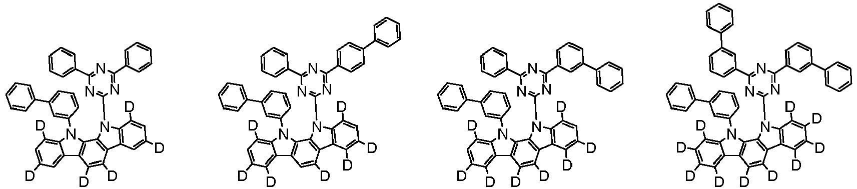

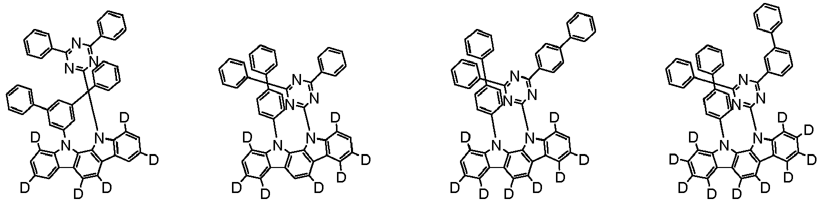

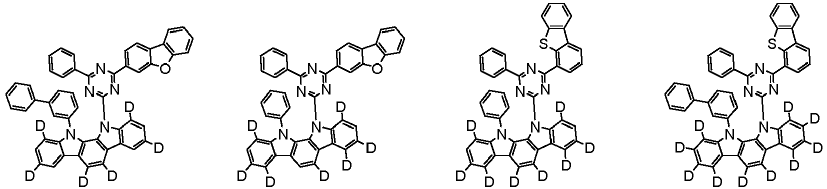

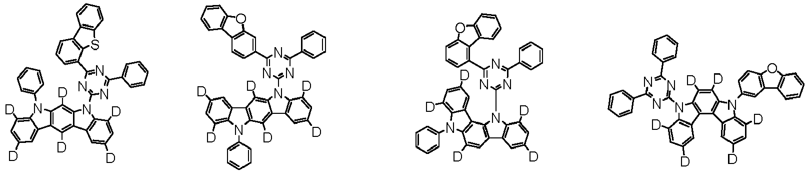

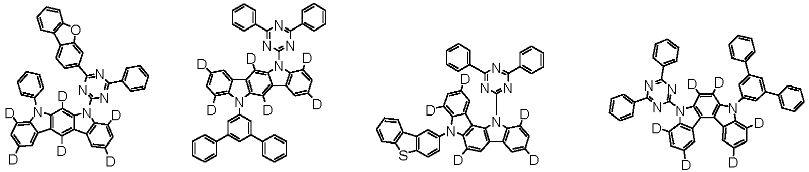

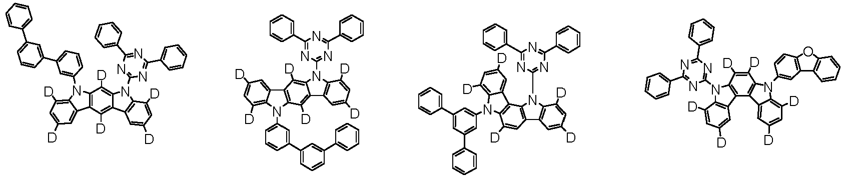

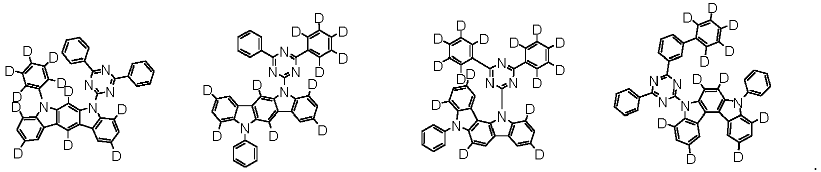

The first compound represented by Chemical Formula 1 may be prepared, for example, by the preparation method as shown in the following Reaction Scheme 1.

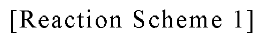

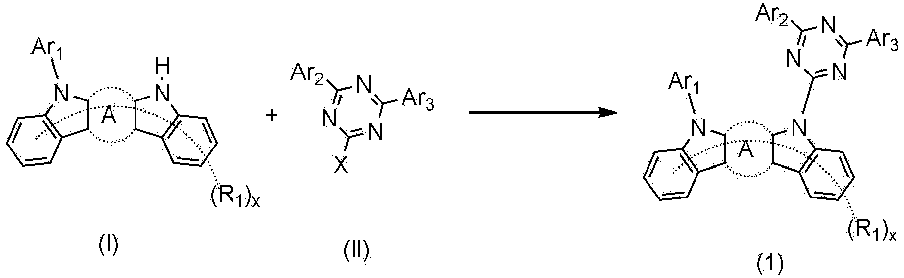

In Reaction Scheme 1, Ar₁ to Ar₃, R1, and x are as defined in Chemical Formula 1, and X is halogen, preferably chloro or bromo.

The Reaction Scheme 1 is an amine substitution reaction, which is preferably carried out in the presence of a palladium catalyst and a base, and a reactive group for the amine substitution reaction can be modified as known in the art. The above preparation method may be further specified in Preparation Examples described hereinafter.

Meanwhile, the Chemical Formula 2 may be preferably represented by any one selected from the group consisting of the following Chemical Formulas 2-1 to 2-6. More preferably, the Chemical Formula 2 may be represented by any one selected from the group consisting of the following Chemical Formulas 2-1 to 2-3: 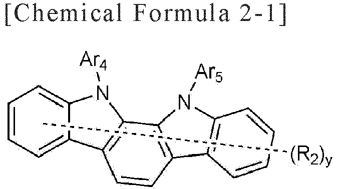 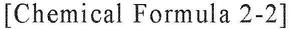 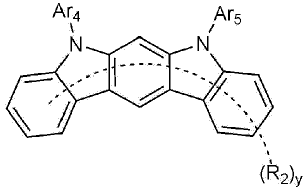 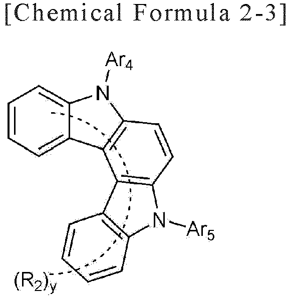 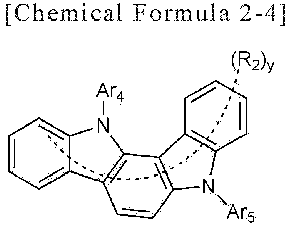 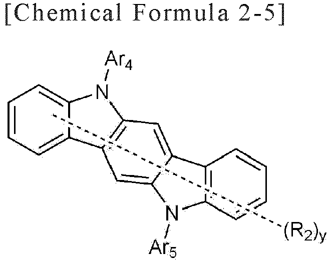 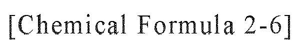 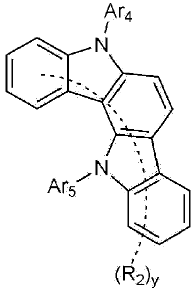 in Chemical Formulas 2-1 to 2-6, Ar₄, Ar₅, R₂, and y are as defined above.

In Chemical Formulas 2, preferably Ar₄ and Ar₅ may be each independently an unsubstituted C₆₋₃₀ aryl, more preferably, Ar₄ and Ar₅ are each independently, phenyl, biphenyl, terphenyl, fluorenyl, 9,9-dimethylfluorenyl, or 9,9-diphenylfluorenyl, each of which is unsubstituted.

Further, in Chemical Formula 2, preferably Ar4 is an unsubstituted C₆₋₃₀ aryl, and Ar₅ may be an unsubstituted C₅₋₆₀ heteroaryl containing one heteroatom selected from the group consisting of N, O and S. More preferably, Ar₄ may be phenyl, biphenyl or terphenyl, each of which is unsubstituted, and Ar₅ may be dibenzofuranyl, dibenzothiophenyl, carbazol-9-yl, or 9-phenyl-9H-carbazolyl, each of which is unsubstituted.

In Chemical Formula 2, preferably, all of R₂ may be hydrogen, and in this case, y may be an integer of 10.

Representative examples of the compound represented by Chemical Formula 2 are as follows:

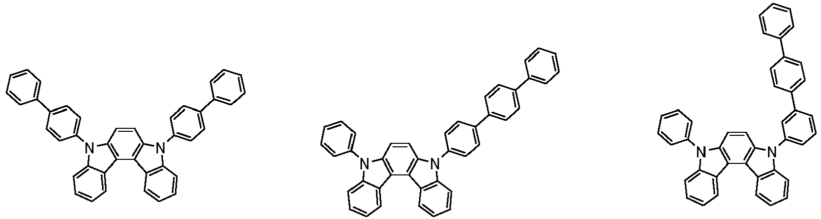

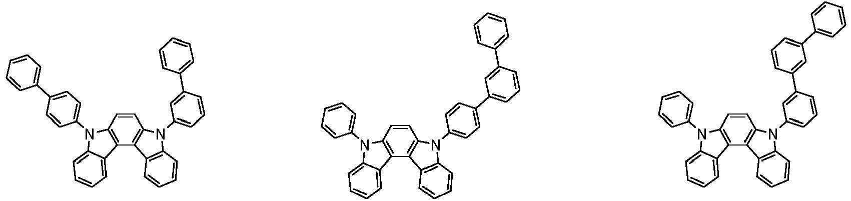

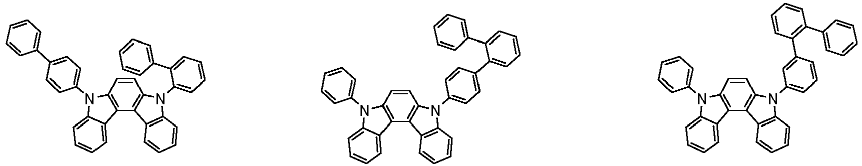

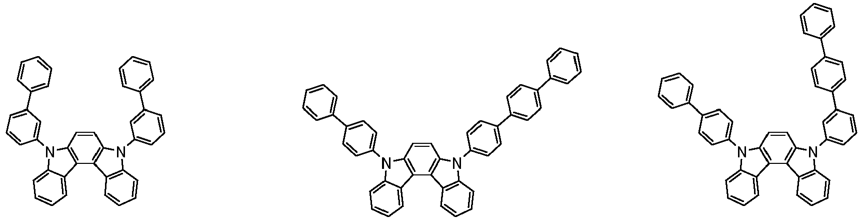

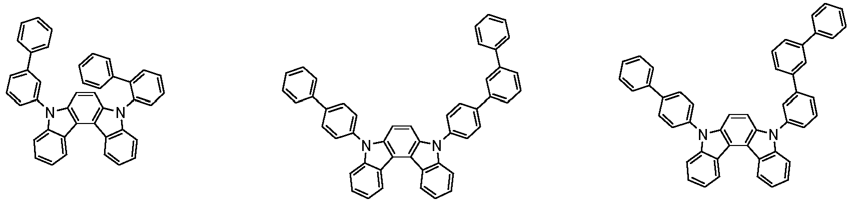

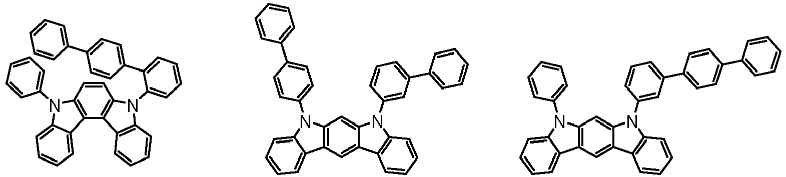

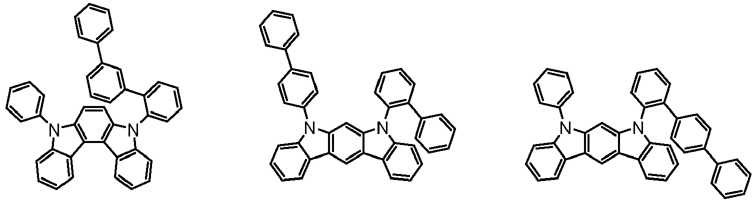

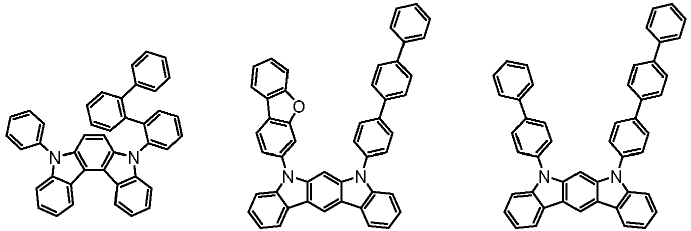

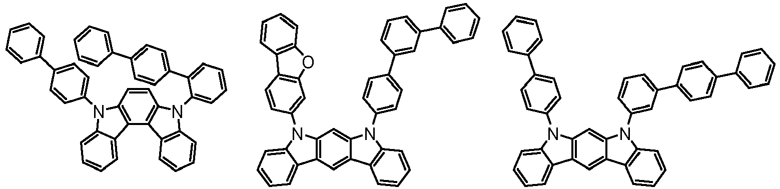

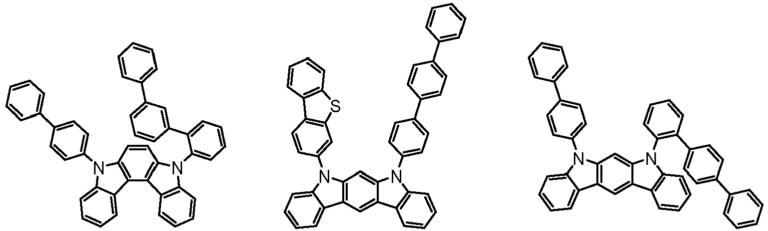

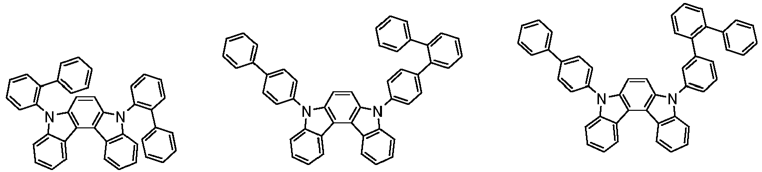

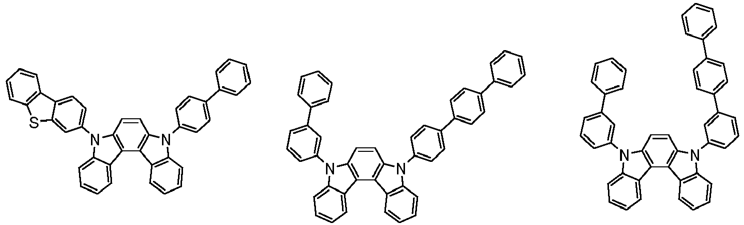

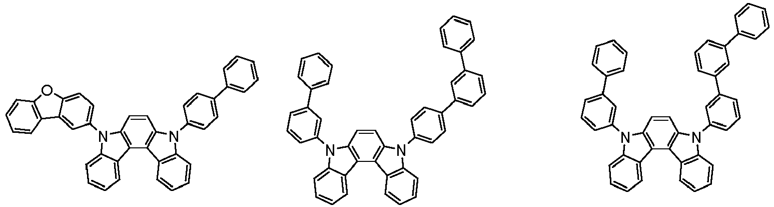

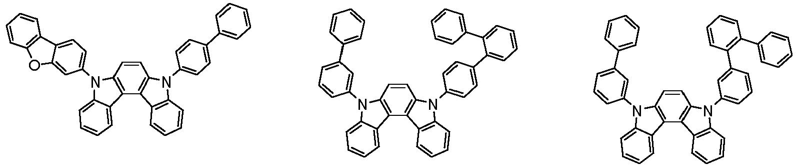

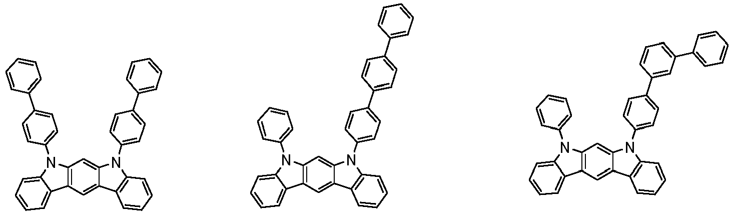

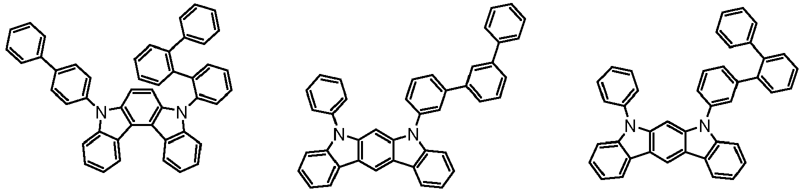

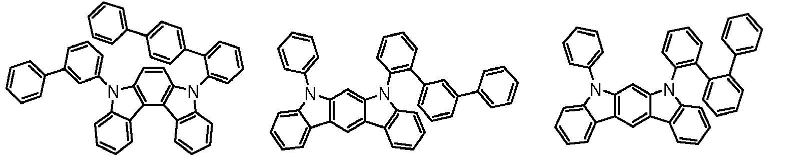

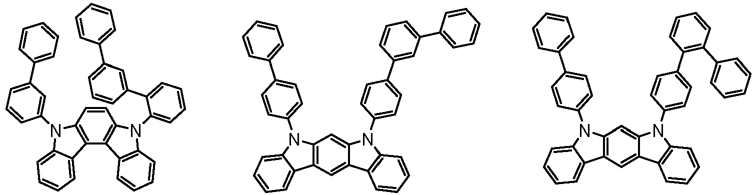

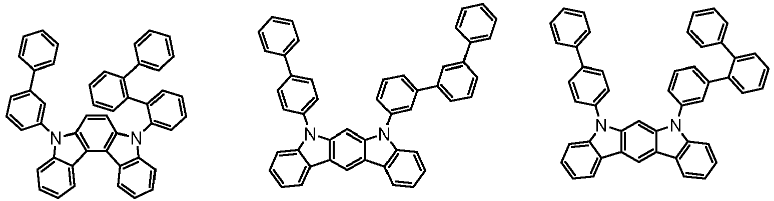

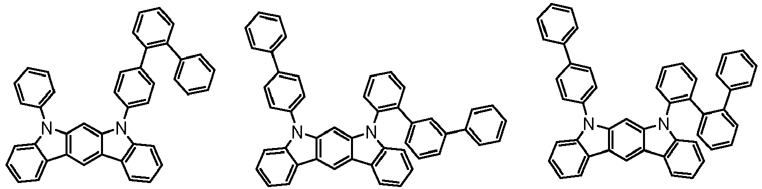

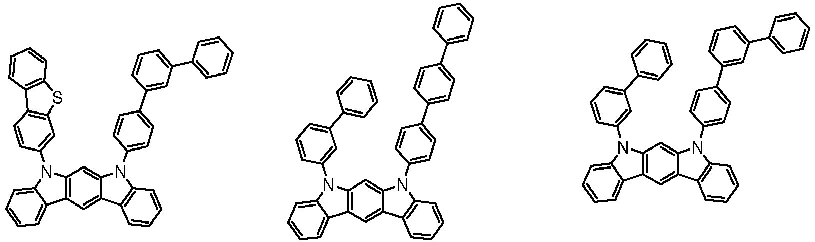

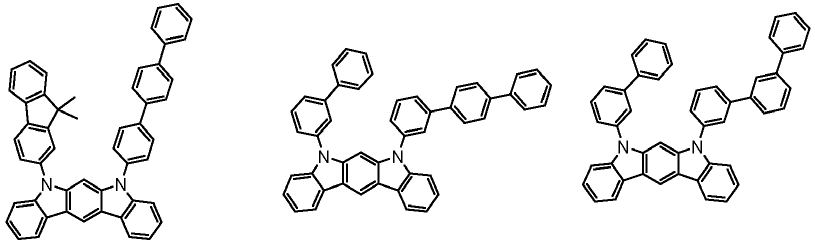

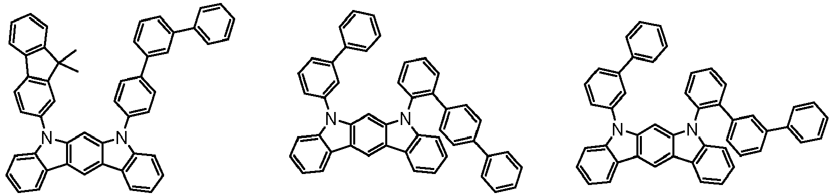

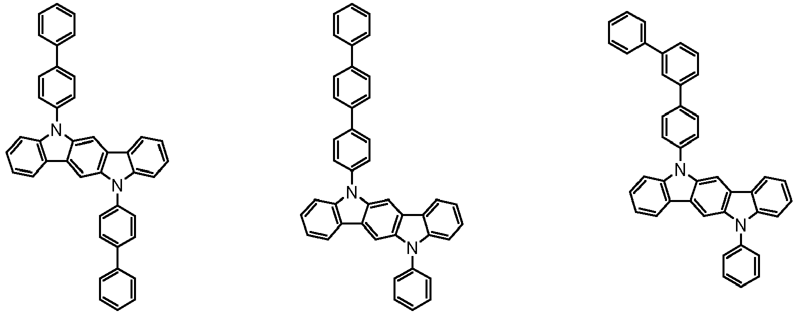

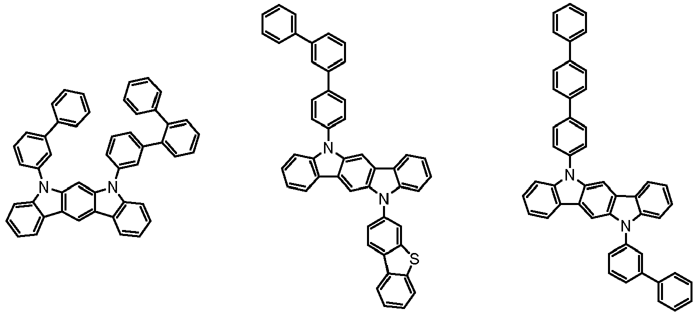

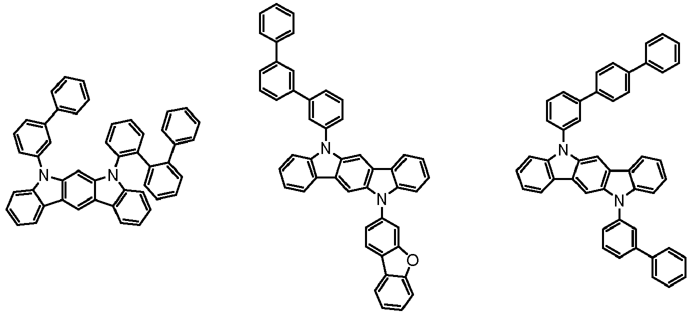

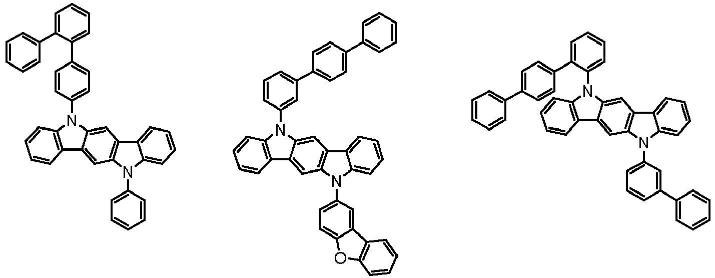

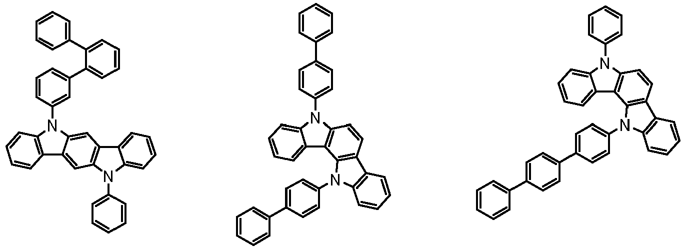

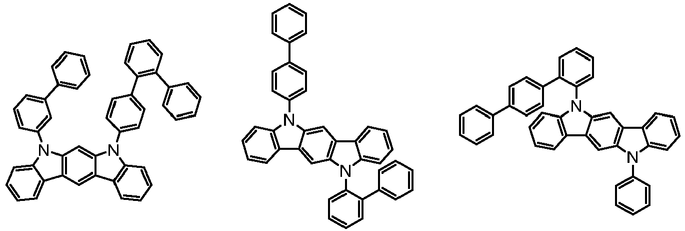

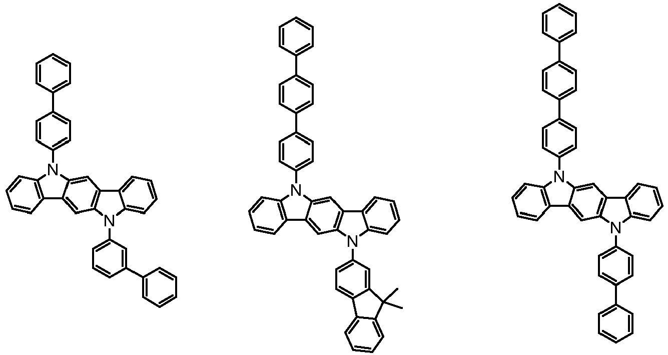

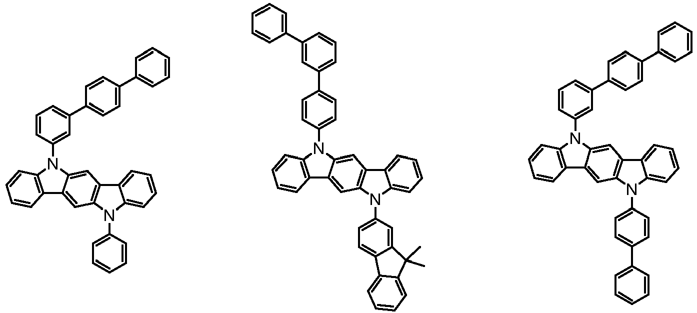

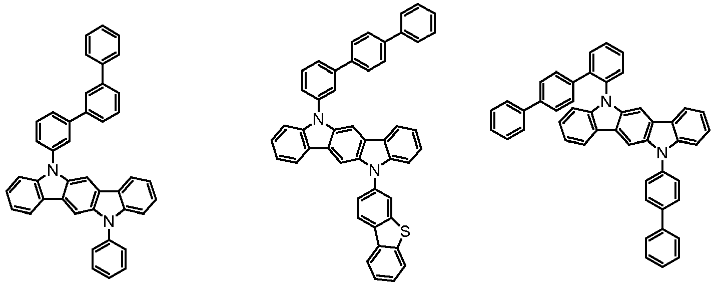

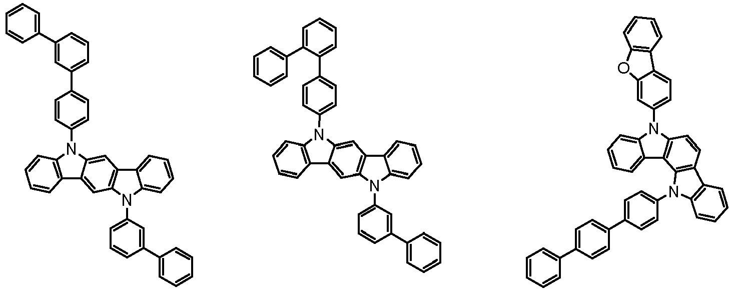

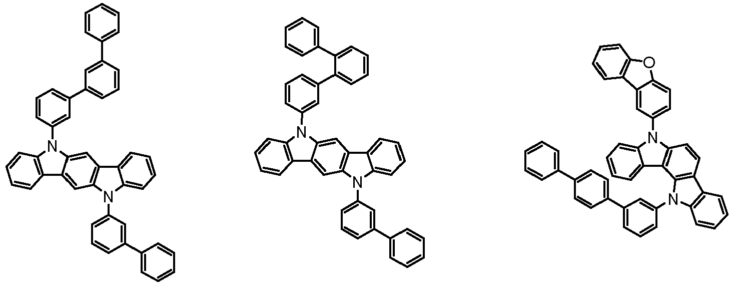

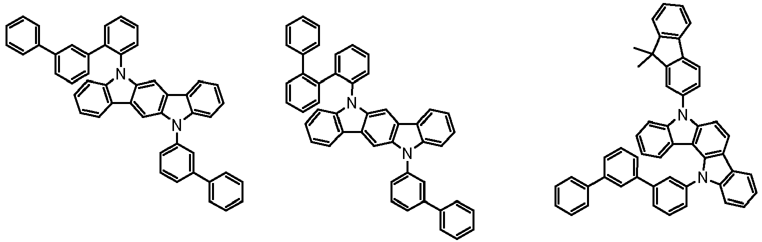

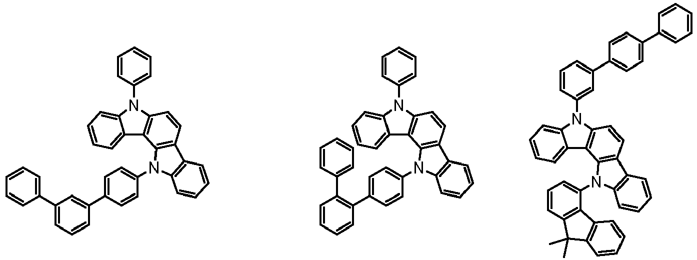

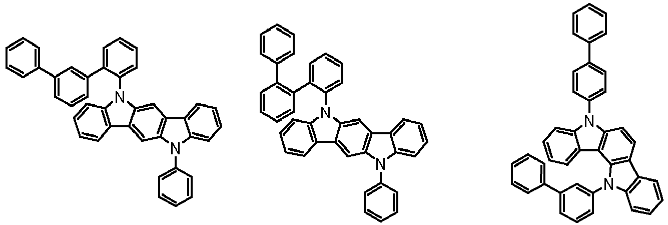

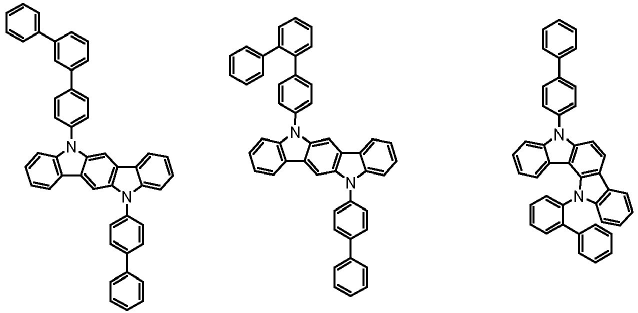

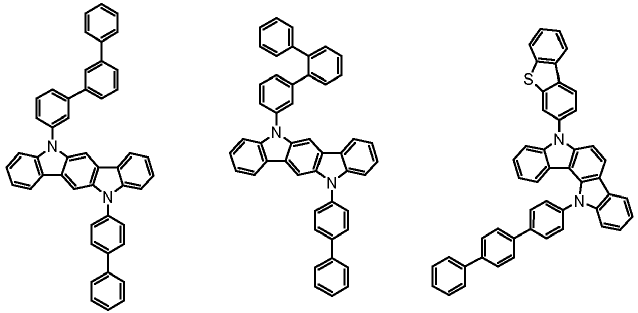

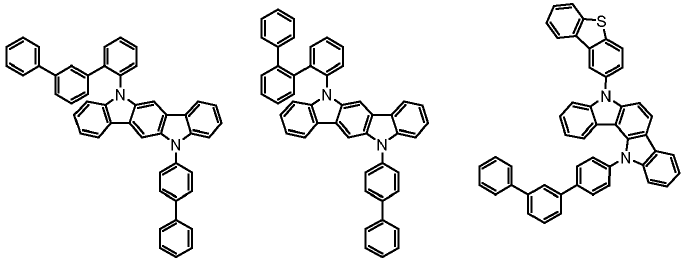

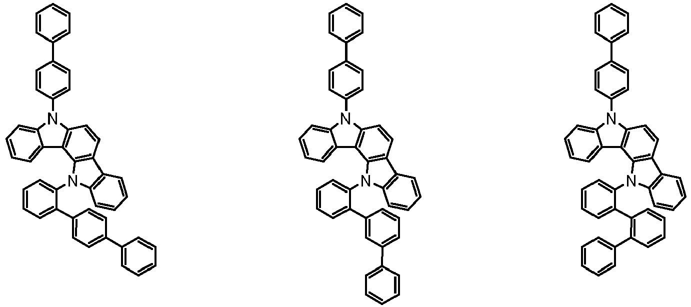

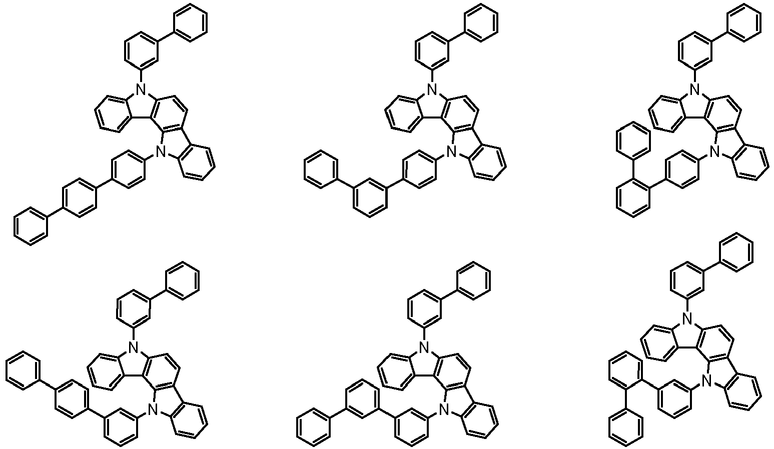

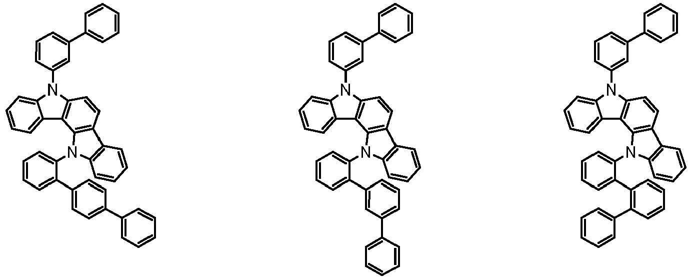

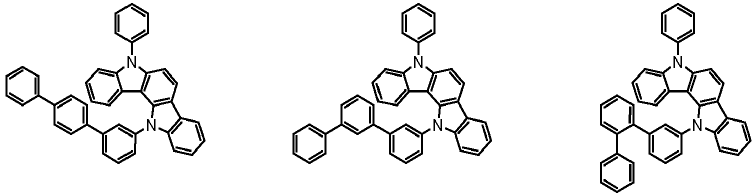

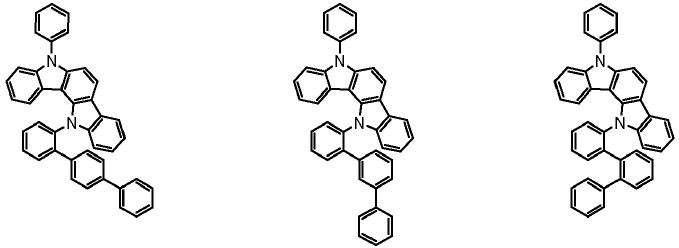

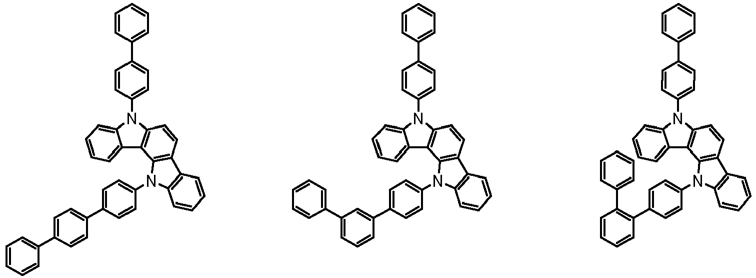

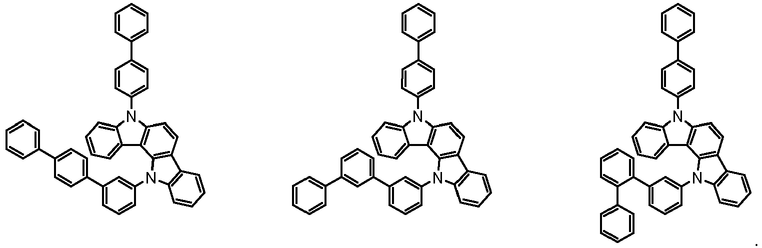

The compound represented by Chemical Formula 2 may be prepared, for example, by the preparation method as shown in the following Reaction Scheme 2. 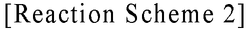 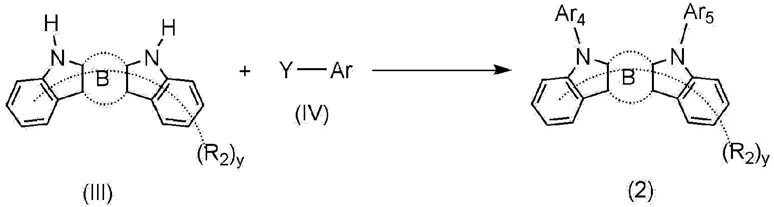 in Reaction Scheme 2, Ar₄, Ar₅, R₂, and y are as defined in Chemical Formula 2, and Y is halogen, preferably chloro or bromo. The Ar is as defined in Ar₄ or Ar₅.

The Reaction Scheme 2 is an amine substitution reaction, which is preferably carried out in the presence of a palladium catalyst and a base, and a reactive group for the amine substitution reaction can be modified as known in the art. The above preparation method may be further specified in the Preparation Examples described hereinafter.

In Reaction Scheme 2, the reaction of Compound (III) and Compound (IV) is shown in one step, but in Compound (2), when Ar₄ and Ar₅ are different from each other, it may be carried out in two steps by changing Ar in Compound (IV) to functional groups corresponding to Ar₄ and Ar₅, respectively. At this time, the order of the reaction steps is not particularly limited.

Preferably, in the light emitting layer, the weight ratio of the first compound represented by Chemical Formula 1 and the second compound represented by Chemical Formula 2 is 10:90 to 90:10, more preferably, 20:80 to 80:20, 30:70 to 70:30, 30:70 to 50:50, or 30:70 to 40:60.

Meanwhile, the light emitting layer may further include a dopant in addition to the host. The dopant material is not particularly limited as long as it is a material used for the organic light emitting device. As an example, an aromatic amine derivative, a styrylamine compound, a boron complex, a fluoranthene compound, a metal complex, and the like can be mentioned. Specific examples of the aromatic amine derivatives include substituted or unsubstituted fused aromatic ring derivatives having an arylamino group, examples thereof include pyrene, anthracene, chrysene, and periflanthene having the arylamino group, and the like. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, examples of the metal complex include an iridium complex, a platinum complex, and the like, but are not limited thereto.

### Electron Transport Layer

The organic light emitting device according to the present disclosure may include an electron transport layer on the light emitting layer, if necessary.

The electron transport layer is a layer that receives the electrons from the electron injection layer formed on the cathode or the cathode and transports the electrons to the light emitting layer, and that suppress the transfer of holes from the light emitting layer, and an electron transport material is suitably a material which may receive electrons well from a cathode and transfer the electrons to a light emitting layer, and has a large mobility for electrons.

Specific examples of the electron transport material include: an Al complex of 8-hydroxyquinoline; a complex including Alq₃; an organic radical compound; a hydroxyflavone-metal complex, and the like, but are not limited thereto. The electron transport layer may be used with any desired cathode material, as used according to a conventional technique. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

### Electron Injection Layer

The organic light emitting device according to the present disclosure may further include an electron injection layer on the light emitting layer (or on the electron transport layer, if the electron transport layer exists).

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film.

Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

### Organic Light Emitting Device

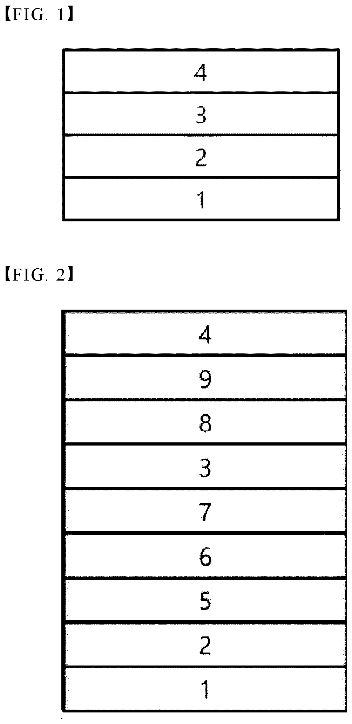

The structure of the organic light emitting device according to the present disclosure is illustrated in FIGS. 1 and 2. FIG. 1 shows an example of an organic light emitting device comprising a substrate 1, an anode 2, a light emitting layer 3, and a cathode 4. FIG. 2 shows an example of an organic light emitting device comprising a substrate 1, an anode 2, a hole injection layer 5, a hole transport layer 6, an electron blocking layer 7, a light emitting layer 3, a hole blocking layer 8, an electron injection and transport layer 9, and a cathode 4.

The organic light emitting device according to the present disclosure can be manufactured by sequentially stacking the above-described structures. In this case, the organic light emitting device may be manufactured by depositing a metal, metal oxides having conductivity, or an alloy thereof on the substrate by using a PVD (physical vapor deposition) method such as a sputtering method or an e-beam evaporation method to form the anode, forming the respective layers described above thereon, and then depositing a material that can be used as the cathode thereon. In addition to such a method, the organic light emitting device may be manufactured by sequentially depositing from the cathode material to the anode material on a substrate in the reverse order of the above-mentioned configuration (WO 2003/012890). Further, the light emitting layer may be formed by subjecting hosts and dopants to a vacuum deposition method and a solution coating method. Herein, the solution coating method means a spin coating, a dip coating, a doctor blading, an inkjet printing, a screen printing, a spray method, a roll coating, or the like, but is not limited thereto.

On the other hand, the organic light emitting device according to the present disclosure may be a front side emission type, a back side emission type, or a double side emission type according to the used material.

Hereinafter, preferred examples of the present disclosure will be provided for a better understanding of the invention. However, these examples are presented for illustrative purposes only, and the scope of the present disclosure is not limited thereto.

### Synthesis Example 1-1: Synthesis of Compound 1-1

### Step 1) Preparation of Intermediate A

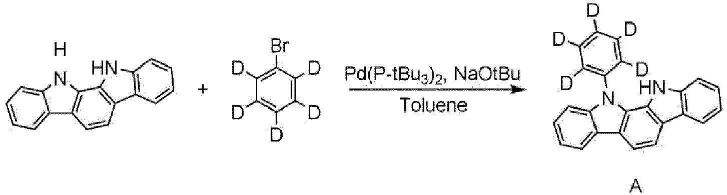

11,12-dihydroindolo[2,3-a]carbazole (15.0g, 58.5mmol), 1-bromobenzene-2,3,4,5,6-d5 (10.4g, 64.4mmol), bis(tri-tert-butylphosphine)palladium(0) (Pd(P-t-Bu₃)₂) (0.6g, 1.2mmol), sodium tert-butoxide (NaOtBu)(8.4g, 87.8mmol), and toluene 500ml were added to a three-necked flask, and the mixture was stirred at reflux under an argon atmosphere for 8 hours. After completion of the reaction, the reaction temperature was cooled to room temperature, then H₂O was added, and the reaction solution was transferred to a separatory funnel, and extracted. The extract was dried over MgSO₄ and concentrated, and then the sample was purified by silica gel column chromatography to give 13.2 g of Intermediate A. (Yield: 67%, MS [M+H] + = 337)

### Step 2) Synthesis of Compound 1-1

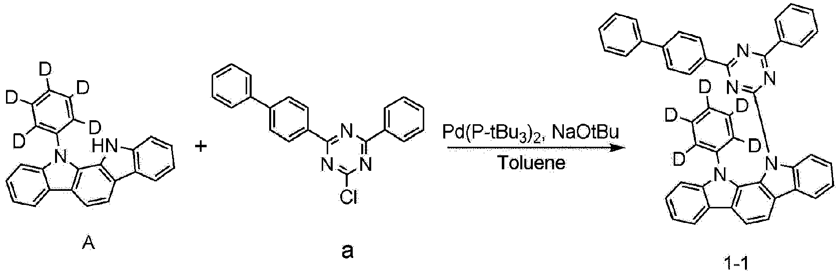

Intermediate A (13.0g, 38.5mmol), Compound a (14.6g, 42.4mmol), bis(tri-tert-butylphosphine)palladium(0) (0.4g, 0.8mmol), sodium tert-butoxide (5.6g, 57.8mmol) and xylene 400ml were added to a three-necked flask, and the mixture was stirred at room temperature under an argon atmosphere for 8 hours. After completion of the reaction, the reaction temperature was cooled to room temperature, then H₂O was added, and the reaction solution was transferred to a separatory funnel, and extracted. The extract was dried over MgSO₄ and concentrated, and then the sample was purified by silica gel column chromatography and then purified by sublimation to give 7.9 g of Compound 1-1. (Yield: 32%, MS [M+H] + = 644)

### Synthesis Example 1-2: Synthesis of Compound 1-2

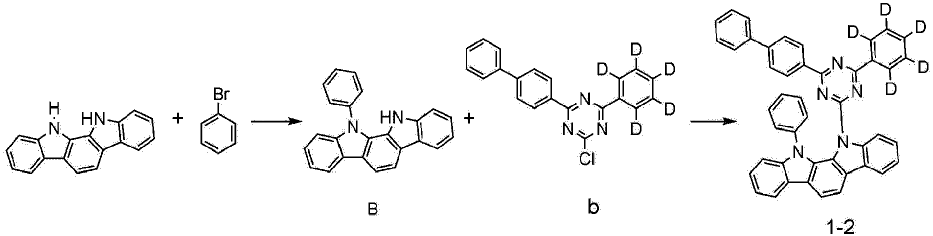

Compound 1-2 was prepared in the same manner as in the Preparation of Compound 1-1, except that in step 1 of Synthesis Example 1-1, bromobenzene was used instead of 1-bromobenzene-2,3,4,5,6-d5 to prepare Intermediate B, and in step 2, Compound b was used instead of Compound a. (MS[M+H]⁺ = 644)

### Synthesis Example 1-3: Synthesis of Compound 1-3

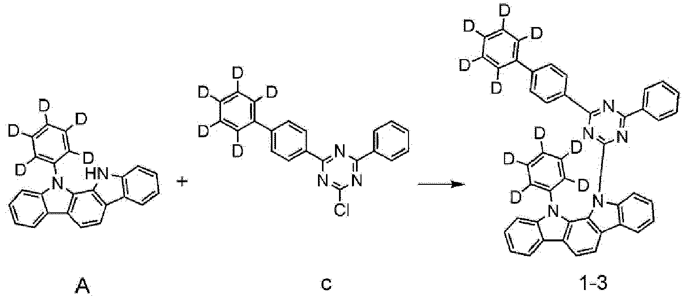

Compound 1-3 was prepared in the same manner as in the Preparation of Compound 1-1, except that in step 2 of Synthesis Example 1-1, Compound c was used instead of Compound a. (MS[M+H]⁺ = 649)

### Synthesis Example 1-4: Synthesis of Compound 1-4

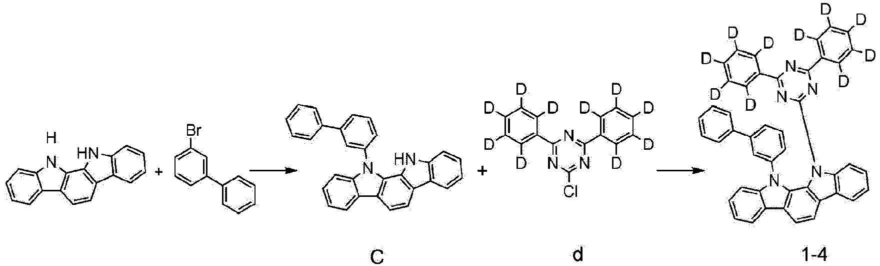

Compound 1-4 was prepared in the same manner as in the Preparation of Compound 1-1, except that in step 1 of Synthesis Example 1-1,3-bromo-1,1'-biphenyl was used instead of 1-bromobenzene-2,3,4,5,6-d5 to prepare Intermediate C, and in step 2, Compound d was used instead of Compound a. (MS[M+H]⁺ = 649)

### Synthesis Example 1-5: Synthesis of Compound 1-5

### Step 1) Synthesis of Intermediate 1-5-1

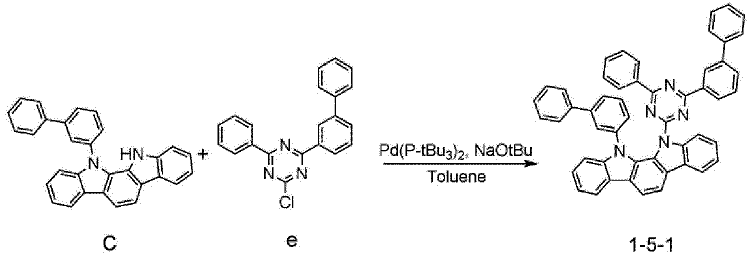

Intermediate C (15.0g, 36.7mmol), Compound e (13.9g, 40.4mmol), bis(tri-tert-butylphosphine)palladium(0) (0.4g, 0.7mmol), sodium tert-butoxide (5.3g, 55.1mmol) and xylene 400ml were added to a three-necked flask, and the mixture was stirred at room temperature under an argon atmosphere for 8 hours. After completion of the reaction, the reaction temperature was cooled to room temperature, then H₂O was added, and the reaction solution was transferred to a separatory funnel, and extracted. The extract was dried over MgSO₄ and concentrated, and then the sample was purified by silica gel column chromatography to give 17.1 g of Compound 1-5-1. (Yield: 65%, MS [M+H]⁺ = 715)

### Step 2) Synthesis of Compound 1-5

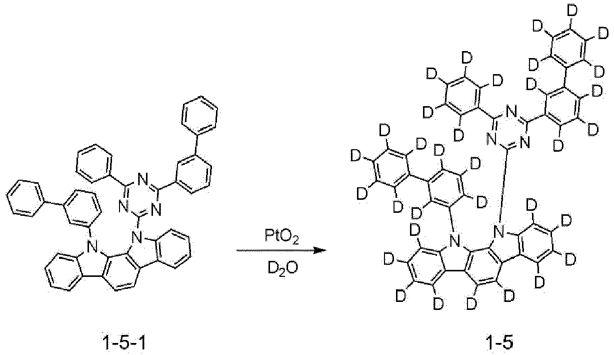

Compound 1-5-1 (10.0g, 14.0mmol), PtO₂ (1.0g, 4.2mmol), and 70ml of D2O were added to a shaker tube, and then the tube was sealed and heated at 250°C and 600 psi for 12 hours. After completion of the reaction, chloroform was added, and the reaction solution was transferred to a separatory funnel, and extracted. The extract was dried over MgSO₄ and concentrated, and then the sample was purified by silica gel column chromatography and purified by sublimation to give 4.4 g of Compound 1-5. (Yield: 42%, deuterium substitution rate: 82%, MS[M+H]⁺ = 749).

### Synthesis Example 1-6: Synthesis of Compound 1-6

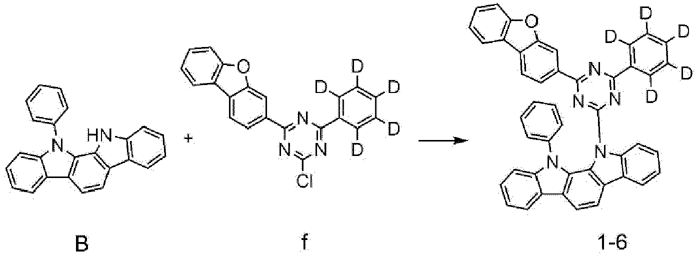

Compound 1-6 was prepared in the same manner as in the Preparation of Compound 1-1, except that in step 2 of Synthesis Example 1-1, Intermediate B was used instead of Intermediate A, and Compound f was used instead of Compound a. (MS[M+H]⁺ = 658)

### Synthesis Example 1-7: Synthesis of Compound 1-7

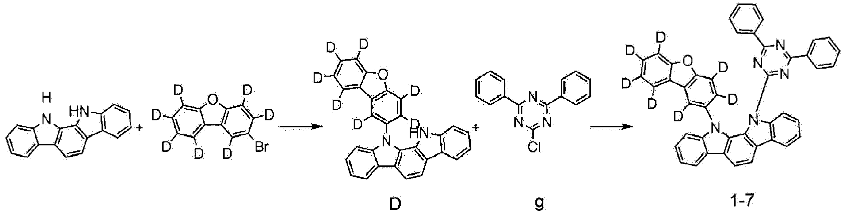

Compound 1-7 was prepared in the same manner as in the Preparation of Compound 1-1, except that in step 1 of Synthesis Example 1-1, 2-bromodibenzo[b,d] furan-1,3,4,6,7,8,9-d7 was used instead of 1-bromobenzene-2,3,4,5,6-d5 to prepare Intermediate D, and in step 2, Compound g was instead of Compound a. (MS[M+H]⁺ = 660)

### Synthesis Example 1-8: Synthesis of Compound 1-8

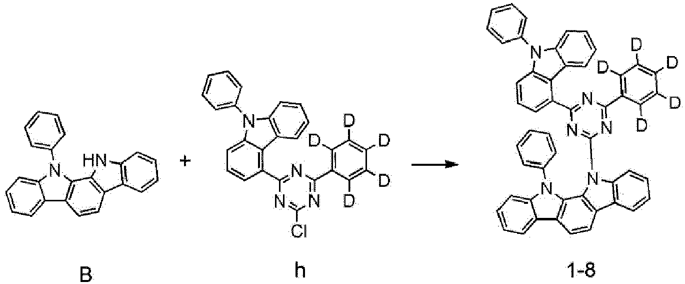

Compound 1-8 was prepared in the same manner as in the Preparation of Compound 1-1, except that in step 2 of Synthesis Example 1-1, Intermediate B was used instead of Intermediate A and Compound h was used instead of Compound a. (MS[M+H]⁺ = 733)

### Synthesis Example 1-9: Synthesis of Compound 1-9

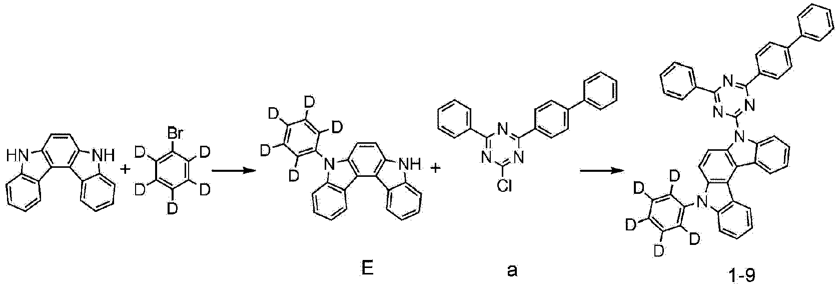

Compound 1-9 was prepared in the same manner as in the Preparation of Compound 1-1, except that in step 1 of Synthesis Example 1-1, 5,8-dihydroindolo[2,3-c]carbazole was used instead of 11,12-dihydroindolo[2,3-a]carbazole. (MS[M+H]⁺ = 644)

### Synthesis Example 1-10: Synthesis of Compound 1-10

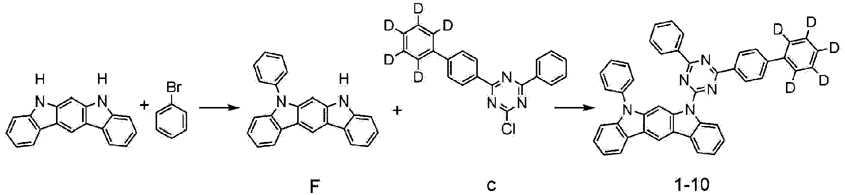

Compound 1-10 was prepared in the same manner as in the Preparation of Compound 1-1, except that in step 1 of Synthesis Example 1-1, 5,7-dihydroindolo[2,3-b]carbazole was used instead of 11,12-dihydroindolo[2,3-a]carbazole and bromobenzene was used instead of 1-bromobenzene-2,3,4,5,6-d5 to prepare Intermediate F, and in step 2, Compound c was used instead of Compound a. (MS[M+H]⁺ = 644)

### Synthesis Example 1-11: Synthesis of Compound 1-11

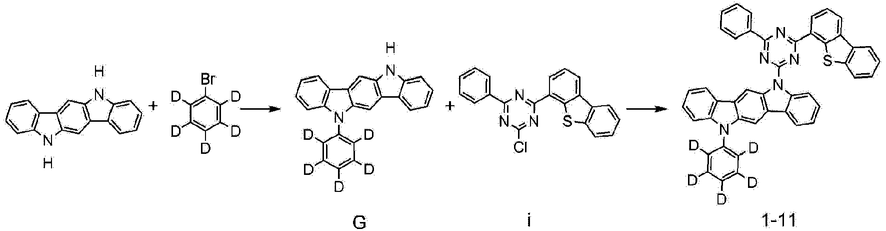

Compound 1-11 was prepared in the same manner as in the Preparation of Compound 1-1, except that in step 1 of Synthesis Example 1-1, 5,11-dihydroindolo[3,2-b]carbazole was used instead of 11,12-dihydroindolo[2,3-a]carbazole to prepare Intermediate G, and in step 2, Compound i was used instead of Compound a. (MS[M+H]⁺ = 674)

### Synthesis Example 1-12: Synthesis of Compound 1-12

### Step 1) Synthesis of Intermediate H

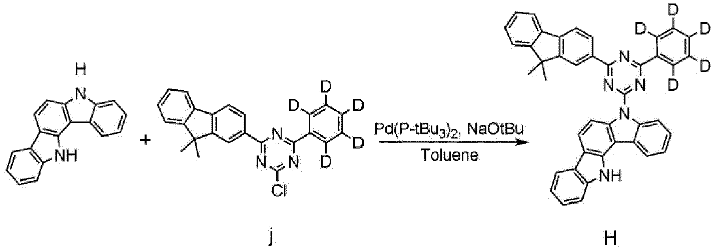

5,12-Dihydroindolo[3,2-a]carbazole (10.0g, 39.0mmol), Compound j (16.7g, 42.9mmol), bis(tri-tert-butylphosphine)palladium(0) (0.4g, 0.8mmol), sodium tert-butoxide (5.6g, 58.5mmol) and toluene 400ml were added to a three-necked flask, and the mixture was stirred at room temperature under an argon atmosphere for 8 hours. After completion of the reaction, the reaction temperature was cooled to room temperature, then H₂O was added, and the reaction solution was transferred to a separatory funnel, and extracted. The extract was dried over MgSO₄ and concentrated, and then the sample was purified by silica gel column chromatography to give 16.9 g of Intermediate H. (Yield: 71%, MS [M+H]⁺ = 608)

### Step 2) Synthesis of Compound 1-12

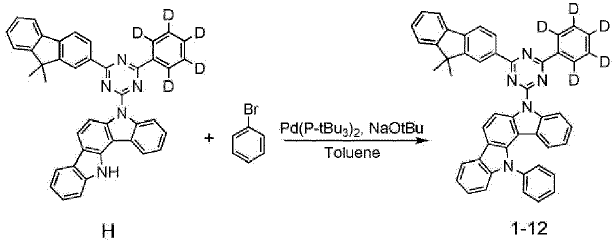

Intermediate H (15.0g, 24.6mmol), bromobenzene (4.3g, 27.1mmol), bis(tri-tert-butylphosphine)palladium(0) (0.3g, 0.5mmol), sodium tert-butoxide (3.6g, 37.0mmol) and xylene 250ml were added to a three-necked flask, and the mixture was stirred at room temperature under an argon atmosphere for 8 hours. After completion of the reaction, the reaction temperature was cooled to room temperature, then H₂O was added, and the reaction solution was transferred to a separatory funnel, and extracted. The extract was dried over MgSO₄ and concentrated, and then the sample was purified by silica gel column chromatography and purified by sublimation to give 5.4g g of Compound 1-12. (Yield: 32%, MS [M+H]⁺ = 684)

### Synthesis Example 1-13: Synthesis of Compound 1-13

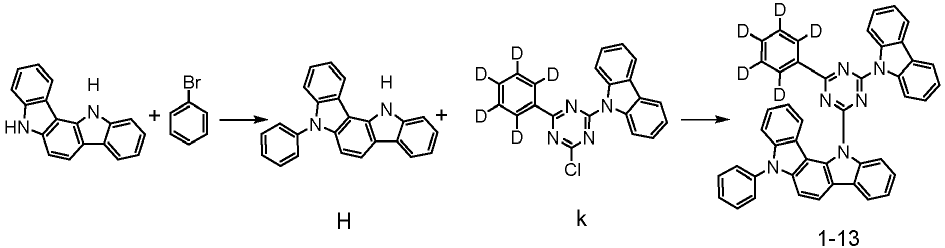

Compound 1-13 was prepared in the same manner as in the Preparation of Compound 1-1, except that in step 1 of Synthesis Example 1-1, 5,12-dihydroindolo[3,2-a]carbazole was used instead of 11,12-dihydroindolo[2,3-a]carbazole and bromobenzene was used instead of 1-bromobenzene-2,3,4,5,6-d5 to prepare Intermediate I, and in step 2, Compound k was used instead of Compound a. (MS[M+H]⁺ = 657)

### Synthesis Example 1-14: Synthesis of Compound 1-14

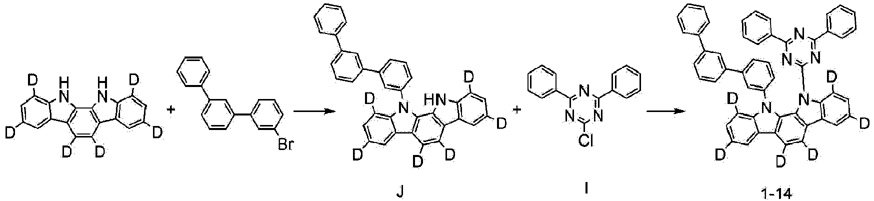

Compound 1-14 was prepared in the same manner as in the Preparation of Compound 1-1, except that in step 1 of Synthesis Example 1-1, 11,12-dihydroindolo[2,3-a]carbazole-1,3,5,6,8,10-d6 was used instead of 11,12-dihydroindolo[2,3-a]carbazole and 3-bromo-1,1':3',1"-terphenylwas used instead of 1-bromobenzene-2,3,4,5,6-d5 to prepare Intermediate J, and in step 2, Compound 1 was used instead of Compound a. (MS[M+H]⁺ = 723)

### Synthesis Example 1-15: Synthesis of Compound 1-15

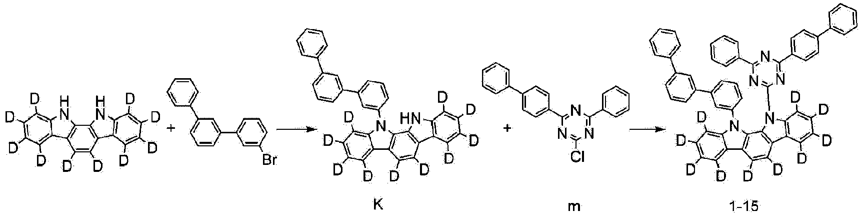

Compound 1-15 was prepared in the same manner as in the Preparation of Compound 1-1, except that in step 1 of Synthesis Example 1-1, 11,12-dihydroindolo[2,3-a]carbazole-1,2,3,4,5,6,7,8,9,10-d10 was used instead of 11,12-dihydroindolo[2,3-a]carbazole and 3-bromo-1,1':3',1"-terphenyl used instead of 1-bromobenzene-2,3,4,5,6-d5 to prepare Intermediate K, and in step 2, Compound m was used instead of Compound a. (MS[M+H]⁺ = 803)

### Synthesis Example 2-1: Synthesis of Compound 2-1

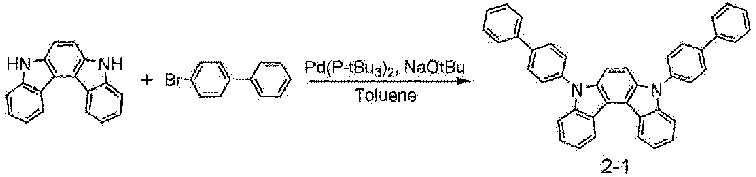

5,8-dihydroindolo[2,3-c]carbazole (15.0g, 58.5mmol) and 4-bromo-1,1'-biphenyl (30.0g, 128.8mmol) were added to 300ml of toluene under a nitrogen atmosphere, and the mixture was stirred and refluxed. Then, sodium tert-butoxide (16.9g, 175.6mmol) and bis(tri-tert-butylphosphine) palladium(0) (0.9g, 1.8mmol) were added thereto. After the reaction for 12 hours, the mixture was cooled to room temperature, and the organic layer was separated using chloroform and water, and the organic layer was distilled. This was again dissolved in chloroform, washed twice with water, and then the organic layer was separated, anhydrous magnesium sulfate was added thereto, stirred, filtered, and the filtrate was distilled under reduced pressure. The concentrated compound was purified by silica gel column chromatography and then purified by sublimation to give 9.8 g of Compound 2-1. (Yield: 30%, MS: [M+H]⁺ = 562)

### Synthesis Example 2-2: Synthesis of Compound 2-2

### Step 1) Synthesis of Intermediate 2-1-1

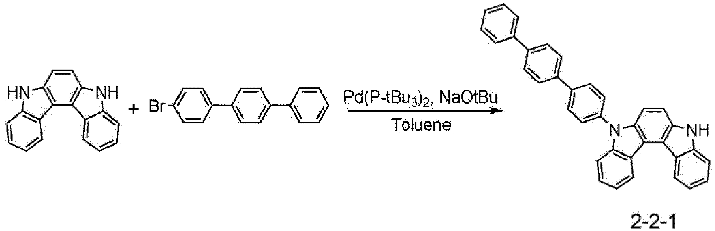

5,8-Dihydroindolo[2,3-c]carbazole (15.0g, 58.5mmol) and 4-bromo-1,1':4',1"-terphenyl (19.9g, 64.4mmol) were added to 300ml of toluene under nitrogen atmosphere, and the mixture was stirred and refluxed. Then, sodium tert-butoxide (8.4g, 87.8mmol) and bis(tri-tert-butylphosphine)palladium(0) (0.9g, 1.8mmol) were added thereto. After the reaction for 11 hours, the mixture was cooled to room temperature, and the organic layer was separated using chloroform and water, and the organic layer was distilled. This was again dissolved in chloroform, washed twice with water, and then the organic layer was separated, anhydrous magnesium sulfate was added thereto, stirred, filtered, and the filtrate was distilled under reduced pressure. The concentrated compound was purified by silica gel column chromatography to give 19.3 g of Intermediate 2-2-1. (Yield: 68%, MS: [M+H]⁺ = 486)

### Step 2) Synthesis of Compound 2-2

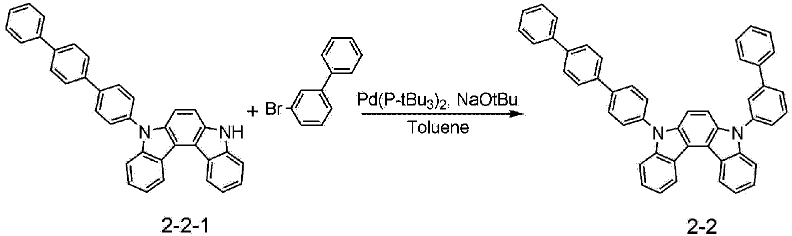

Intermediate 2-2-1 (15.0g, 31.0mmol) and 3-bromo-1,1'-biphenyl (7.9g, 34.0mmol) were added to 300ml of toluene under a nitrogen atmosphere, and the mixture was stirred and refluxed. Then, sodium tert-butoxide (4.5 g, 46.4 mmol) and bis(tri-tert-butylphosphine) palladium(0) (0.5g, 0.9mmol) were added thereto. After the reaction for 7 hours, the mixture was cooled to room temperature, and the organic layer was separated using chloroform and water, and the organic layer was distilled. This was again dissolved in chloroform, washed twice with water, and then the organic layer was separated, anhydrous magnesium sulfate was added thereto, stirred, filtered, and the filtrate was distilled under reduced pressure. The concentrated compound was purified by silica gel column chromatography and then purified by sublimation to give 9.5 g of Compound 2-2. (Yield: 48%, MS: [M+H]⁺ = 638)

### Synthesis Example 2-3: Synthesis of Compound 2-3

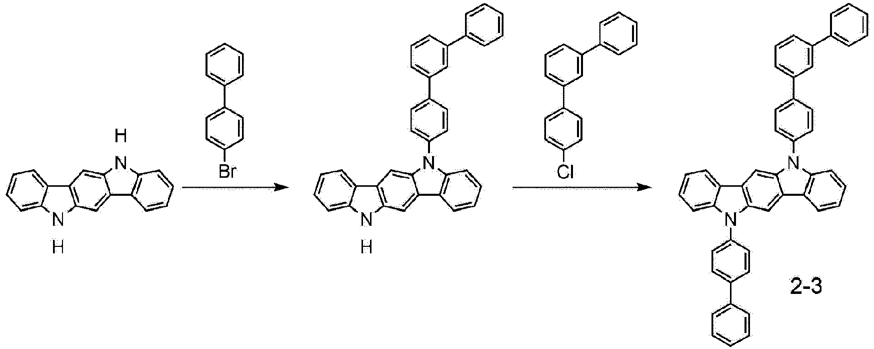

Compound 2-3 was prepared in the same manner as in the Preparation of Compound 2-2, except that in step 1 of Synthesis Example 2-2, 5,11-dihydroindolo[3,2-b]carbazole was used instead of 5,8-dihydroindolo[2,3-c]carbazole and 4-bromo-1,1'-biphenyl was used instead of 4-bromo-1,1':4',1"-terphenyl, and in step 2, 4-chloro-1,1':3',1"-terphenyl was used instead of 3-bromo-1,1'-biphenyl. (MS[M+H]⁺ = 638)

### Synthesis Example 2-4: Synthesis of Compound 2-4

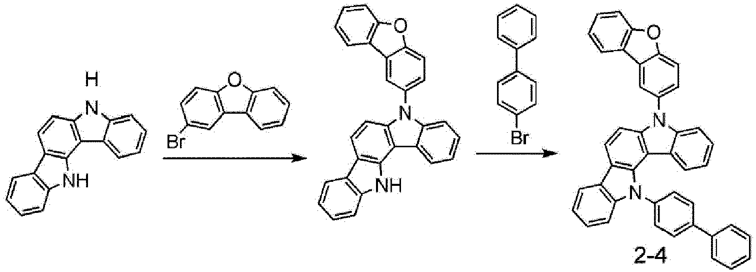

Compound 2-4 was prepared in the same manner as in the Preparation of Compound 2-2, except that in step 1 of Synthesis Example 2-2, 5,12-dihydroindolo[3,2-a]carbazole was used instead of 5,8-dihydroindolo[2,3-c]carbazole and 2-bromodibenzo[b,d]furan was used instead of 4-bromo-1,1':4',1"-terphenyl, and in step 2, 4-bromo-1,1'-biphenyl was used instead of 3-bromo-1,1'-biphenyl. (MS[M+H]⁺ = 576)

### Example 1

A glass substrate on which a thin film of ITO (indium tin oxide) was coated in a thickness of 1,400 Å was put into distilled water containing the detergent dissolved therein and ultrasonically washed. In this case, the detergent used was a product commercially available from Fisher Co. and the distilled water was one which had been twice filtered by using a filter commercially available from Millipore Co. The ITO was washed for 30 minutes, and ultrasonic washing was then repeated twice for 10 minutes by using distilled water. After the washing with distilled water was completed, the substrate was ultrasonically washed with isopropyl alcohol, acetone, and methanol solvent, and dried, after which it was transported to a plasma cleaner. Then, the substrate was cleaned with oxygen plasma for 5 minutes, and then transferred to a vacuum evaporator.

On the ITO transparent electrode thus prepared, the following HT-A and 5 wt.% of PD were thermally vacuum deposited to a thickness of 100Å to form a hole injection layer, and then only the HT-A material was deposited to a thickness of 1150Å to form a hole transport layer. The following HT-B was thermally vacuum deposited thereon to a thickness of 450Å as an electron blocking layer. Then, vacuum deposition was performed to a thickness of 400 Å by using Compound 1-1 and Compound 2-1 in a weight ratio of 40:60 as the host of the light emitting layer, and 8 wt.% of GD of the host as a dopant. Then, the following ET-A was vacuum-deposited to a thickness of 50 Å as a hole blocking layer. Then, the following ET-B and Liq were thermally vacuum-deposited in a ratio of 2: 1 to a thickness of 250 Å on the hole blocking layer, and LiF and magnesium were then vacuum deposited in a ratio of 1: 1 to a thickness of 30 Å to sequentially form an electron transport layer and an electron injection layer. Magnesium and silver were deposited in a ratio of 1: 4 to a thickness of 160 Å on the electron injection layer to form a cathode, thereby completing the manufacture of an organic light emitting device.

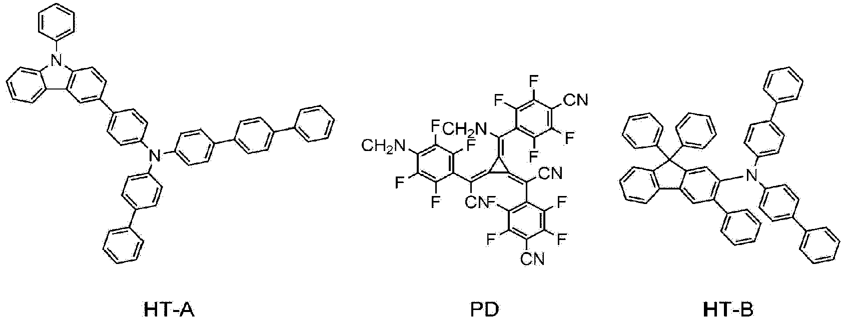

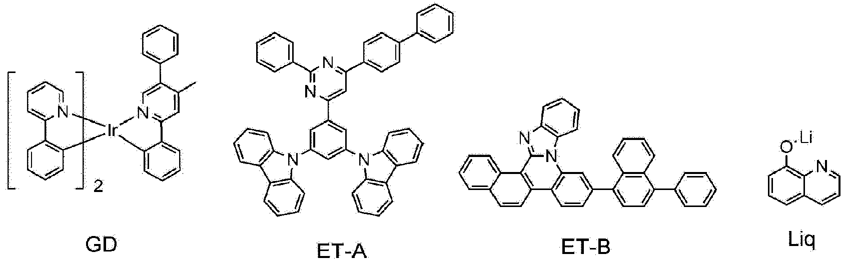

### Examples 2 to 23, and Comparative Examples 1 to 20

The organic light emitting devices of Examples 2 to 23 and Comparative Examples 1 to 20 were respectively manufactured in the same manner as in Example 1, except that the host material was changed as shown in Table 1 below. In this case, when a mixture of two kinds of compounds was used as the host, the parenthesis means the weight ratio between the host compounds.

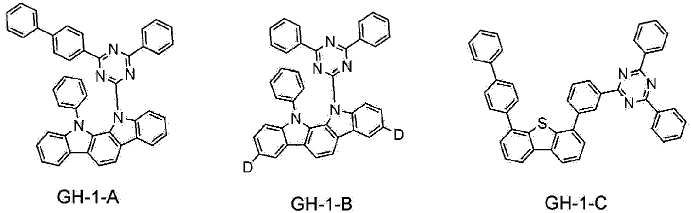

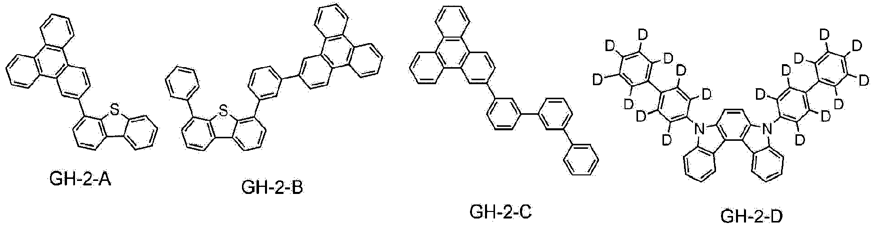

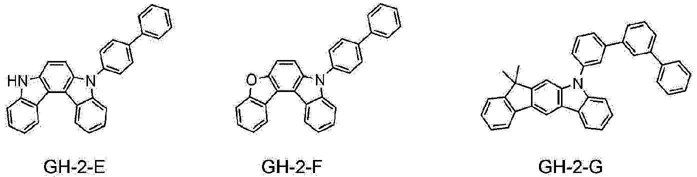

### Experimental Example: Evaluation of Device Characteristics

The voltage, efficiency and lifetime (T95) were measured by applying a current to the organic light emitting devices manufactured in Examples and Comparative Examples, and the results are shown in Table 1 below. At this time, the voltage and efficiency were measured at a current density of 10 mA/cm², and T95 means the time (hr) required for the luminance to be reduced to 95% of the initial luminance at a current density of 20 mA/cm².

**[Table 1]**

| | Host material | @ 10mA/cm² | | @ 20m A/cm ² |
|---|---|---|---|---|
| | | Volta ge (V) | Effic iency (cd/A ) | Lifet ime (T95, hr) |
| Example 1 | Compound 1-1: Compound 2-1 (40:60) | 4.01 | 64.1 | 132 |
| Example 2 | Compound 1-2:Compound 2-1 (40:60) | 4.00 | 64.1 | 135 |
| Example 3 | Compound 1-3:Compound 2-1 (40:60) | 4.01 | 64.2 | 143 |
| Example 4 | Compound 1-4:Compound 2-1 (40:60) | 4.08 | 62.2 | 138 |
| Example 5 | Compound 1-5:Compound 2-1 (40:60) | 4.02 | 64.3 | 152 |
| Example 6 | Compound 1-6:Compound 2-1 (40:60) | 4.07 | 64.7 | 145 |
| Example 7 | Compound 1-7:Compound 2-1 (40:60) | 4.08 | 64.2 | 130 |
| Example 8 | Compound 1-8:Compound 2-1 (40:60) | 4.05 | 64.1 | 128 |
| Example 9 | Compound 1-9:Compound 2-1 (40:60) | 4.17 | 63.7 | 133 |
| Example10 | Compound 1-10:Compound 2-1 (40:60) | 4.16 | 64.2 | 130 |
| Example11 | Compound 1-11:Compound 2-1 (40:60) | 4.13 | 63.8 | 131 |
| Example12 | Compound 1-12:Compound 2-1 (40:60) | 4.19 | 64.7 | 127 |
| Example13 | Compound 1-13:Compound 2-1 (40:60) | 4.21 | 62.9 | 137 |
| Example14 | Compound 1-14:Compound 2-1 (40:60) | 4.03 | 65.1 | 138 |
| Example15 | Compound 1-15:Compound 2-1 (40:60) | 4.07 | 64.9 | 141 |
| Example16 | Compound 1-1:Compound 2-2 (40:60) | 4.03 | 64.6 | 135 |
| Example17 | Compound 1-2:Compound 2-2 (40:60) | 4.02 | 64.5 | 130 |
| Example18 | Compound 1-2:Compound 2-3 (40:60) | 4.07 | 63.1 | 122 |
| Example19 | Compound 1-3:Compound 2-3 (40:60) | 4.08 | 63.2 | 128 |
| Example20 | Compound 1-4:Compound 2-3 (40:60) | 4.09 | 63.3 | 125 |
| Example21 | Compound 1-5:Compound 2-4 (40:60) | 4.11 | 64.1 | 141 |
| Example22 | Compound 1-6:Compound 2-4 (40:60) | 4.18 | 64.0 | 130 |
| Example23 | Compound 1-10:Compound 2-4 (30:70) | 4.21 | 63.1 | 119 |
| Comparative Example 1 | Compound 1-1 | 4.71 | 54.1 | 91 |
| Comparative Example 2 | Compound 1-6 | 4.67 | 52.7 | 84 |
| Comparative Example 3 | Compound 2-1 | 7.62 | 7.5 | 5 |
| Comparative Example 4 | Compound 2-3 | 7.82 | 5.8 | 3 |
| Comparative Example 5 | Compound GH-1-A:Compound 2-1 (40:60) | 4.02 | 62.5 | 82 |
| Comparative Example 6 | Compound GH-1-B:Compound 2-1 (40:60) | 4.23 | 61.7 | 71 |
| Comparative Example 7 | Compound GH-1-C:Compound 2-1 (40:60) | 4.65 | 56.6 | 85 |
| Comparative Example 8 | Compound GH-1-A:Compound 2-2 (40:60) | 4.07 | 62.3 | 86 |
| Comparative Example 9 | Compound GH-1-B:Compound 2-3 (40:60) | 4.35 | 58.2 | 65 |
| Comparative Example 10 | Compound 1-1:Compound GH-2-A (40:60) | 4.51 | 56.6 | 90 |
| Comparative Example 11 | Compound 1-1:Compound GH-2-B (40:60) | 4.57 | 57.7 | 92 |
| Comparative Example 12 | Compound 1-1:Compound GH-2-C (40:60) | 4.48 | 55.7 | 93 |
| Comparative Example 13 | Compound 1-6:Compound GH-2-B (40:60) | 4.51 | 56.5 | 103 |
| Comparative Example 14 | Compound GH-1-A:Compound GH-2-D (40:60) | 4.02 | 62.5 | 82 |
| Comparative Example 15 | Compound 1-1:Compound GH-2-E (40:60) | 5.51 | 31.0 | 21 |
| Comparative Example 16 | Compound 1-1:Compound GH-2-F (40:60) | 5.23 | 42.3 | 40 |
| Comparative Example 17 | Compound 1-1:Compound GH-2-G (40:60) | 5.07 | 44.5 | 51 |
| Comparative Example 18 | Compound GH-1-A | 4.71 | 54.2 | 60 |
| Comparative Example 19 | Compound GH-1-B | 4.83 | 50.3 | 53 |
| Comparative Example 20 | Compound GH-2-A | 7.76 | 6.7 | 8 |

In the present disclosure, both the first compound represented by Chemical Formula 1 and the second compound represented by Chemical Formula 2 have an indolocarbazole skeleton. Here, the first compound has a strong ability to transport electrons by containing a triazine group, and the second compound has a strong ability to transport holes, and thus has properties suitable for using a mixture of two materials as a host. In particular, since both materials have an indolocarbazole structure, they are well mixed with each other and are advantageous for the formation of exciplex, and are advantageous in effectively transferring energy to a dopant. At this time, the first compound becomes an anion and the second compound becomes a cation to form an exciplex, and the first compound, which becomes an anion state, has a more unstable state. By substituting five or more deuteriums therein, the first compound has a reduced vibration energy even in an anion state and a more stable energy.

Consequently, as can be seen in Table 1, Examples 1 to 23 in which the first compound represented by Chemical Formula 1 and the second compound represented by Chemical Formula 2 were used together as a light emitting layer of an organic electroluminescent device in the present disclosure exhibited significantly improved low voltage, high efficiency, and long life characteristics.

## Claims

1. An organic light emitting device comprising:
an anode, a cathode, and a light emitting layer interposed between the anode and the cathode,
wherein the light emitting layer comprises a first compound represented by the following Chemical Formula 1 and a second compound represented by the following Chemical Formula 2. 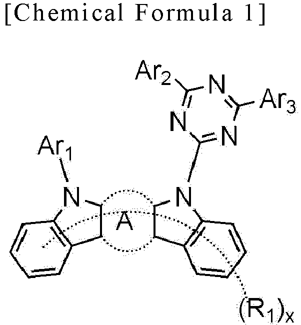
in the Chemical Formula 1,
A is a benzene ring fused with two adjacent pentagonal rings, respectively,
Ar₁ to Ar₃ are each independently a substituted or unsubstituted C₆₋₆₀ aryl; or a substituted or unsubstituted C₅₋₆₀ heteroaryl containing any one or more heteroatoms selected from the group consisting of N, O and S,
each R₁ is independently hydrogen; deuterium; halogen; cyano; a substituted or unsubstituted C₁₋₆₀ alkyl; a substituted or unsubstituted C₁₋₆₀ alkoxy; a substituted or unsubstituted C₂₋₆₀ alkenyl; a substituted or unsubstituted C₂₋₆₀ alkynyl; a substituted or unsubstituted C₃₋₆₀ cycloalkyl; a substituted or unsubstituted C₆₋₆₀ aryl; or a substituted or unsubstituted C₂₋₆₀ heteroaryl containing any one or more heteroatoms selected from the group consisting of N, O and S,
x is an integer of 1 to 10,
provided that at least one of Ar₁ to Ar₃, and R₁ is substituted with at least one deuterium, or at least one of R₁ is deuterium, and the first compound comprises at least 5 deuteriums, 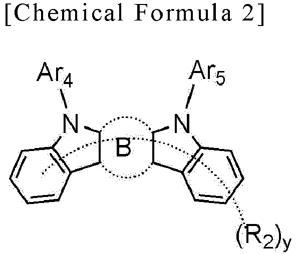
in the Chemical Formula 2,
B is a benzene ring fused with two adjacent pentagonal rings, respectively,
Ar4 is a substituted or unsubstituted C₆₋₆₀ aryl,
Ar₅ is a substituted or unsubstituted C₆₋₆₀ aryl; or a substituted or unsubstituted C₅₋₆₀ heteroaryl containing one heteroatom selected from the group consisting of N, O and S,
each R₂ is independently hydrogen; deuterium; halogen; cyano; a substituted or unsubstituted C₁₋₆₀ alkyl; a substituted or unsubstituted C₁₋₆₀ alkoxy; a substituted or unsubstituted C₂₋₆₀ alkenyl; a substituted or unsubstituted C₂₋₆₀ alkynyl; a substituted or unsubstituted C₃₋₆₀ cycloalkyl; a substituted or unsubstituted C₆₋₆₀ aryl; or a substituted or unsubstituted C₂₋₆₀ heteroaryl containing any one or more heteroatoms selected from the group consisting of N, O and S, and
y is an integer of 1 to 10.

2. The organic light emitting device according to claim 1, wherein the Chemical Formula 1 is represented by any one selected from the group consisting of the following Chemical Formulas 1-1 to 1-6: 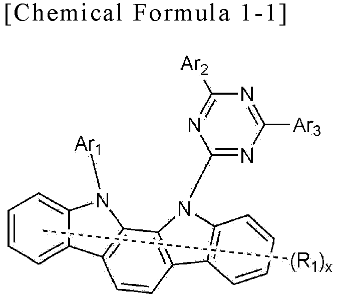 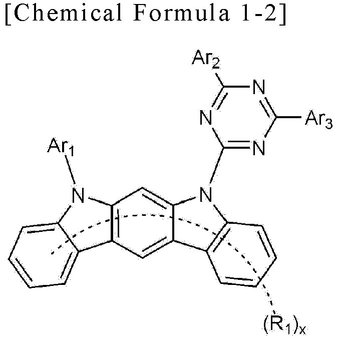 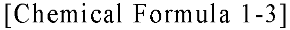 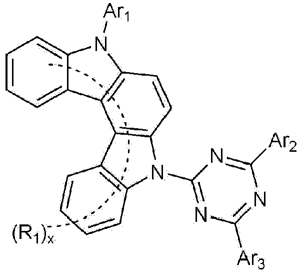 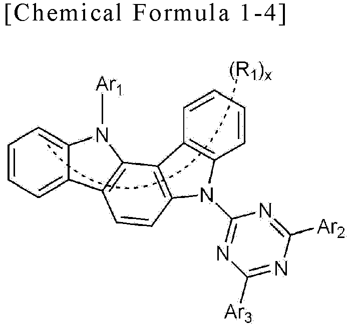 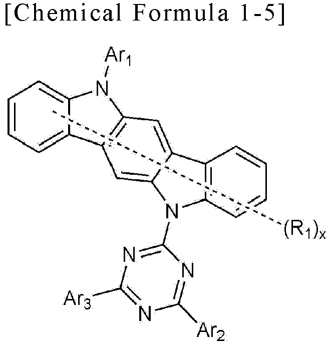 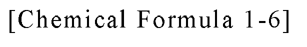 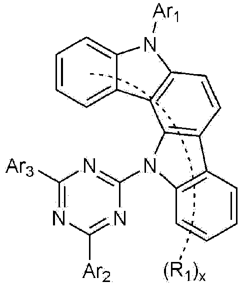 in the Chemical Formulas 1-1 to 1-6, Ar₁ to Ar₃, R₁, and x are as defined in claim 1.

3. The organic light emitting device according to claim 1, wherein Ar₁ to Ar₃ are each independently phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl.

4. The organic light emitting device according to claim 1, wherein at least one of Ar₁ to Ar₃ are phenyl, and the rest are each independently phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl.

5. The organic light emitting device according to claim 1, wherein at least one of Ar₁ to Ar₃ are phenyl substituted with 5 deuterium, and the rest are phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is unsubstituted.

6. The organic light emitting device according to claim 1, wherein one of the Ar₁ to Ar₃ is biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, each of which is substituted with 5 or more deuteriums, and
the rest are independently unsubstituted phenyl or unsubstituted biphenyl.

7. The organic light emitting device according to claim 1, wherein one of Ar₁ to Ar₃ is phenyl, biphenyl, terphenyl, dibenzofuranyl, dibenzothiophenyl, fluorenyl, 9,9-dimethylfluorenyl, 9,9-diphenylfluorenyl, carbazol-9-yl, or 9-phenyl-9H-carbazolyl, and
the Ar₁ to Ar₃ are completely substituted with deuterium.

8. The organic light emitting device according to claim 1, wherein all of the R₁ are hydrogen.

9. The organic light emitting device according to claim 1, wherein at least 5 of the R₁ are deuterium, and the rest is hydrogen.

10. The organic light emitting device according to claim 1, wherein the first compound is any one selected from the group consisting of the following:

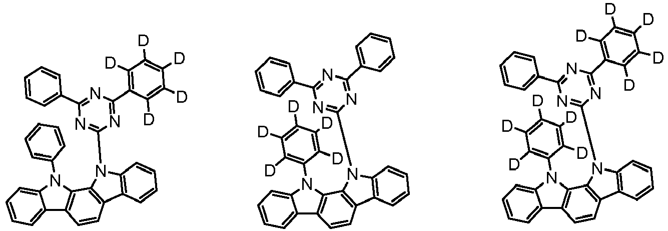

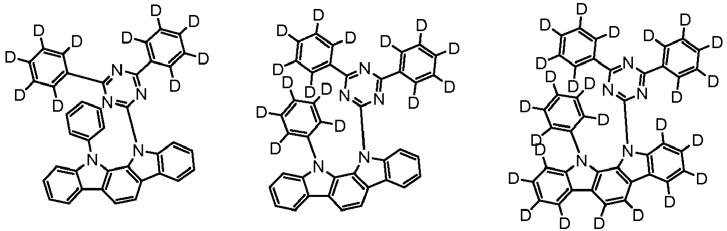

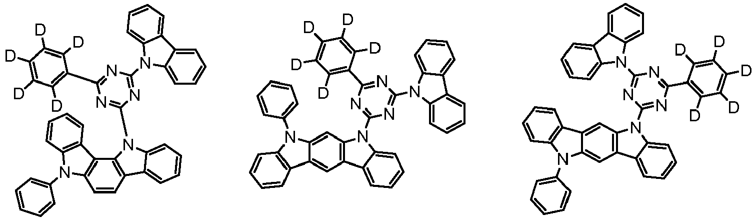

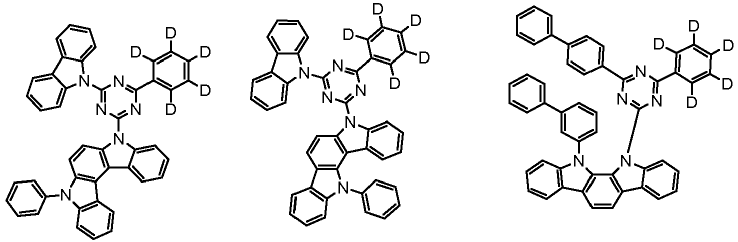

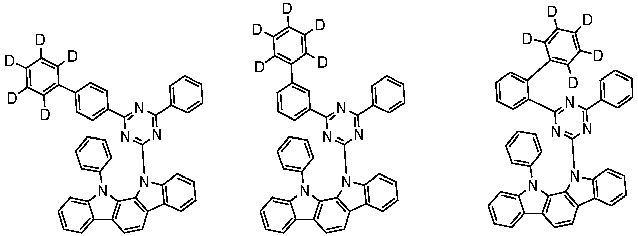

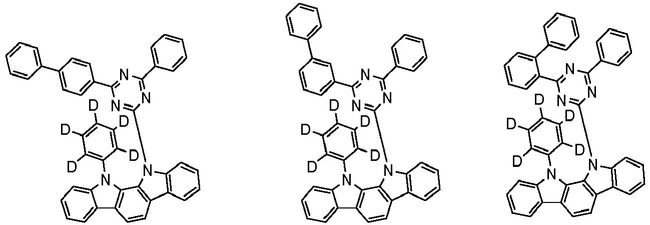

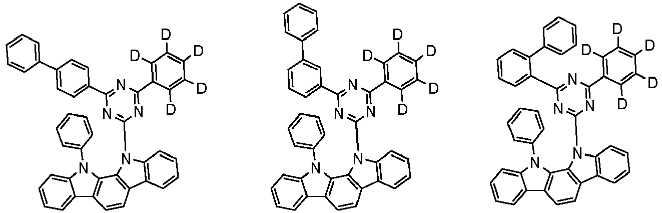

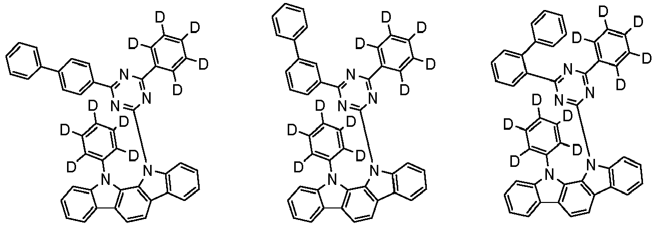

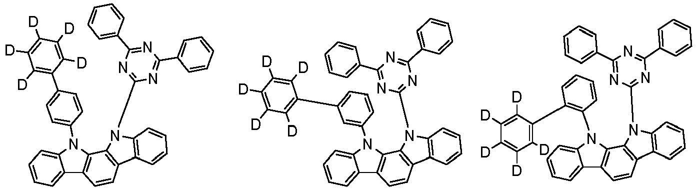

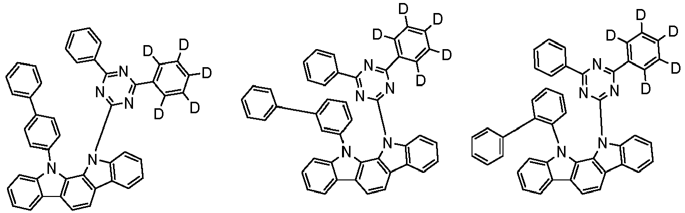

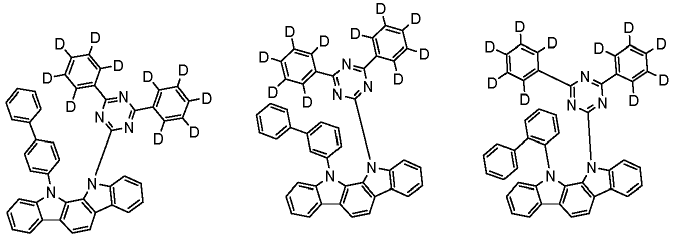

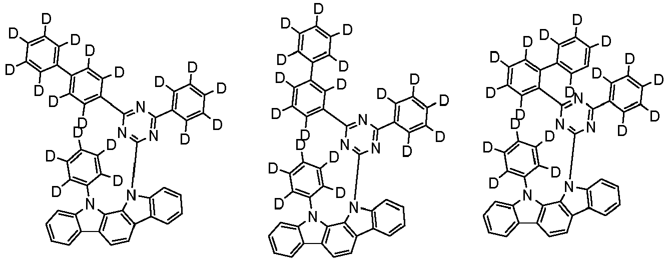

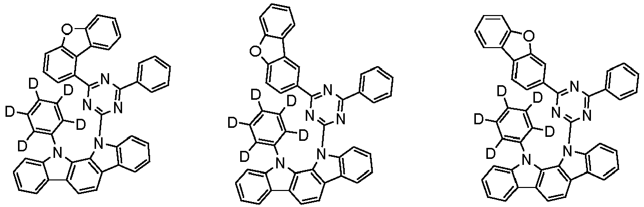

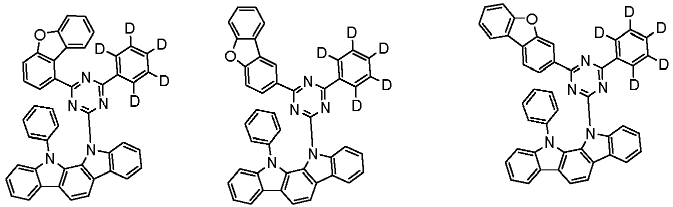

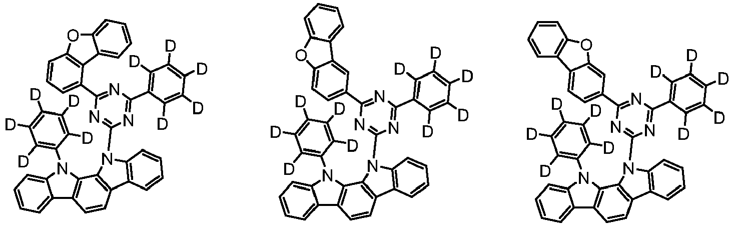

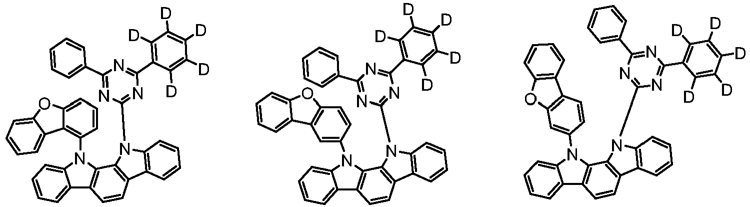

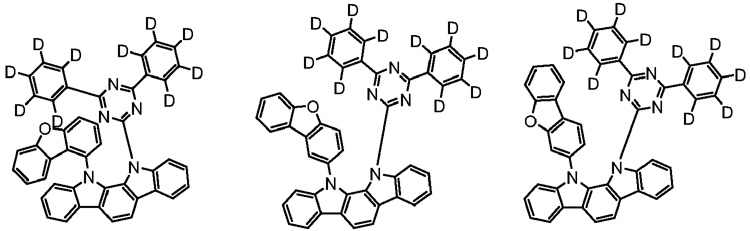

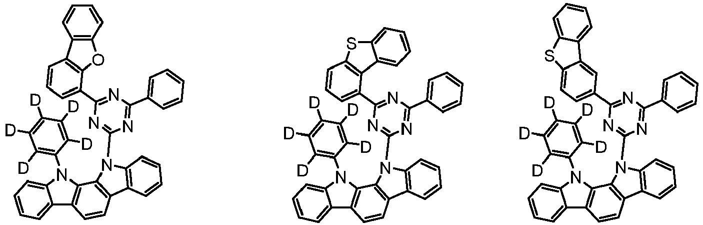

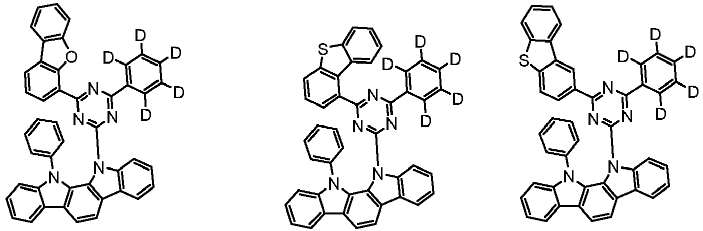

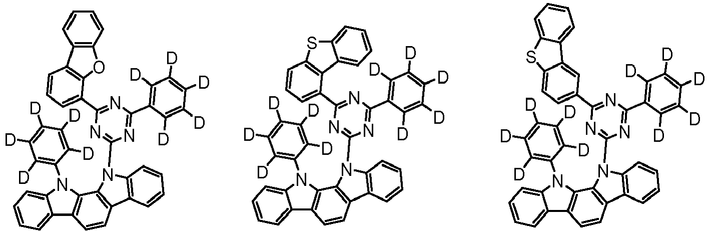

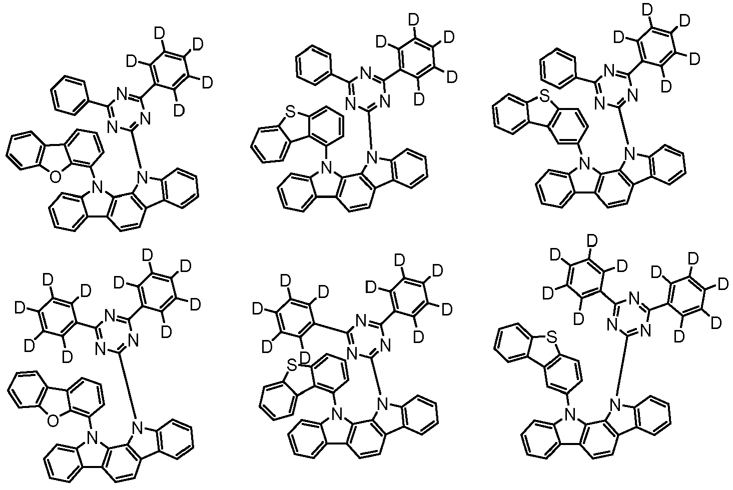

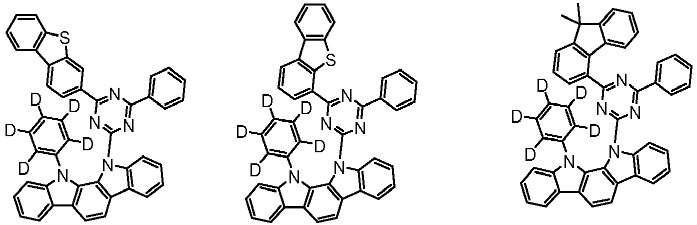

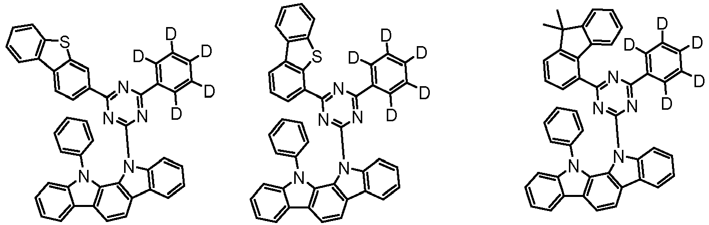

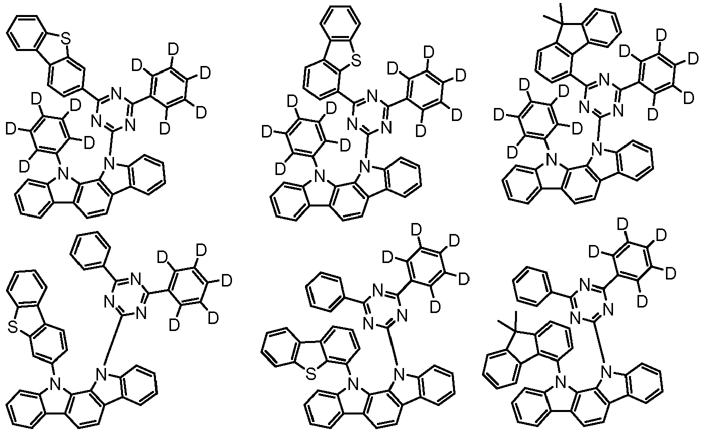

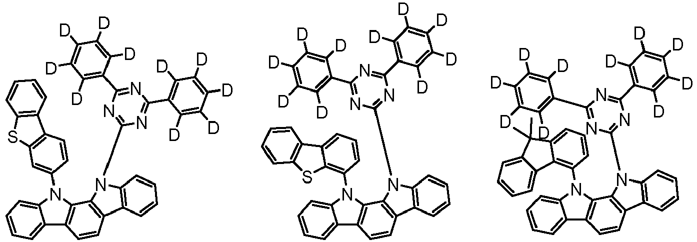

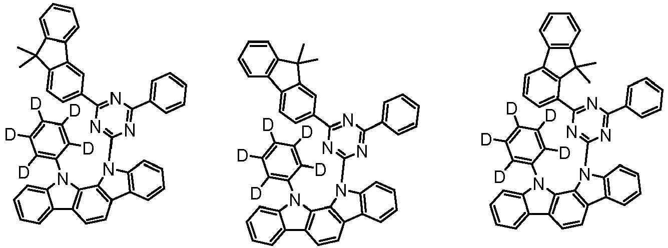

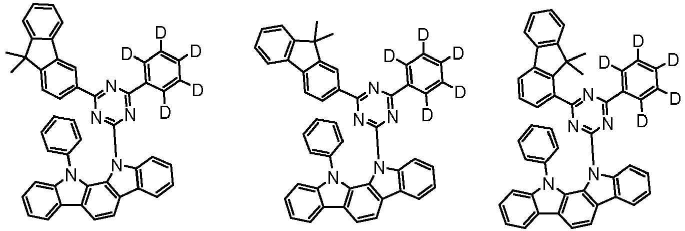

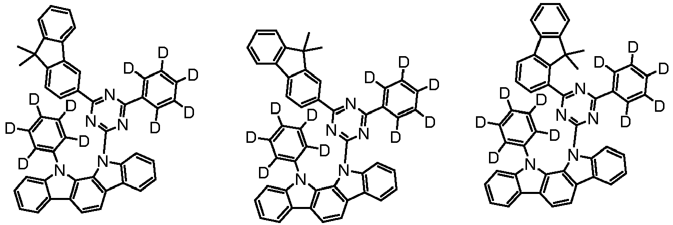

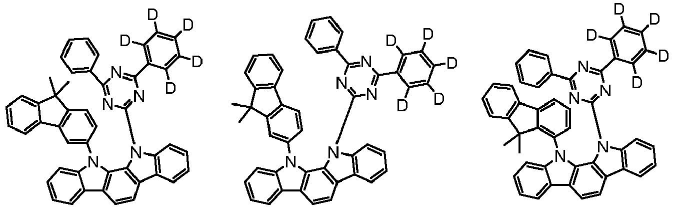

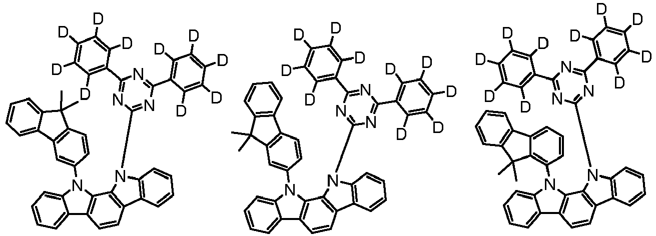

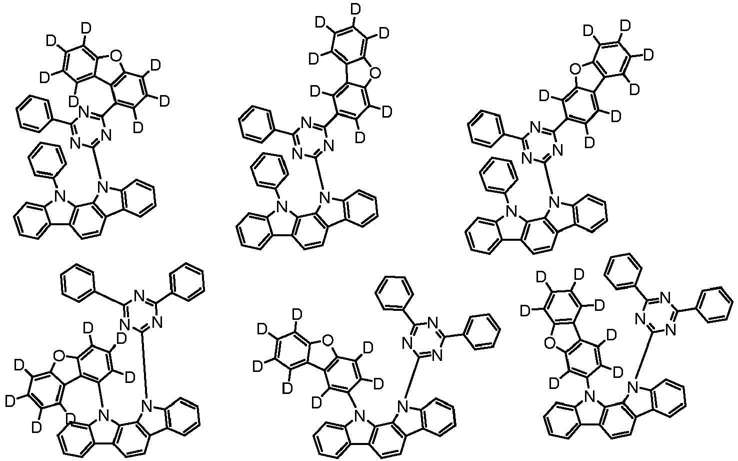

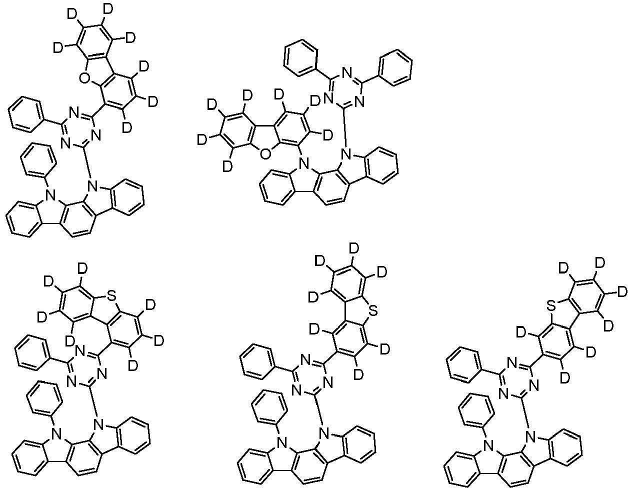

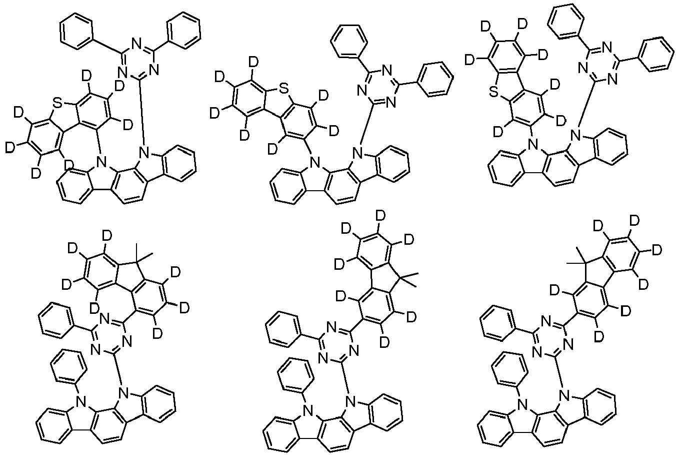

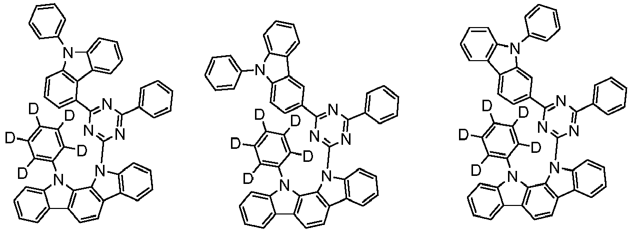

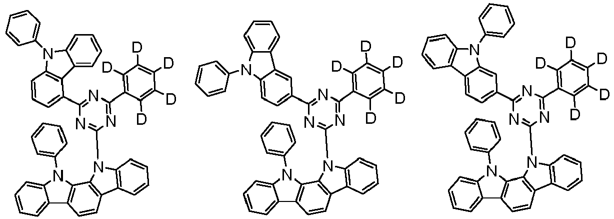

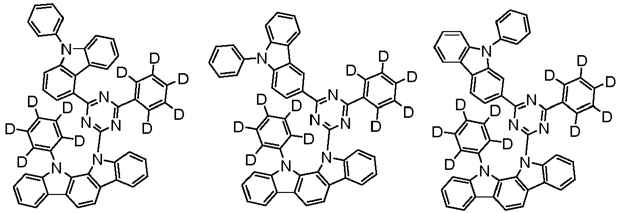

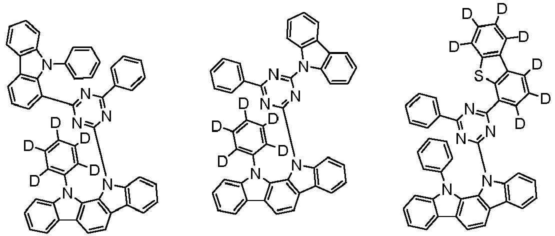

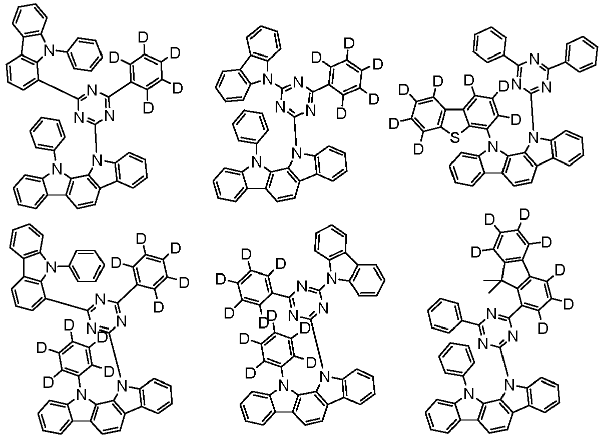

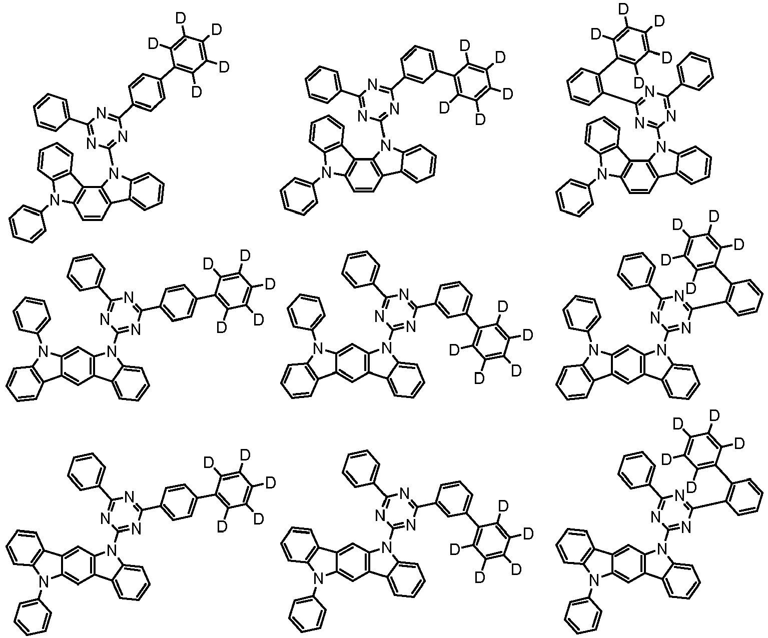

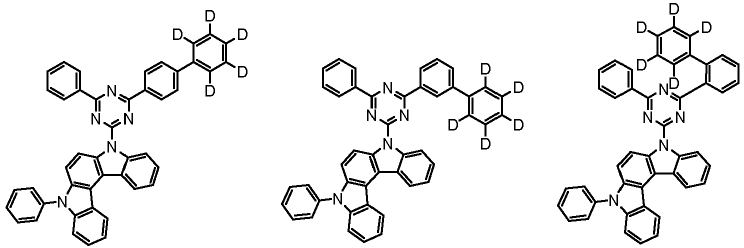

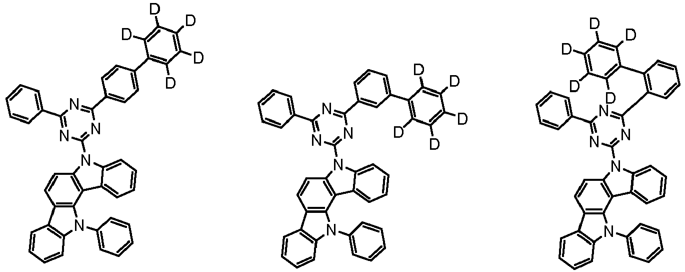

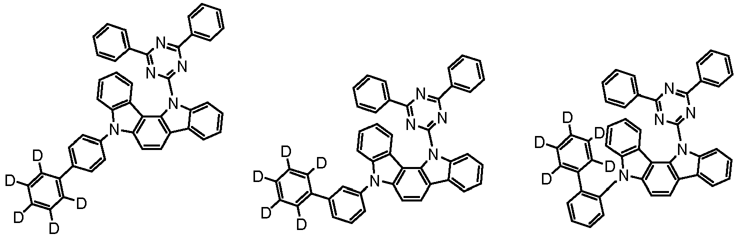

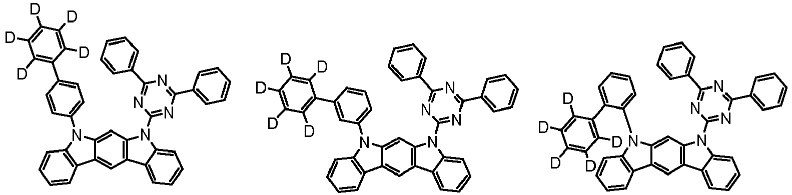

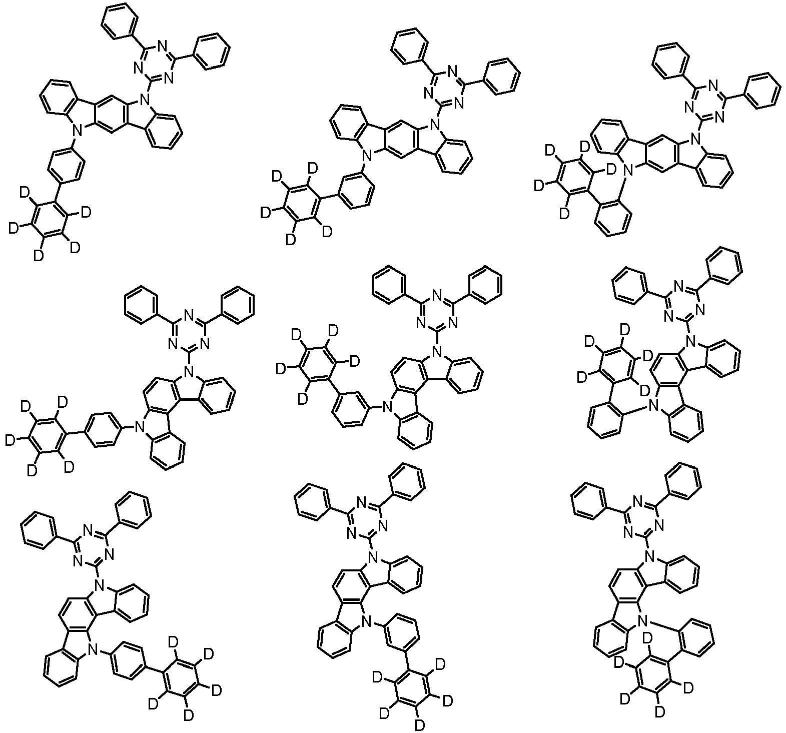

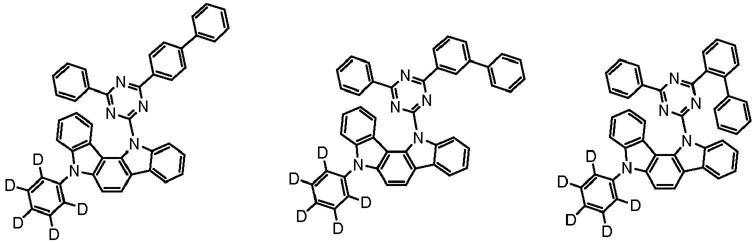

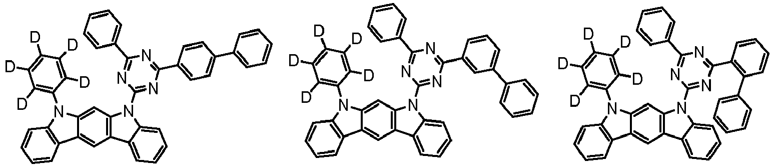

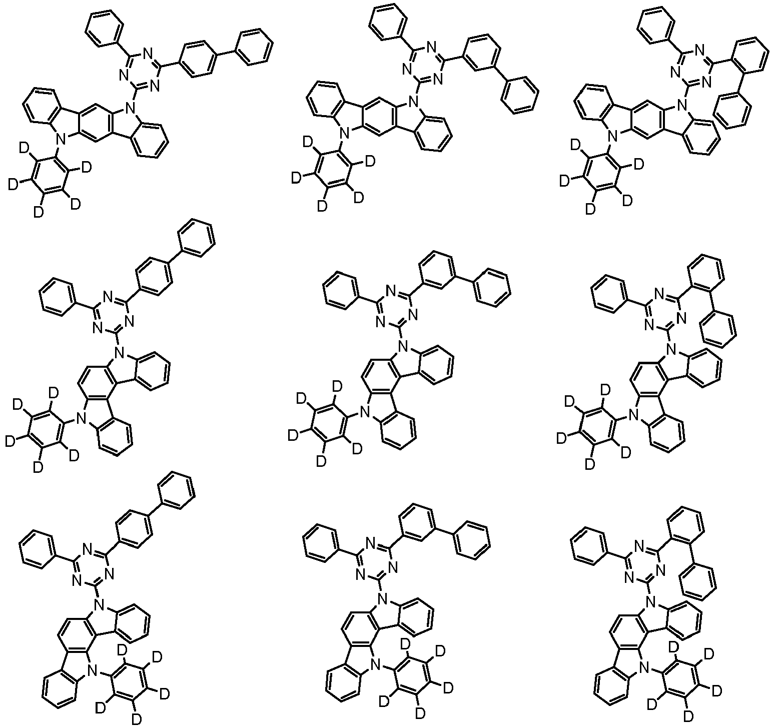

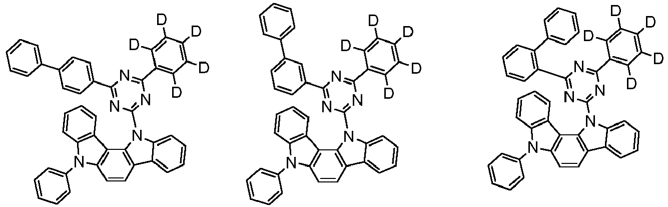

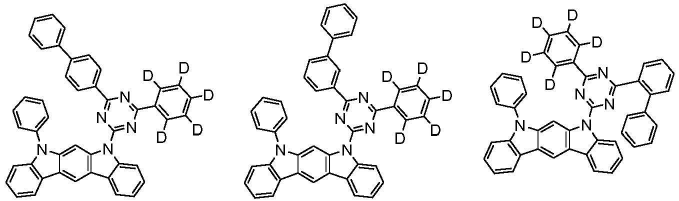

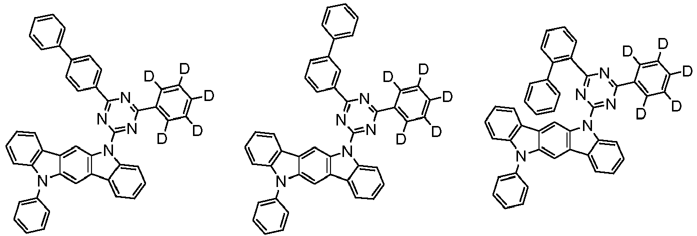

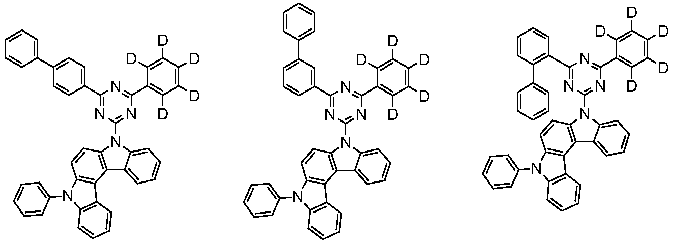

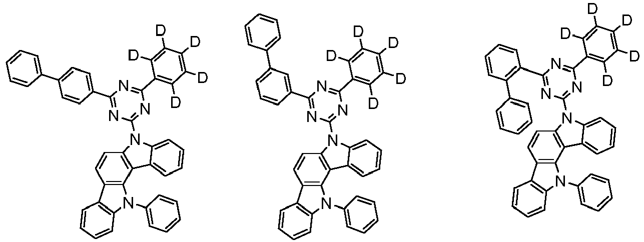

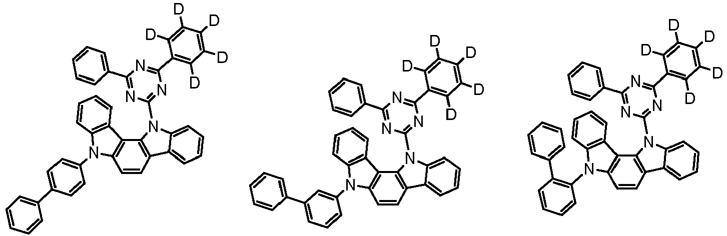

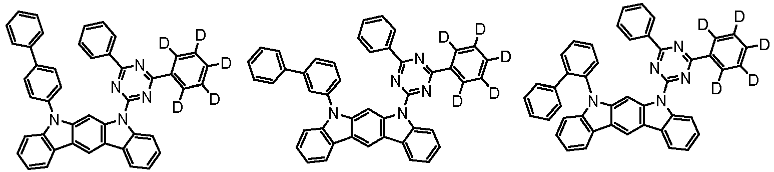

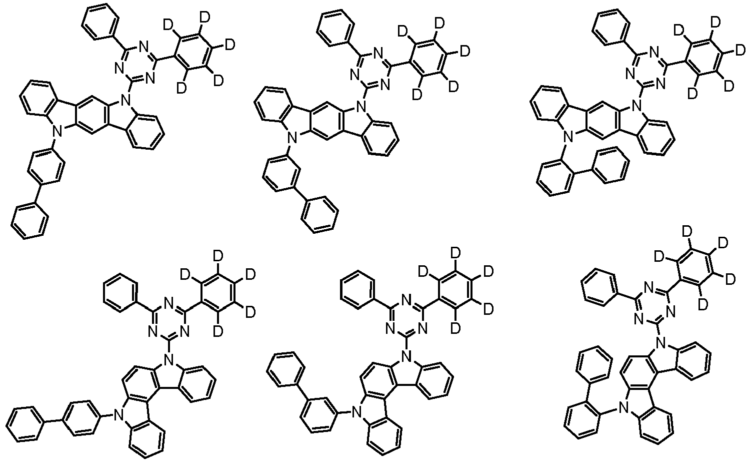

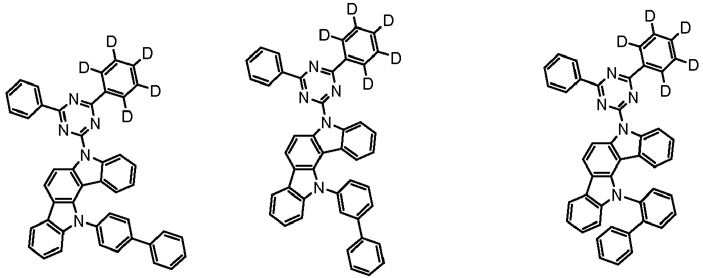

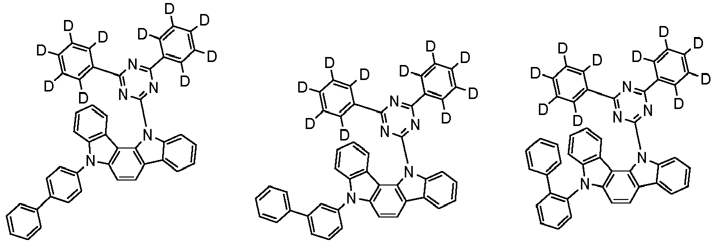

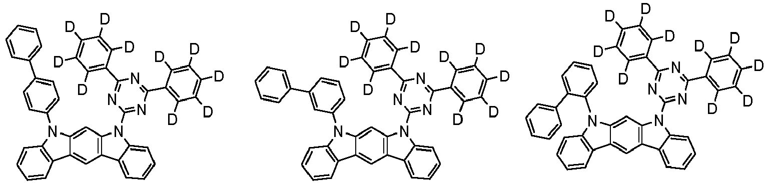

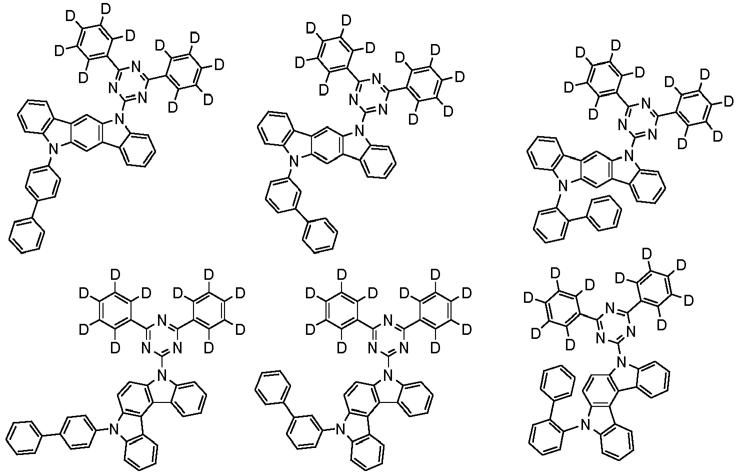

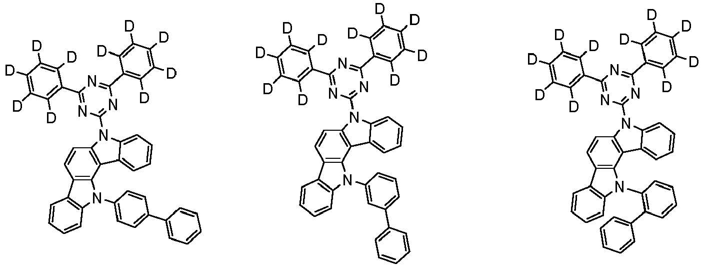

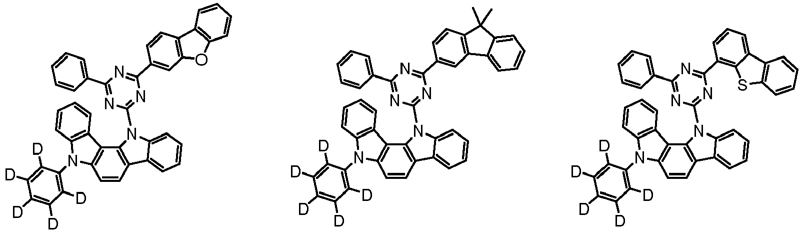

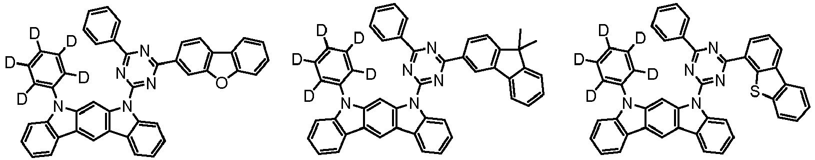

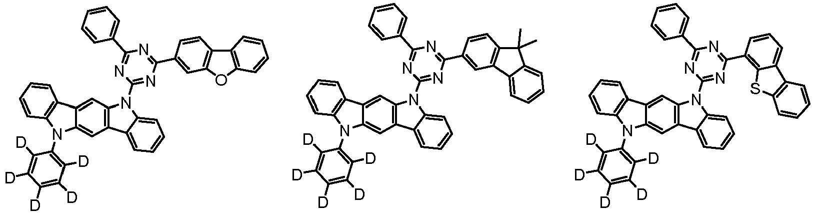

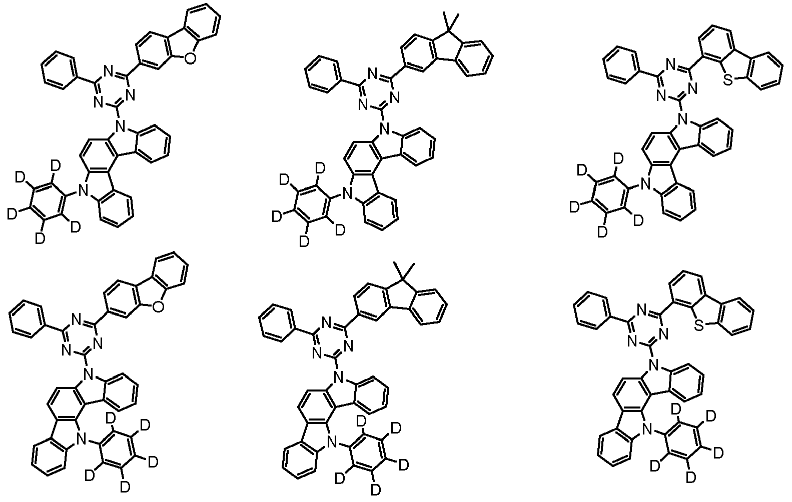

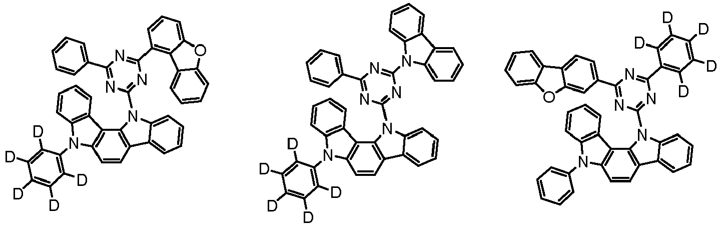

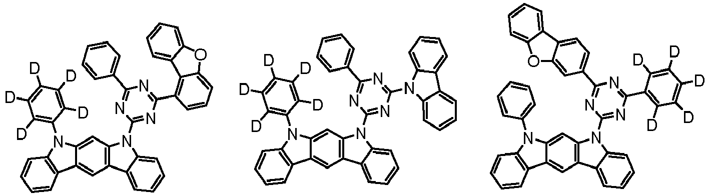

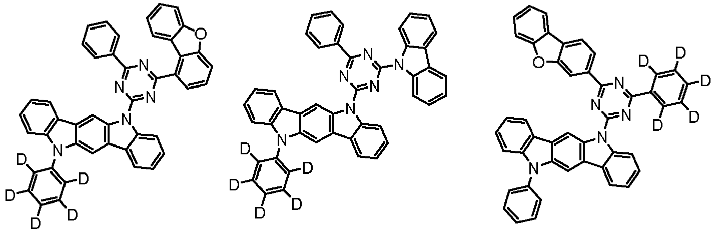

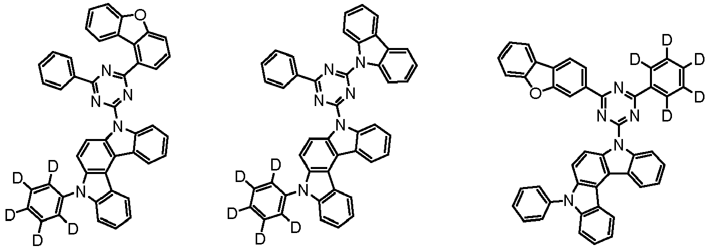

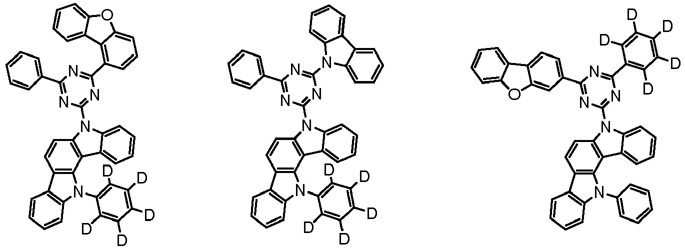

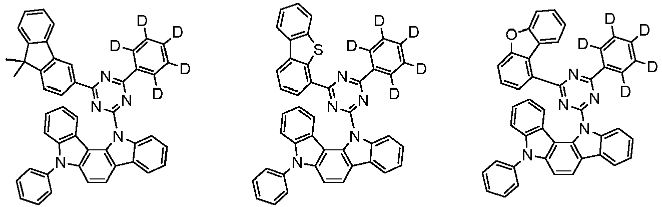

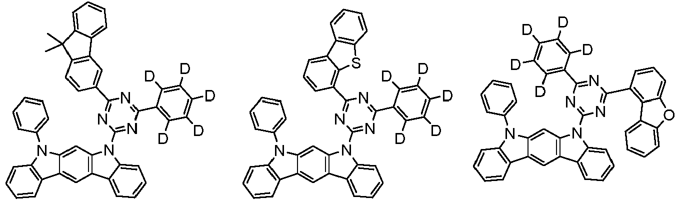

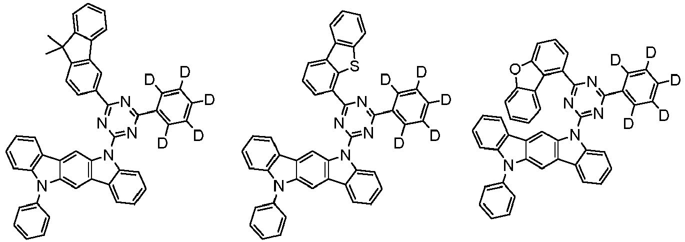

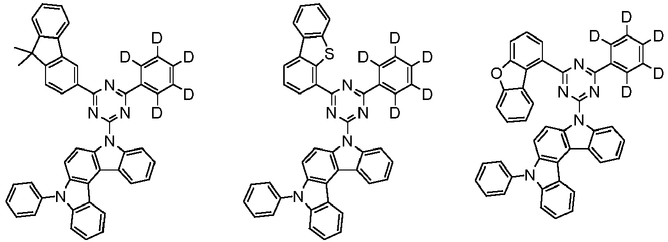

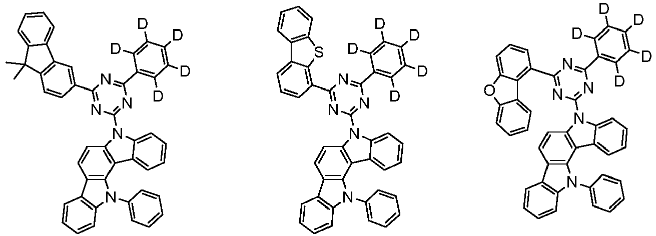

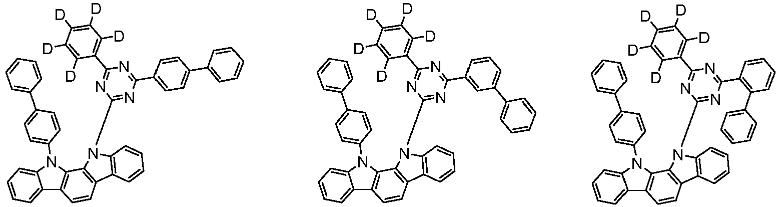

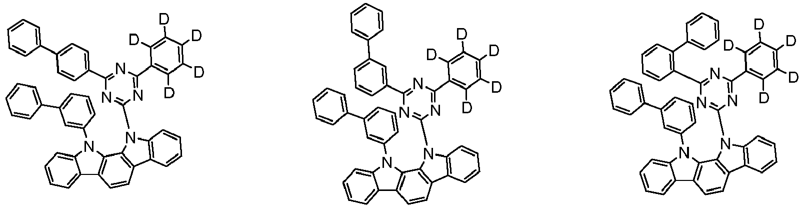

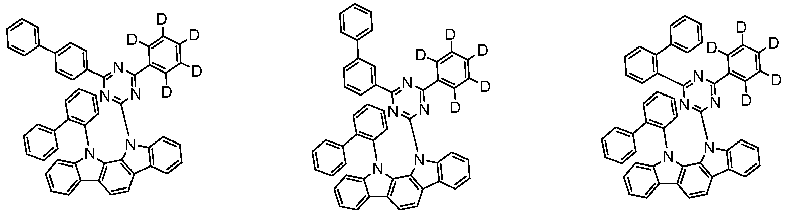

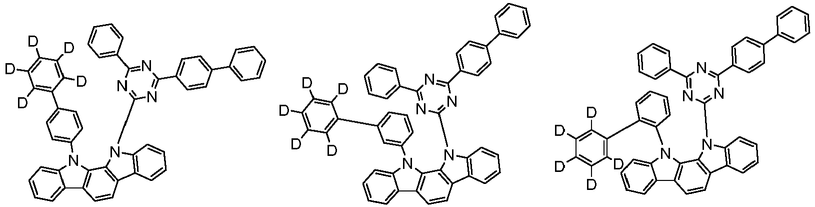

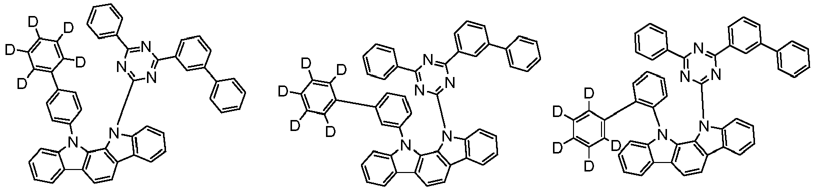

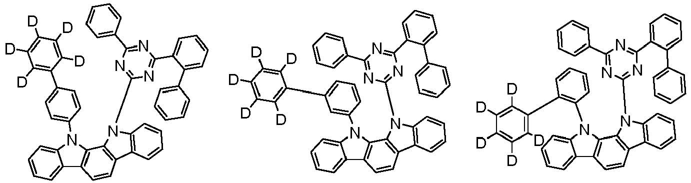

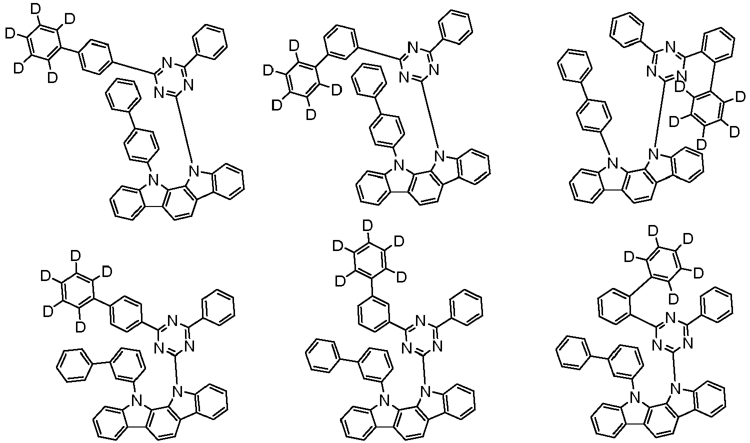

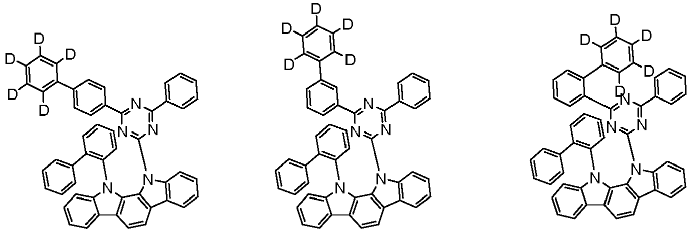

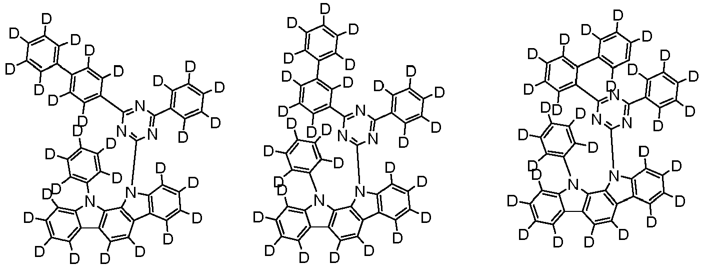

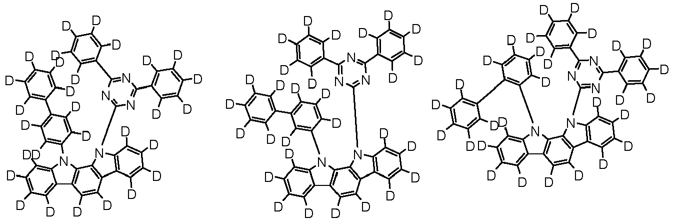

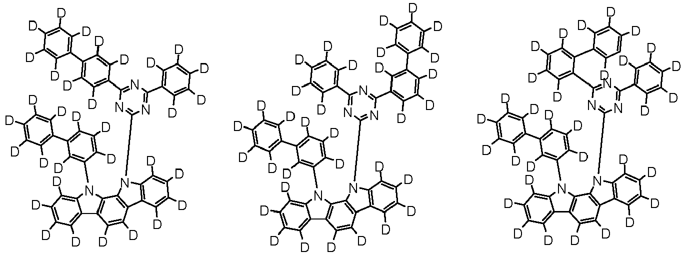

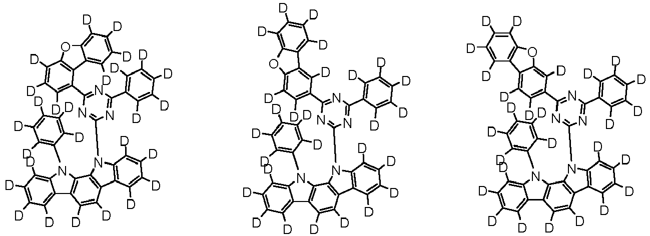

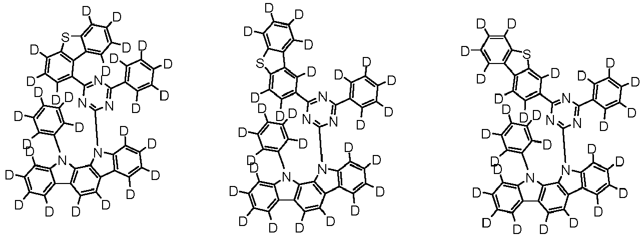

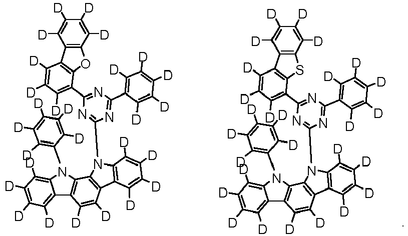

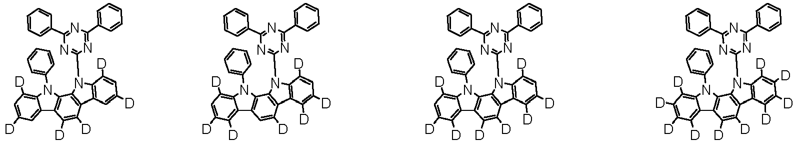

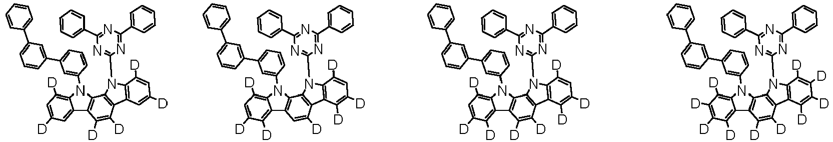

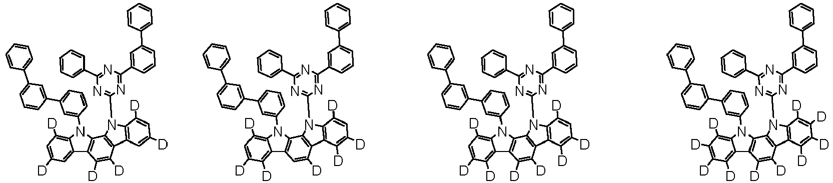

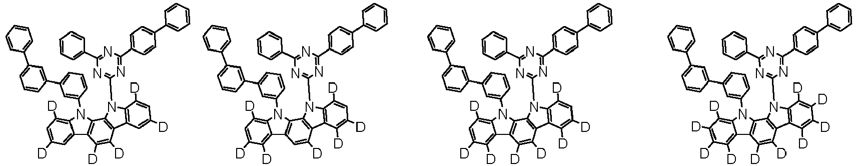

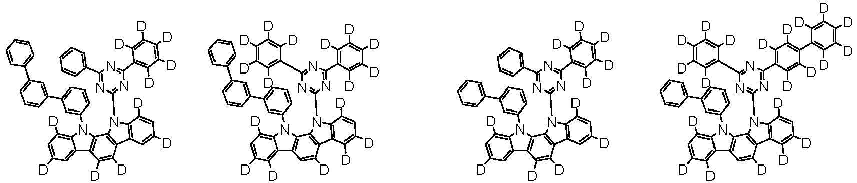

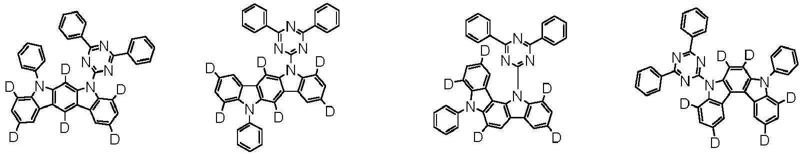

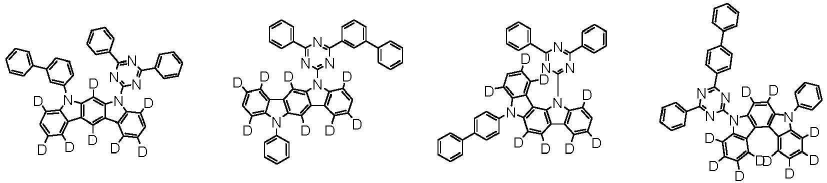

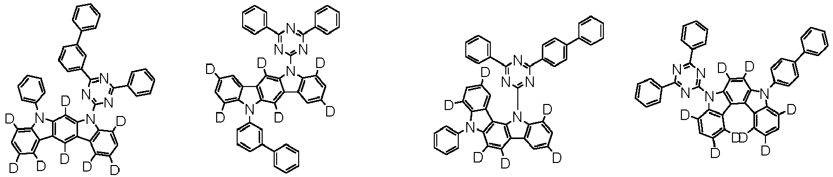

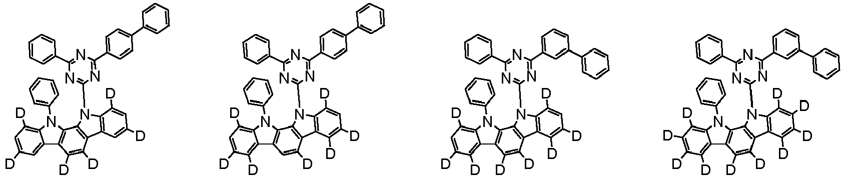

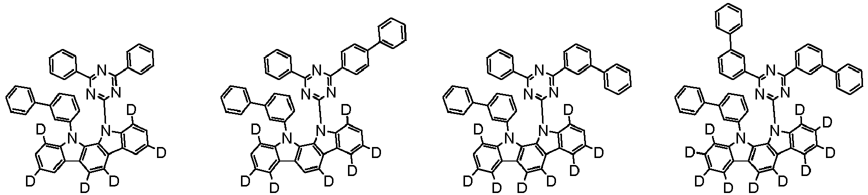

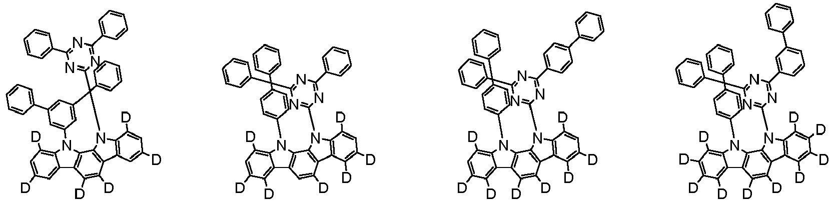

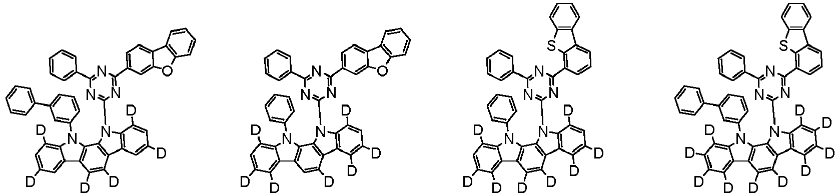

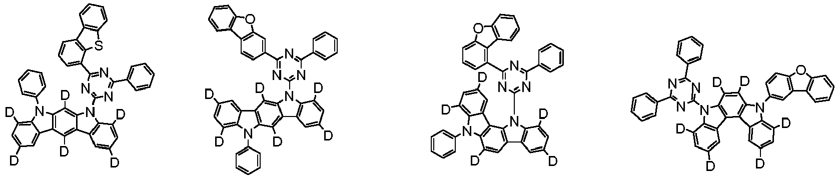

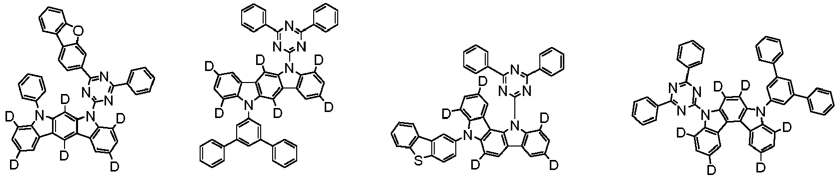

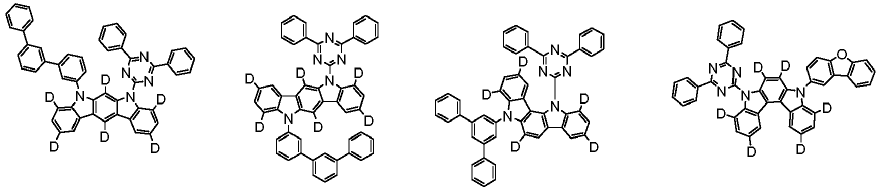

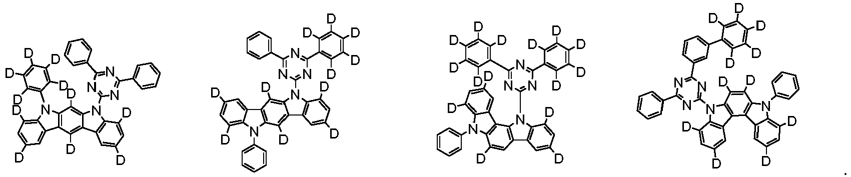

11. The organic light emitting device according to claim 1, wherein the Chemical Formula 2 is represented by any one selected from the group consisting of the following Chemical Formulas 2-1 to 2-6: 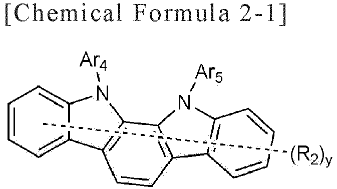 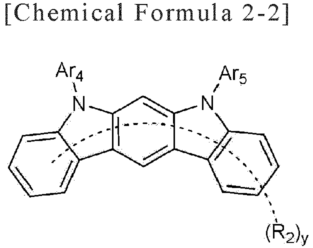 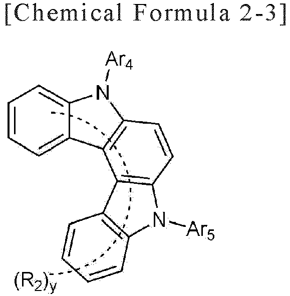 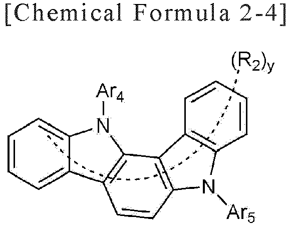 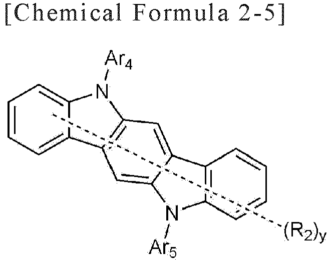 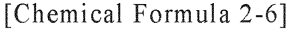 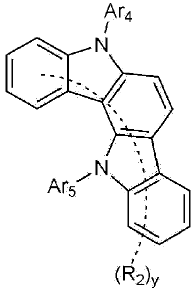 in Chemical Formulas 2-1 to 2-6, Ar4, Ars, R₂, and y are as defined in claim 1.

12. The organic light emitting device according to claim 1, wherein Ar₄ and Ars are each independently, phenyl, biphenyl, terphenyl, fluorenyl, 9,9-dimethylfluorenyl, or 9,9-diphenylfluorenyl, each of which is unsubstituted.

13. The organic light emitting device according to claim 1, wherein Ar₄ is phenyl, biphenyl or terphenyl, each of which is unsubstituted, and
Ars is dibenzofuranyl, dibenzothiophenyl, carbazol-9-yl, or 9-phenyl-9H-carbazolyl, each of which is unsubstituted.

14. The organic light emitting device according to claim 1, wherein all of R₂ are hydrogen.

15. The organic light emitting device according to claim 1, wherein the second compound is any one selected from the group consisting of the following:
